# EUROPEAN PATENT SPECIFICATION

(11) **EP 4 296 672 B1**
(45) Date of publication and mention of the grant of the patent: **13.11.2024**
(21) Application number: 23180231.5
(22) Date of filing: 20.06.2023
(51) Int. Cl.: G01N 33/533, C07K 2/00, C07K 16/46, G01N 33/535, G01N 33/58

(54) **LABELED POLYPEPTIDE, MODIFIED POLYPEPTIDE, PRODUCTION METHOD FOR THESE POLYPEPTIDES, REAGENT CONTAINING THESE POLYPEPTIDES, AND MEASUREMENT METHOD FOR TARGET SUBSTANCE**
MARKIERTES POLYPEPTID, MODIFIZIERTES POLYPEPTID, HERSTELLUNGSVERFAHREN FÜR DIESE POLYPEPTIDE, REAGENS MIT DIESEN POLYPEPTIDEN UND MESSVERFAHREN FÜR ZIELSUBSTANZ
POLYPEPTIDE MARQUÉ, POLYPEPTIDE MODIFIÉ, PROCÉDÉ DE PRODUCTION DE CES POLYPEPTIDES, RÉACTIF CONTENANT CES POLYPEPTIDES ET PROCÉDÉ DE MESURE POUR SUBSTANCE CIBLE

(30) Priority: 20.06.2022 JP 2022099159; 31.01.2023 JP 2023012581
(43) Date of publication of application: 27.12.2023
(73) Proprietor: SYSMEX CORPORATION, Kobe-shi Hyogo 651-0073 (JP)
(72) Inventor: SATO, Haruya, Kobe-shi, Hyogo, 651-0073 (JP); NAITOH, Katsuki, Kobe-shi, Hyogo, 651-0073 (JP); KATAOKA, Yukiko, Kobe-shi, Hyogo, 651-0073 (JP); NISHIKAWA, Yochi, Kobe-shi, Hyogo, 651-0073 (JP); OKANO, Daiki, Kobe-shi, Hyogo, 651-0073 (JP)
(74) Representative: De Clercq & Partners

(56) References cited:
- WO-A1-2016/144608
- WO-A1-2021/151984
- US-A1- 2020 101 146
- US-B2- 8 658 389
- MARCULESCU CRISTINA ET AL: "Probing the limits of Q-tag bioconjugation of antibodies", CHEMICAL COMMUNICATIONS, vol. 55, no. 76, 2019, pages 11342 - 11345, XP093090015

## Description

### FIELD OF THE INVENTION

The present invention relates to a labeled polypeptide and a production method therefor. The present invention relates to a modified polypeptide and a production method therefor. The present invention relates to a reagent containing a labeled polypeptide or a modified polypeptide. The present invention relates to a measurement method for a target substance.

### BACKGROUND

Transglutaminase (TG) is an enzyme that recognizes a glutamine residue in a polypeptide as a substrate, and catalyzes a reaction between a carboxamide side chain of the glutamine residue and an amino group of a primary amine, to form an amide bond. More specifically, in the TG-catalyzed reaction, the amino group in the carboxamide side chain of the glutamine residue in the polypeptide condenses with the primary amine, and a substituent on the primary amine is transferred to the glutamine residue, to produce ammonia. In recent years, techniques with use of TG, for binding a functional substance, such as drug or label, to a glutamine residue-containing polypeptide have been known.

For example, U.S. Patent Application Publication No. 2018/0037921 describes that an anticancer drug-bound antibody was obtained, by reacting an antibody having a glutamine residue-containing peptide tag bound to the C-terminal of the heavy chain, with an anticancer drug bound to an amino group-containing linker, in the presence of TG. TG-catalyzed binding of a functional substance to the polypeptide usually employs a bifunctional linker that has functional groups at both ends. The functional groups of the bifunctional linker are selectable from an amino group (-NH₂) as an amine donor in the TG-catalyzed reaction, and any functional group that covalently binds the functional substance to the linker. The glutamine residue in the polypeptide and the functional substance are thus bound through such linker.

### SUMMARY OF THE INVENTION

Efforts of the present inventors to bind a functional substance to a glutamine residue in a polypeptide using the linker described in U.S. Patent Application Publication No. 2018/0037921 have, however, not always succeeded in binding them with high efficiency. It is, therefore, an object of the present invention to provide a way to bind a label to a polypeptide with high efficiency.

The present inventors examined, as the bifunctional linker, a polyethylene glycol (PEG) chain having a thiol group (-SH) and an amino group, and found that such linker of which the PEG chain moiety has a molecular weight of 1100 or larger could efficiently bind the linker to the glutamine residue in the polypeptide, thereby completing the present invention.

According to the present invention, provided is a labeled polypeptide comprising a side chain-containing glutamine residue, the side chain being represented by formula (I) below: 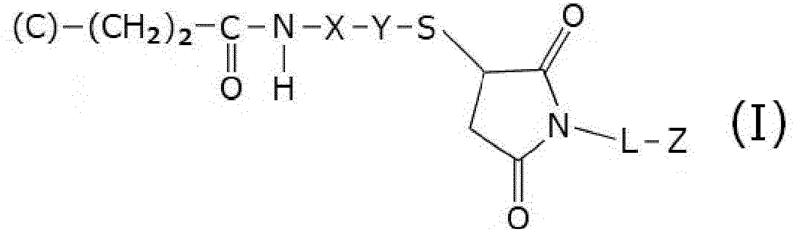 wherein, (C) represents an α-carbon of the glutamine residue, X represents a straight chain alkylene group, Y represents a polyethylene glycol chain, Z represents a label, L represents a spacer or an atomic bond, and the polyethylene glycol chain has a molecular weight of 1100 or larger.

In certain embodiments, the labeled polypeptide is a fusion polypeptide of an antibody, with a peptide tag that comprises the side chain-containing glutamine residue.

The present invention also provides a modified polypeptide comprising a side chain-containing glutamine residue, the side chain being represented by formula (II) below: 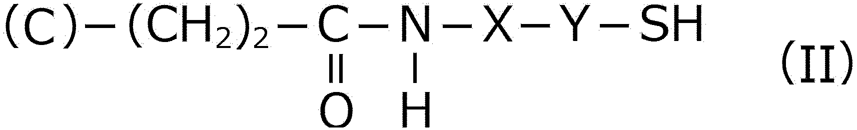 wherein, (C) represents an α-carbon of the glutamine residue, X represents a straight chain alkylene group, Y represents a polyethylene glycol chain, and the polyethylene glycol chain has a molecular weight of 1100 or larger.

In certain embodiments, the modified polypeptide is a fusion polypeptide of an antibody, with a peptide tag that comprises the side chain-containing glutamine residue.

In certain embodiments, the polyethylene glycol chain has a weight average molecular weight of 1300 or larger.

In certain embodiments, the polyethylene glycol chain has a weight average molecular weight of 1700 or larger.

In certain embodiments, the polyethylene glycol chain is represented by formula (III) below:

-(OCH₂CH₂)ₙ- or -(CH₂CH₂O)ₙ- (III)

wherein, n represents an integer of 25 or larger.

In certain embodiments, n represents an integer of 39 or larger.

In certain embodiments, the straight chain alkylene group has 2 or more and 10 or less carbon atoms.

In certain embodiments, the label is at least one substance selected from the group consisting of biotins, enzyme, fluorescent dye, fluorescent protein, and hapten.

In certain embodiments, the antibody is Fab, Fab', F(ab')₂, Fd, Fd', Fv, scFv, dAb, rIgG, light chain, heavy chain antibody, variable domain of heavy chain antibody, diabody, or triabody.

The present invention also provides a reagent comprising the polypeptide.

The present invention also provides a production method for a modified polypeptide, the method comprising:
contacting, in the presence of a transglutaminase, a glutamine residue-containing polypeptide with a linker represented by formula (VI) below:

   NH₂-X-Y-SH (VI)
wherein
X represents a straight chain alkylene group, Y represents a polyethylene glycol chain, the polyethylene glycol chain having a molecular weight of 1100 or larger, to bind the linker to a carboxamide side chain of the glutamine residue; and
obtaining a modified polypeptide produced by the binding of the carboxamide side chain with the linker;
wherein,
in the modified polypeptide,
a side chain of the glutamine residue is represented by formula (II) below: 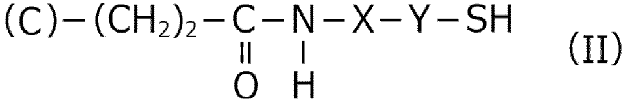
wherein,
(C) represents an α-carbon atom of the glutamine residue, X represents a straight chain alkylene group, Y represents a polyethylene glycol chain, and the polyethylene glycol chain has a molecular weight of 1100 or larger.

The present invention also provides a production method for a labeled polypeptide, the method comprising:
contacting, in the presence of a transglutaminase, a glutamine residue-containing polypeptide with a linker represented by formula (VI) below:

   NH₂-X-Y-SH (VI)
wherein
X represents a straight chain alkylene group, Y represents a polyethylene glycol chain, the polyethylene glycol chain having a molecular weight of 1100 or larger, to bind the linker to a carboxamide side chain of the glutamine residue;
obtaining a modified polypeptide produced by the binding of the carboxamide side chain with the linker;
contacting the modified polypeptide with a maleimide group-containing label, to bind the label to the linker bound to the modified polypeptide; and
obtaining a labeled polypeptide produced by the binding of the modified polypeptide and the label,
wherein,
in the labeled polypeptide,
a side chain of the glutamine residue is represented by formula (I) below: 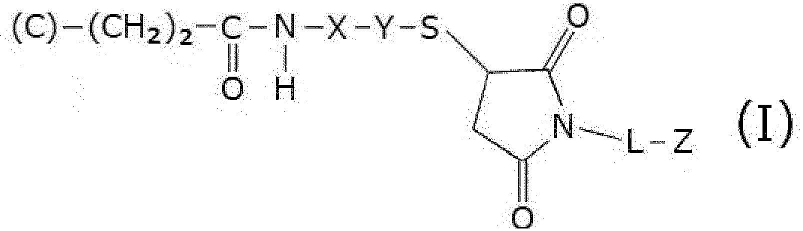
wherein,
(C) represents an α-carbon atom of the glutamine residue, X represents a straight chain alkylene group, Y represents a polyethylene glycol chain, Z represents a label, L represents a spacer or an atomic bond, and the polyethylene glycol chain has a molecular weight of 1100 or larger.

In certain embodiments, the glutamine residue-containing polypeptide is a fusion polypeptide of an antibody, with a peptide tag that contains the glutamine residue.

In certain embodiments, the polyethylene glycol chain has anaverage molecular weight of 1300 or larger.

In certain embodiments, the polyethylene glycol chain has an average molecular weight of 1700 or larger.

In certain embodiments, the polyethylene glycol chain is represented by formula (III) below:

-(OCH₂CH₂)ₙ- or -(CH₂CH₂O)ₙ- (III)

wherein, n represents an integer of 25 or larger.

In certain embodiments, n represents an integer of 39 or larger.

The present invention also provides a measurement method for a target substance, the method comprising:
forming an immune complex of the labeled polypeptide, wherein the labeled polypeptide comprises a side chain-containing glutamine residue, the side chain being represented by formula (I) below: 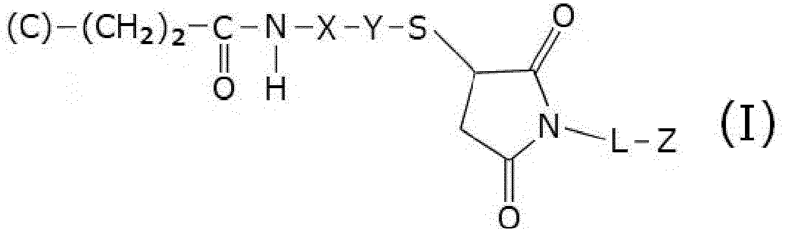
wherein,
(C) represents an α-carbon of the glutamine residue, X represents a straight chain alkylene group, Y represents a polyethylene glycol chain, Z represents a label, L represents a spacer or an atomic bond, and the polyethylene glycol chain has a molecular weight of 1100 or larger and wherein the labeled polypeptide is a fusion polypeptide of an antibody with a peptide tag that comprises the side chain-containing glutamine residue, with a target substance; and
detecting a signal generated by the label contained in the immune complex.
In certain embodiments, in the forming step, the immune complex is formed on a solid phase.

The present invention enables highly efficient binding of a label to a polypeptide, with use of a linker having an amino group and a thiol group.

### BRIEF DESCRIPTION OF THE DRAWINGS

Fig. 1A is a schematic drawing illustrating an exemplary reagent of this embodiment.
Fig. 1B is a schematic drawing illustrating an exemplary reagent kit of this embodiment.
Fig. 2A is a spectral chart illustrating an analytical result of an SH-PEG-NH₂ linker (2K) (Sigma-Aldrich), analyzed by infusion mass spectrometry (infusion MS).
Fig. 2B is an infusion MS chart illustrating an analytical result of the SH-PEG-NH₂ linker (2K) from another lot (Sigma-Aldrich).
Fig. 2C is an infusion MS chart illustrating an analytical result of an SH-PEG-NH₂ linker (2K) (Biopharma PEG Scientific Inc.).
Fig. 2D is an infusion MS chart illustrating an analytical result of an SH-PEG-NH₂ linker (2K) (Creative PEGWorks).
Fig. 3 is a chart illustrating analytical results of size exclusion column chromatography (SEC) of a TG-catalyzed reaction product formed between csF001-5Fab-Qtag and the SH-PEG-NH₂ linker (2K) (Sigma-Aldrich).
Fig. 4 is an SEC chart illustrating analytical results of a TG-catalyzed reaction product formed between csF001-5Fab-Qtag and the SH-PEG-NH₂ linker (2K) (Sigma-Aldrich) under different pH values.
Fig. 5 is an SEC chart illustrating analytical results of a TG-catalyzed reaction product formed between csF028-22Fab-Qtag and the SH-PEG-NH₂ linker (2K) (Sigma-Aldrich) under different pH.
Fig. 6 is an SEC chart illustrating analytical results of a TG-catalyzed reaction product formed between csF001-5Fab-Qtag and an SH-PEG-NH₂ linker (3.5K) (Sigma-Aldrich).
Fig. 7 is a drawing that contains an SEC chart and a non-reducing SDS-PAGE chart illustrating analytical results of a reaction solution for coupling between SH-PEG(2K)-Fab, derived from csF001-5Fab-Qtag, and maleimide-modified ALP.
Fig. 8A is an SEC chart illustrating analytical results of a reaction solution for coupling between SH-PEG(2K)-Fab, derived from csF001-5Fab-Qtag, and maleimide-modified ALP.
Fig. 8B is a non-reducing SDS-PAGE chart illustrating analytical results of a collected fraction.
Fig. 8C is an SEC chart illustrating analytical results of the collected fractions.
Fig. 9 is an SEC chart illustrating analytical results of a reaction solution for coupling between SH-PEG(2K)-Fab, derived from csF028-22Fab-Qtag, and maleimide-modified ALP.
Fig. 10A is an SEC chart illustrating analytical results of a fraction from a reaction solution for coupling between SH-PEG(2K)-Fab, derived from csF028-22Fab-Qtag, and maleimide-modified ALP.
Fig. 10B is a non-reducing SDS-PAGE chart illustrating analytical results of a fraction collected from a reaction solution for coupling between SH-PEG(2K)-Fab, derived from csF028-22Fab-Qtag, and maleimide-modified ALP.
Fig. 11A is an SEC chart illustrating analytical results of a reaction solution for coupling between sF001-5Fab' and the maleimide-modified ALP.
Fig. 11B is a non-reducing SDS-PAGE chart illustrating analytical results of a fraction collected from a reaction solution for coupling between sF001-5Fab' and maleimide-modified ALP.
Fig. 12 is an SEC chart illustrating an analytical result of a fraction collected from a reaction solution for coupling between sF001-5Fab' and maleimide-modified ALP.
13A is a graph illustrating signal levels of HIV-1 p24 measured by enzyme-linked immunosorbent assay (ELISA) individually with use of Fab-PEG-ALP and (Fab')ₙ-ALP.
Fig. 13B is a graph illustrating noise levels of HIV-1 p24 measured by ELISA individually with use of Fab-PEG-ALP and (Fab')ₙ-ALP.
Fig. 13C is a graph illustrating signal-to-noise ratio (S/N) of HIV-1 p24 measured by ELISA individually with use of Fab-PEG-ALP and (Fab')ₙ-ALP.
Fig. 14 is a graph illustrating backgrounds of human serum measured by ELISA individually with use of Fab-PEG-ALP and (Fab')ₙ-ALP.
Fig. 15 is an SEC chart illustrating analytical results of a TG-catalyzed reaction product formed between HBs628Fab-Qtag and the SH-PEG-NH₂ linker (2K) (Sigma-Aldrich).
Fig. 16A is an SEC chart illustrating analytical results of a reaction solution for coupling between SH-PEG(2K)-Fab, derived from HBs628Fab-Qtag, and maleimide-modified biotin.
Fig. 16B contains non-reducing SDS-PAGE charts illustrating analytical results of a fraction collected from a reaction solution for coupling between SH-PEG(2K)-Fab, derived from HBs628Fab-Qtag, and maleimide-modified biotin.
Fig. 16C contains SEC charts illustrating analytical results of fractions collected from a reaction solution for coupling between SH-PEG(2K)-Fab, derived from HBs628Fab-Qtag, and the maleimide-modified biotin.
Fig. 17A is a chart illustrating an analytical result of a non-reduced sample of HBs628Fab-Qtag, analyzed by liquid chromatography mass spectrometry (LC-MS).
Fig. 17B is an LC-MS chart illustrating an analytical result of a reduced sample of HBs628Fab-Qtag.
Fig. 18 contains LC-MS charts illustrating analytical results of non-reduced samples of HBs628Fab-Qtag, SH-PEG-Fab thereof, and biotin-PEG-Fab.
Fig. 19 contains LC-MS charts illustrating analytical results of reduced samples of HBs628Fab-Qtag and SH-PEG-Fab thereof.
Fig. 20 contains SEC charts illustrating analytical results of a reaction solution for coupling between SH-PEG(2K)-Fab, derived from HBs628Fab-Qtag, and maleimide-modified ALP.
Fig. 21 is a non-reducing SDS-PAGE chart illustrating analytical results of a coupling reaction solution that contains SH-PEG(2K)-Fab, derived from HBs628Fab-Qtag, and maleimide-modified ALP, and the individual raw materials.
Fig. 22 is an SEC chart illustrating analytical results of a TG-catalyzed reaction product formed between csF001-25Fab-Qtag and the SH-PEG-NH₂ linker (2K) (Sigma-Aldrich).
Fig. 23A is an SEC chart illustrating analytical results of a reaction solution for coupling between SH-PEG(2K)-Fab, derived from csF001-25Fab-Qtag, and Alexa 488-maleimide.
Fig. 23B is an SEC chart illustrating analytical results of a desalted and purified fraction collected from a reaction solution for coupling between SH-PEG(2K)-Fab, derived from csF001-25Fab-Qtag, and Alexa 488-maleimide, for confirmation of UV and fluorescence absorption.
Fig. 24A contains LC-MS charts illustrating analytical results of csF001-25Fab-Qtag, SH-PEG-Fab thereof, and a reduced sample of Alexa 488-PEG-Fab.
Fig. 24B contains enlarged views of the analytical results shown in Fig. 24A.
Fig. 25 is an SEC chart illustrating analytical results of a TG-catalyzed reaction product formed between anti-CD20 Fab-Qtag and the SH-PEG-NH₂ linker (2K) (Sigma-Aldrich) from another lot.
Fig. 26A is an SEC chart illustrating analytical results of a reaction solution that contains R-phycoerythrin (R-PE) and an EMCS reagent.
Fig. 26B is a reverse-phase HPLC chart illustrating analytical results of a reaction solution that contains R-PE and the EMCS reagent.
Fig. 27 contains SEC charts illustrating analytical results of a coupling reaction solution that contains SH-PEG(2K)-Fab derived from csF001-25Fab-Qtag, and maleimide-modified R-PE. wherein the left panel is a chromatogram detected by UV (280 nm); and the right panel is a chromatogram of the coupling reaction solution, detected by UV (280 nm) and fluorescence (excited at 565 nm, fluorescence at 574 nm).
Fig. 28 is an SEC chart illustrating analytical results of a fraction collected from a reaction solution for coupling between SH-PEG(2K)-Fab, derived from sF001-25Fab-Qtag, and the maleimide-modified R-PE, wherein the left panel is a chromatogram detected by UV (280 nm); and the right panel is a chromatogram detected by fluorescence (excited at 565 nm, fluorescence at 574 nm) for the coupling reaction.
Fig. 29 is an SEC chart illustrating analytical results of a coupling reaction solution that contains SH-PEG(2K)-Fab derived from anti-CD20 Fab-Qtag, and the maleimide-modified R-PE, wherein the left panel is a chromatogram detected by UV (280 nm); and the right panel is a chromatogram of the coupling reaction solution, detected by UV (280 nm) and fluorescence (excited at 565 nm, fluorescence at 574 nm).
Fig. 30 is an SEC chart illustrating analytical results of a fraction collected from a reaction solution for coupling between SH-PEG(2K)-Fab, derived anti-CD20 Fab-Qtag, and the maleimide-modified R-PE wherein the left panel is a chromatogram detected by UV (280 nm); and the right panel is a chromatogram detected by fluorescence (excited at 565 nm, fluorescence at 574 nm) for the coupling reaction.
Fig. 31A is a chart illustrating results of interaction between csF001-25Fab-Qtag and an antigen, measured with Biacore (trademark) T200 (Cytiva).
Fig. 31B is a chart illustrating results interaction between SH-PEG(2K)-Fab derived from csF001-25Fab-Qtag, and an antigen, measured with Biacore (trademark) T200 (Cytiva).
Fig. 31C is a chart illustrating results of interaction between csF001-25Fab-Qtag and an antigen, measured with Biacore (trademark) T200 (Cytiva), on a date different from the measurement in Fig. 31A.
Fig. 31D is a chart illustrating results of interaction between Alexa 488-PEG-Fab, derived from csF001-25Fab-Qtag, and an antigen, measured with Biacore (trademark) T200 (Cytiva).
Fig. 32A is a diagram illustrating results of ELISA individually with use of Fab-PEG-R-PE, (Fab-PEG)₂-R-PE, and (Fab-PEG)₃-R-PE derived from csF001-25Fab-Qtag (antigen not added).
Fig. 32B is a diagram illustrating results of ELISA individually with use of Fab-PEG-R-PE, (Fab-PEG)₂-R-PE, and (Fab-PEG)₃-R-PE derived from csF001-25Fab-Qtag (antigen added).
Fig. 33 is a diagram illustrating results of FCM of CD20-expressing cell, individually with use of Fab-PEG-R-PE, (Fab-PEG)₂-R-PE, and (Fab-PEG)₃-R-PE derived from anti-CD20 Fab-Qtag.
Fig. 34A is an infusion MS chart illustrating analytical results of SH-PEG-NH₂ linker (3.5K) (Sigma-Aldrich).
Fig. 34B is an infusion MS chart illustrating analytical results of SH-PEG-NH₂ linker (5K) (Sigma-Aldrich).
Fig. 35A is an SEC chart illustrating analytical results of a TG-catalyzed reaction product formed between csF001-25Fab-Qtag and SH-PEG-NH₂ linker (3.5K) (Sigma-Aldrich).
Fig. 35B is an SEC chart illustrating analytical results of a TG-catalyzed reaction product formed between csF001-25Fab-Qtag and the SH-PEG-NH₂ linker (5K) (Sigma-Aldrich).
Fig. 35C is a non-reducing SDS-PAGE chart illustrating analytical results of reaction products from TG-catalyzed reaction of csF001-25Fab-Qtag, with each of SH-PEG-NH₂ linker (3.5K) (Sigma-Aldrich) and SH-PEG-NH₂ linker (5K) (Sigma-Aldrich).
Fig. 36A is an SEC chart illustrating analytical results of a reaction solution for coupling between SH-PEG(3.5K)-Fab, derived from csF001-25Fab-Qtag, and Alexa 488-maleimide.
Fig. 36B is an SEC chart illustrating analytical results of a desalted and purified fraction collected from a reaction solution for coupling between SH-PEG(3.5K)-Fab, derived from csF001-25Fab-Qtag, and Alexa 488-maleimide, confirmed by UV and fluorescence absorption.
Fig. 37A is an SEC chart illustrating analytical results of a reaction solution for coupling between SH-PEG(5K)-Fab, derived from csF001-25Fab-Qtag, and Alexa 488-maleimide.
Fig. 37B is an SEC chart illustrating analytical results of a desalted and purified fraction collected from a reaction solution for coupling between SH-PEG(5K)-Fab, derived from csF001-25Fab-Qtag, and Alexa 488-maleimide, confirmed by UV and fluorescence absorption.
Fig. 38 is an SEC chart illustrating analytical results of a TG-catalyzed reaction product formed between csF001-5Fab-Qtag and SH-PEG-NH₂ linker (400Da) (Nanocs Inc.).
Fig. 39 is an SEC chart illustrating analytical results of a reaction solution for coupling between SH-PEG(400Da)-Fab, derived from csF001-5Fab-Qtag, and maleimide-modified ALP.
Fig. 40 is an SEC chart illustrating analytical results of a TG-catalyzed reaction product formed between csF001-5Fab-Qtag and the SH-PEG-NH₂ linker (1K) (Creative PEGWorks).
Fig. 41 is an SEC chart illustrating analytical results of a reaction solution for coupling between SH-PEG(1K)-Fab, derived from csF001-5Fab-Qtag, and maleimide-modified ALP.

### DETAILED DESCRIPTION OF THE PREFERRED EMBODIMENTS

A labeled polypeptide according to the invention has a glutamine residue that includes a side chain. The side chain is represented by formula (I) above. In the labeled polypeptide, a glutamine residue in the polypeptide, before the label is added, is bound to the label through a bifunctional linker having a PEG chain. More specifically, the labeled polypeptide is obtainable by bonding a PEG chain, as a bifunctional linker, having a thiol group and an amino group, to a glutamine residue of the polypeptide, making use of a TG-catalyzed reaction, and then reacting the thiol group of the linker with a label having a maleimide group (for details, see the production method for a labeled polypeptide, described later).

In formula (I), (C) represents the α-carbon of the glutamine residue in the labeled polypeptide. The side chain represented by formula (I) is structured to contain a linker and a label, which are bound to the side chain of the glutamine residue. The labeled polypeptide of the invention is a polypeptide in which a glutamine residue, which can be a substrate for TG, in the polypeptide before the label is added, is modified to the glutamine residue having the side chain represented by the above formula (I). That is, the amino acid sequence of the labeled polypeptide is the same as that of the polypeptide before the label is added, except for containing the side chain-containing glutamine residue represented by formula (I) above. The type of the polypeptide in the labeled polypeptide is not particularly limited. For example, the labeled polypeptide may be any protein and may be or comprise an antibody, antigen, ligand, or receptor. Among them, a polypeptide which is or comprises (as a fusion polypeptide) an antibody is preferred.

The term "antibody" in this specification encompasses antibody fragment. The antibody fragment is exemplified by Fab, Fab', F(ab')₂, Fd, Fd', Fv, scFv, domain antibody (dAb), reduced IgG (rIgG), light chain, heavy chain antibody, variable domain of heavy chain antibody (VHH), diabody, and triabody. The antibody may be either monoclonal antibody or polyclonal antibody. The antibody may be derived from any origin not specifically limited, typically from animals such as mouse, rat, hamster, rabbit, goat, horse, camel, alpaca, chicken, ostrich, and shark. Isotype of antibody may be any of IgG, IgA, IgM, IgD and IgE, among which IgG is preferred. The antibody that specifically binds to the tag may be a commercially available antibody, or an antibody produced by a method known in the art.

In the polypeptide before the label is added, the glutamine residue capable of serving as a substrate for TG may be, for example, a glutamine residue that resides on the surface of the polypeptide, with the carboxamide side chain directed outwards from the polypeptide. Alternatively, a peptide tag that contains a glutamine residue (also referred to as "Q-tag", hereinafter) may be added to the polypeptide before the label is added. The Q-tag contains a glutamine residue in a predetermined amino acid sequence, so that the glutamine residue can serve as a substrate for TG. Since not all glutamine residues in a given polypeptide can serve as the substrate for TG, the use of polypeptides to which a Q-tag is added is preferred. The Q-tag per se is known to the skilled person, as typically described in U.S. Patent Application Publication No. 2018/0037921. The number of amino acid residues contained in the Q-tag is preferably 3 or larger, and more preferably 4 or larger. The number of amino acid residues contained in the Q-tag is preferably 20 or smaller, more preferably 15 or smaller, and most preferably 10 or smaller. One or two residues in the amino acid sequences of the Q-tag are glutamine residues. The C-terminal amino acid residue of the Q-tag is preferably a proline residue, from the viewpoint of protecting the Q-tag from being cleaved by peptidase. The Q-tag is exemplified by peptide tags having amino acid sequences such as GVLNLAQSP (SEQ ID NO: 1), GLLQGP (SEQ ID NO: 2), or LLQGP (SEQ ID NO: 3).

The position to which the Q-tag is added in the polypeptide before the label is added, is preferably, but not restrictively, the N-terminal or C-terminal of the polypeptide, and more preferably the C-terminal of the polypeptide. Where the polypeptide comprises a full-length antibody such as Fab, Fab', or F(ab')₂, the Q-tag may be added to the C-terminal of the heavy chain or the light chain. The method for adding the Q-tag to the polypeptide is not particularly limited. For example, a crosslinker or a linker may be used to covalently attach the Q-tag to the polypeptide. Alternatively, a fusion polypeptide of the polypeptide and the Q-tag may be produced by a known genetic recombination method.

The labeled polypeptide according to the invention is thus preferably a fusion polypeptide formed between a polypeptide and a peptide tag that contains the glutamine residue having the side chain represented by formula (I) above. That is, the labeled polypeptide is a Q-tag-added polypeptide, in which a glutamine residue, which can be a substrate for TG, in the Q-tag is modified to the glutamine residue having the side chain represented by formula (I) above. More preferably, the labeled polypeptide is a fusion polypeptide formed between an antibody, and the peptide tag that contains the glutamine residue having the side chain represented by formula (I) above.

In formula (I) above, "(C)-(CH₂)₂-(C=O)-" is attributable to a carboxamide side chain of glutamine residue in the polypeptide, and "-NH-X-Y-S-" is attributable to a bifunctional linker, that is a PEG chain having a thiol group and an amino group. Here, X represents a straight chain alkylene group, whose number of carbon atoms is preferably 2 or larger and 10 or smaller, more preferably 2 or larger and 8 or smaller, and most preferably 2 or larger and 6 or smaller. The straight chain alkylene group, represented by X, preferably has no substituent. More preferably, X represents an unsubstituted straight chain alkylene group having 2 or more and 6 or less carbon atoms.

In formula (I) above, Y represents a PEG chain having a molecular weight of 1100 or larger. Since being a polymer, the PEG having a thiol group and an amino group, which is used for preparing the labeled polypeptide, may be an assemblage of multiple molecules whose molecular weight distributes over a certain range. Hence, the labeled polypeptides prepared from such PEG will have the molecular weight which distributes over a certain range. In this specification, the phrase "the PEG chain having a molecular weight of 1100 or larger" means that the minimum value of the molecular weight of the PEG chain moiety in the labeled polypeptide is 1100. The PEG chain represented by Y has a molecular weight whose lower limit is preferably 1200, 1300, 1400 or 1500. The PEG chain represented by Y has a molecular weight whose upper limit is typically 20000, 10000, 7500, 7000, 6500, 6000 or 5500. The molecular weight of the PEG chain may be determined typically by analyzing, by mass spectrometry, the PEG chain as a bifunctional linker having a thiol group and an amino group, before being bound to the polypeptide. The mass spectrometry is exemplified by infusion MS, LC-MS, time-of-flight mass spectrometry (TOF-MS), and matrix-assisted laser desorption/ionization mass spectrometry (MALDI-MS). Conditions for mass spectrometry may be appropriately determined. For example, conditions for infusion MS, when applied, may be those described later in EXAMPLE.

Among the molecular weights of the PEG chain measured by mass spectrometry, the molecular weight that appears in the largest abundance is referred to as "mode molecular weight". The PEG chain represented by Y has a molecular weight, typically with the lower limit of 1200, 1250, 1300, 1350, 1400, 1450, 1500, 1550, 1600, 1650, 1700, 1750, 1800, 1850, 1900, 1950, 2000 or 2050. The PEG chain represented by Y has a molecular weight typically with the upper limit of 18000, 15000, 12000, 9000, 8000, 7000, 6000, 5000, 4000, 3500 or 3000. Also the weight average molecular weight of the PEG chain may be estimated on the basis of the result of mass spectrometry. For example, in a case where the PEG chain as a bifunctional linker having a thiol group and an amino group, and before being bound to the polypeptide, is analyzed by Infusion MS, the weight average molecular weight of the PEG chain may be estimated from the molecular weight value and intensity of an isotopic molecular peak that appears highest in intensity. Conditions for analysis by infusion MS will be described later in EXAMPLES. The PEG chain represented by Y preferably has a weight average molecular weight of 1300 or larger, and more preferably 1700 or larger. The PEG chain represented by Y has a molecular weight, typically with the lower limit of 1350, 1400, 1450, 1500, 1550, 1600, 1650, 1700, 1750, 1800, 1850, 1900, 1950, 2000 or 2050. The PEG chain represented by Y preferably has an upper limit value of the molecular weight of smaller than 20000, which is typically 15000, 12000, 10000, 8000, 7000, 6000, 5000, 4500, 4000, 3500, 3000 or 2500.

The structure of Y is represented by formula (III) below. In formula (III), n represents an integer of 25 or larger, which is preferably 39 or larger.

-(OCH₂CH₂)ₙ- or - (CH₂CH₂O)ₙ- (III)

In formula (III), the lower limit of n is preferably 25, 26, 27, 28, 29, 30, 31, 32, 33, 34, 35, 36, 37, 38, 39, 40 or 41. More preferably, the lower limit of n is 31, 32, 33, 34, 35, 36, 37, 38, 39 or 40. The upper limit of n is typically 455, 228, 171, 159, 147, 136, 125, 113, 102, 90, 79, 68, or 56.

In the side chain represented by formula (I) above, a structural moiety represented by formula (IV) below is derived from a label having a maleimide group. Z represents a label, and L represents a spacer that binds the nitrogen atom of the imide ring to Z, or an atomic bond. The atomic bond represented by L means a bond through which the nitrogen atom of the imide ring is directly bound to Z, without any other atom interposed in between.

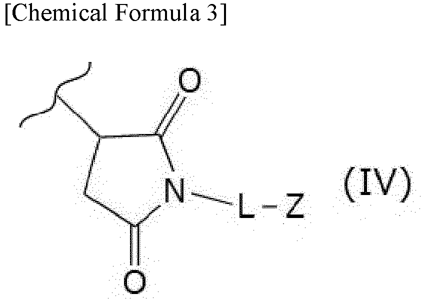

The label is a substance that can be added to a polypeptide, and is synonymous to "labeling substance". The label may be a substance that is specifically detectable, a substance capable of generating some signal by itself (also referred to as "signal generating substance", hereinafter), or a substance that catalyzes reaction of some other substance to induce signal generation. The substance that is specifically detectable is not particularly limited, as long as any target that can be specifically bound by such substance is present or obtainable. The substance that is specifically detectable is exemplified by biotins, hapten, and oligonucleotide. The biotins specifically bind to avidins. The hapten is exemplified by 2,4-dinitrophenyl (DNP) group. DNP specifically binds to anti-DNP antibody. The oligonucleotide specifically binds to an oligonucleotide having a sequence complementary to the base sequence of the former.

In this specification, the term "biotins" encompasses biotin and biotin analog. The biotin analog is exemplified by desthiobiotin and biocytin. In this specification, the term "avidins" encompasses and avidin and avidin analog. The avidin analog is exemplified by streptavidin, avidin-like protein derived from Paecilomyces tenuipes (Tamavidin (registered trademark)), bradavidin, and rhizavidin.

The signal generating substance is exemplified by fluorescent substance, radioisotope, and chemiluminescent substance. The substance that catalyzes reaction of some other substance to induce signal generation is exemplified by enzyme. The enzyme reacts with an appropriate substrate to generate a signal such as light or color. The enzyme is exemplified by alkaline phosphatase (ALP), peroxidase, β-galactosidase, and luciferase. The fluorescent substance is exemplified by fluorescent dyes such as Alexa Fluor (registered trademark), fluorescein isothiocyanate (FITC), and rhodamine; and fluorescent protein such as GFP. The radioisotope is exemplified by ¹²⁵I, ¹⁴C, ³²P, ^{99m}Tc, and ²²⁵Ac. The chemiluminescent substance is exemplified by ruthenium pyridine complex and acridinium ester. Since it is difficult to bind the radioisotope per se as a label, a possible way is to use a compound, which contains the radioisotope and a maleimide group, as the label. Such compound is exemplified by nucleic acid, saccharide, and oligopeptide that contain ¹²⁵I, ¹⁴C, or ³²P, and have a maleimide group added thereto. Alternatively usable is a maleimide derivative, which has a maleimide group bound to a chelating agent capable of coordinating to a metal element such as ^{99m}Tc or ²²⁵Ac. With the chelating agent moiety coordinated typically to ^{99m}Tc or ²²⁵Ac, the maleimide derivative is usable as the signal generating substance. The chelating agent is exemplified by deferoxamine.

Preferred labels include biotins, enzyme, fluorescent dye, fluorescent protein and hapten. ALP is particularly preferred as the enzyme.

In formula (I) above with L representing a spacer that binds the nitrogen atom of the imide ring to Z, L is typically exemplified by -(CH₂)ₙ-R-(C=O)-NH-, -(CH₂)ₙ-R-NH-(C=O)-, -(CH₂)ₙ-R-(C=O)-, -(CH₂)ₙ-R-(C=O)-O-, -(CH₂)ₙ-R-O-(C=O)-, -(CH₂)ₙ-R-(C=S)-NH-, -(CH₂)ₙ-R-NH-(C=S)-, -(CH₂)ₙ-R-O-, -(CH₂)ₙ-O-R-, -(CH₂)ₙ-R-S- or - (CH₂)ₙ-S-R-. n Represents an integer of 1 or larger and 10 or smaller.

Each R independently represents an atomic bond, an optionally substituted alkylene group having 1 to 10 carbon atoms, an optionally substituted arylene group or heteroarylene group having 6 to 12 carbon atoms, an optionally substituted cycloalkylene group or heterocycloalkylene group having 3 to 8 carbon atoms, and any combination of these components. The atomic bond represented by R means a bond enabling direct bonding without any other atom interposed in between.

R, when representing an alkylene group having 1 or more and 10 or less carbon atoms, is exemplified by alkylene groups such as methylene, ethylene, propylene, isopropylene, butylene, isobutylene, pentylene, neopentylene, hexylene, heptylene, octylene, 2-ethylhexylene, nonylene, and decylene groups. Among them, alkylene group having 1 or more and 4 or less carbon atoms is preferred. In a case where R represents an alkylene group having a substituent, the aforementioned number of carbon atoms does not include the number of carbon atoms of the substituent.

In a case where R represents an arylene group or a heteroarylene group, such group may only be an aromatic ring having 6 or more and 12 or less carbon atoms, which may contain one or more heteroatoms selected from N, S, O and P. Such group is exemplified by phenylene, naphthylene, biphenylylene, furanylene, pyrolene, thiophenylene, triazolene, oxadiazolene, pyridylene, and pyrimidylene groups. In a case where R represents an arylene group or a heteroarylene group having a substituent, the aforementioned number of carbon atoms does not include the number of carbon atoms of the substituent.

In a case where R represents a cycloalkylene group or heterocycloalkylene group, such group may only be a non-aromatic ring having 3 or more and 8 or less carbon atoms, which may contain one or more heteroatoms selected from N, S, O and P. Such group is exemplified by cyclopropylene, cyclobutylene, cyclopentylene, cyclohexylene, cycloheptylene, cyclooctylene, pyrrolidinylene, piperidinylene, piperazinylene, and morpholinylene. In a case where R represents a cycloalkylene group or a heterocycloalkylene group having a substituent, the aforementioned number of carbon atoms does not include the number of carbon atoms of the substituent.

The substituent in R is exemplified by hydroxy, cyano, alkoxy, nitro, =O, =S, halogen, haloalkyl, heteroalkyl, carboxyalkyl, amine, amide, and thioether groups. R may have multiple substituents. The alkoxy group is represented by "-O-alkyl group", wherein the alkyl group is a straight chain or branched saturated aliphatic hydrocarbon group having 1 or more and 5 or less carbon atoms, and more preferably having 1 or 2 carbon atoms.

One labeled polypeptide molecule may have one, or two or more side chain-containing glutamine residues represented by formula (I) above. Alternatively, two or more polypeptide molecules may share one label molecule, with each polypeptide structured to form the side chain represented by formula (I) above. In this case, the labeled polypeptide of formula (I) includes multiple structures, each having "(C)-(CH₂)₂-(C=O)-NH-X-Y-S-" and a moiety of formula (IV) coupled thereto, is bound to one Z. An exemplary labeled polypeptide is represented by formula (V) below. In formula (V), the definitions of (C), X, Y, Z, and L are same as those in formula (I).

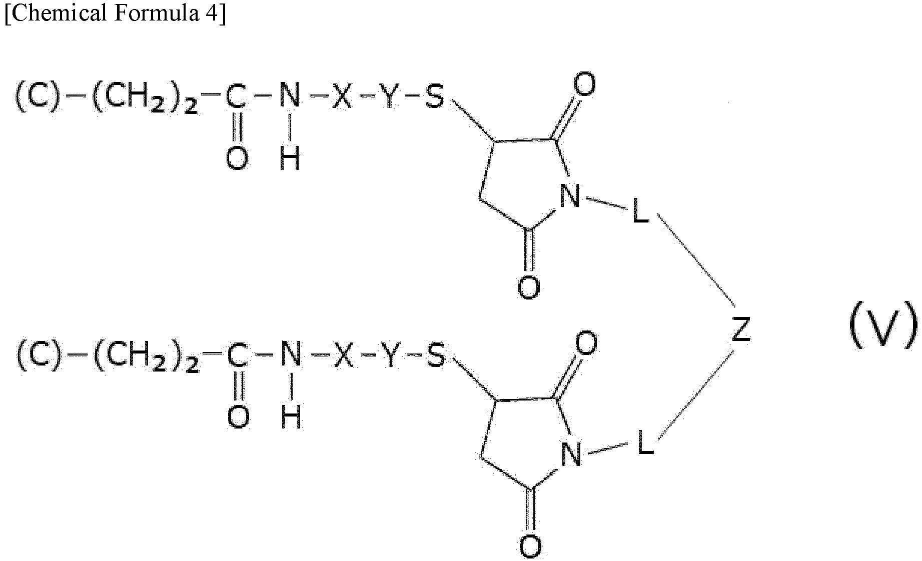

The complex represented by formula (V) is formed of one label molecule having two maleimide groups, to which two modified polypeptide molecules described later are bound. The complex represented by formula (V) is an example of the labeled polypeptide represented by formula (I). The labeled polypeptide may alternatively be a complex formed of one label molecule having three or more maleimide groups, to which three or more modified polypeptide molecules are bound. This sort of complex is prepared by binding one label molecule having multiple maleimide groups, to multiple polypeptide molecules, while individually interposed by a PEG chain having a thiol group and an amino group.

In the labeled polypeptideaccording to the invention, the polypeptide and the label are spaced by the PEG chain having a molecular weight of 1100 or larger. In an exemplary case where the polypeptide is an antibody, an advantage is that the label is prevented from non-specifically binding to the antibody. In an exemplary case where the polypeptide is an antibody, the antibody can swing owing to flexibility of the PEG chain having a molecular weight of 1100 or larger, yielding an advantage that the antibody will be more likely to capture an antigen, that is, a target substance. The labeled polypeptide according to the invention having an antibody as the polypeptide, when used for detecting antigen in serum, is also advantageous in terms of suppressing the background, as described later in Example 8 and the results thereof. This is supposedly because the PEG chain having a molecular weight of 1100 or larger can hydrate in a water-containing sample, so as to suppress any serum-derived contaminant from nonspecifically binding to the labeled polypeptide.

The modified polypeptide according to the invention has the side chain-containing glutamine residue represented by formula (II) above. In the modified polypeptide, the glutamine residue of the polypeptide is bound to the PEG chain having a thiol group. More specifically, the modified polypeptide is obtainable by bonding the PEG chain, as a bifunctional linker, having a thiol group and an amino group, to the glutamine residue of the polypeptide, making use of a TG-catalyzed reaction (for details, see the production method for the modified polypeptide, described later).

Upon contacting the modified polypeptide with the label having a maleimide group, the thiol group of the modified polypeptide reacts with the maleimide group of the label, to produce the labeled polypeptide. That is, the modified polypeptide is an intermediate for obtaining the labeled polypeptide of the present invention as the final product.

In formula (II), (C) represents the α-carbon of the glutamine residue in the modified polypeptide. The side chain represented by formula (II) is structured to contain the bifunctional linker, having a structure of PEG chain with a thiol group and an amino group, bound to the side chain of the glutamine residue. The modified polypeptide according to the invention is a polypeptide in which a glutamine residue, which can be a substrate for TG, in the polypeptide before the linker is bound, is modified to the side chain-containing glutamine residue represented by formula (II) above. That is, the amino acid sequence of the modified polypeptide is the same as that of the polypeptide before the linker is bound, except for having the side chain-containing glutamine residue represented by formula (II) above. The type of polypeptides envisaged for the modified polypeptide is not particularly limited, and can be the same as those described for the labeled polypeptide. That is, the modified polypeptide may be or comprise any protein such as an antibody, antigen, ligand, or receptor. Among them, antibody is preferred.

Details of X and Y in formula (II) are the same as those described for the labeled polypeptide. One modified polypeptide molecule may have one, or two or more side chain-containing glutamine residues represented by formula (II) above.

As has been widely known, the thiol group is susceptible to oxidation, and easily forms a disulfide bond between molecules having thiol groups. Such formation of the disulfide bond is avoidable by protecting the thiol group of the modified polypeptide, with a protective group such as acetyl group, when the labeled polypeptide is prepared. On the other hand, the terminal thiol group in the modified polypeptide is much less susceptible to oxidation, so that an advantage is that such thiol group is almost unlikely to form the disulfide bond between the modified polypeptides. This is supposedly because the modified polypeptide has enhanced dispersibility due to hydration of the PEG chain having a molecular weight of 1100 or larger, thus inhibiting contact between the thiol groups. In light of this advantage, it is not always necessary to preliminarily protect the thiol group in the modified polypeptide. Accordingly, the use of the modified polypeptide according to the invention will make operations, such as blocking and deblocking of the thiol group no longer necessary, and will simplify preparation process of the labeled polypeptide.

Another related aspect of the present invention relates to a production method for the modified polypeptide. In this production method, first, the polypeptide that contains the glutamine residue is contacted with the linker represented by formula (VI) above, in the presence of TG, thereby binding the linker to the carboxamide side chain of the glutamine residue.

TG per se is a known enzyme, and is commercially available. TG may be a natural enzyme extracted and purified from a living body or a biological sample, or may be an enzyme obtained by genetic recombination. The origin of TG may be any organism, without special limitation. TG derived from microorganism (Streptomyces mobaraensis, for example) is preferably used.

The polypeptide is not particularly limited as long as it contains a glutamine residue that can serve as a substrate for TG. The polypeptide is selectable typically from any protein such as proteins which is or comprise an antibody, antigen, ligand, or receptor. Among them, antibody is preferred. The polypeptide, having a glutamine residue that can serve as a substrate for TG, may be a fusion protein, i.e. a polypeptide having the Q-tag attached thereto. Preferred polypeptide is a fusion polypeptide of antibody and the Q-tag.

The linker represented by formula (VI) above is a bifunctional linker which is structured as a PEG chain having a thiol group and an amino group. Details of X and Y in formula (VI) are the same as those described for X and Y in formula (I), in relation to the labeled polypeptide according to the invention. The linker represented by the formula (VI) per se is known in the art and commercially available. The molecular weight of the PEG chain may be determined by mass spectrometry. Details of the mass spectrometry and the conditions thereof are as described above. In a case where a commercially available linker is used, the molecular weight of the PEG chain may be a value disclosed by the manufacturer or supplier. The molecular weight of the linker, disclosed is this case, is preferably 2000 or larger. The examples disclose specific examples of suitable linkers which can be used in the context of the present invention including in embodiments different from those detailed in the examples.

The linker represented by formula (VI) above has improved dispersibility due to hydration of the PEG chain having a molecular weight of 1100 or larger, thus supposedly making the thiol groups less likely to contact to each other. Hence, the terminal thiol group of the linker is less susceptible to oxidation, so that the modified polypeptides are almost unlikely to form a disulfide bond in between. It is therefore unnecessary to preliminarily block the thiol group of the linker, in the step of binding the polypeptide to the linker represented by formula (VI). The reaction between the modified polypeptide and the label having a maleimide group can proceed quantitatively and efficiently, since the thiol group is hardly lost by formation of disulfide bond.

The linker represented by formula (VI) may have a free form, or a salt form with an inorganic acid (hydrochloric acid, for example). The linker represented by formula (VI) is preferably a salt of an inorganic acid (hydrochloric acid, for example). As described above, the linker even in the free from is less likely to form a disulfide bond in between, due to the PEG chain having a molecular weight is 1100 or larger. If given as a salt with an inorganic acid, the linker will have improved dispersibility not only due to hydration of the PEG chain, but also due to electrostatic interaction.

In the presence of TG, the glutamine residue-containing polypeptide is preferably contacted with the linker represented by formula (VI) in a suitable aqueous medium. For example, mixing of a TG solution, a polypeptide solution, and a linker solution can contact these components. Sequential order of the mixing is not particularly limited. The aqueous medium is exemplified by water, physiological saline, and buffer. The buffer is exemplified by phosphate-buffered saline, Tris-HCl, and Good's buffer. In a case where microorganism-derived TG is used, the aqueous medium preferably has the pH around neutrality (pH 6.5 or higher and pH 8.5 or lower), which is more preferably pH 8 or higher and 8.5 or lower. An aqueous solvent that contains dimethyl sulfoxide (DMSO) is also acceptable. TG-containing aqueous solvent preferably has a DMSO concentration of 20 w/v% or lower, which is more preferably 10 w/v% or lower.

In such contact, the solution that contains TG, polypeptide and linker as a result of mixing is preferably incubated under conditions that the TG-catalyzed reaction can proceed. Such conditions per se have been known. For example, the temperature is 4°C or higher and 37°C or lower, and preferably 20°C or higher and 37°C or lower. The incubation time is typically one hour or longer and 24 hours or shorter, although suitably selectable according to the temperature. The contact if conducted under low temperatures, such as 10°C or lower, is preferably kept for a longer time (8 hours or longer, for example). During the contact, the amino group of the carboxamide side chain of the glutamine residue in the polypeptide is exchanged with the amino group in the linker, while catalyzed by TG, to form an amide bond, whereby the linker is bound to the side chain. The modified polypeptide according to the invention is thus produced.

Next, the modified polypeptide thus produced by the binding of the carboxamide side chain and the linker is acquired. For example, the solution that contains TG, the polypeptide, and the linker after the enzymatic reaction may be purified by SEC or affinity chromatography with use of an appropriate column, to remove the enzyme and any unreacted component, thus acquiring the modified polypeptide. The unreacted component is typically the fraction of polypeptide that remained unbound to the linker, and the fraction of the linker that remained unbound to the polypeptide. The solution that contains the thus preparative modified polypeptide may be desalted and concentrated as necessary.

Another related aspect of the present invention relates to a production method for the labeled polypeptide. In this production method, first, the polypeptide that contains the glutamine residue is contacted with the linker represented by formula (VI) above, in the presence of TG, thereby binding the linker to the carboxamide side chain of the glutamine residue. Next, the modified polypeptide thus produced by the binding of the carboxamide side chain and the linker is acquired. Details of these processes are the same as those described for the preparation method for the modified polypeptide. Also details of the label are the same as those described for the labeled polypeptide.

The thus preparative modified polypeptide is contacted with the label having a maleimide group, thereby bonding the label to the linker bound to the modified polypeptide. Since the modified polypeptide has the side chain represented by formula (II) above, so that the maleimide group of the label is bound to the thiol group of the side chain. This forms a thioether bond, thus binding the label to the terminal of the linker bound to the modified polypeptide.

The maleimide group-containing label preferably has a structure represented by formula (VII) below. In formula (VII), Z represents a label, L represents a spacer or an atomic bond, and R¹ and R² identically or differently represent a hydrogen atom or a bromine atom. Preferably, both of R¹ and R² individually represent a hydrogen atom, or a bromine atom. Details of L and Z are the same as those described for formula (I) that represents the labeled polypeptide according to the invention. Given R¹ and/or R² represent a bromine atom, the label whose structure is represented by formula (VII) below is a label having a maleimide derivative, that is, a 3,4-dibromomaleimide group or a 3-bromomaleimide group. In this specification, the term "maleimide group" encompasses 3,4-dibromomaleimide group and 3-bromomaleimide group.

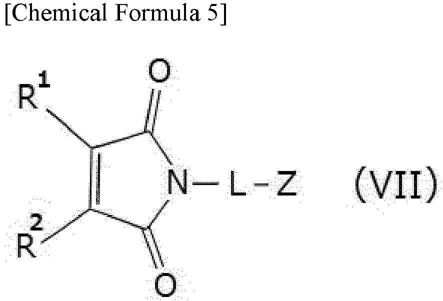

The label having the structure represented by formula (VII) above may be prepared by binding the label with the bifunctional linker that has a maleimide group and a predetermined reactive group. Such bifunctional linker per se is known in the art, and is commercially available. The predetermined reactive group may be appropriately determined according to the functional group owned by the label. In an exemplary case where the label has an amino group, employable is a bifunctional linker that has an N-hydroxysuccinimide (NHS) ester and a maleimide group. In this case, the bifunctional linker binds to the label as a result of the reaction between the amino group of the label, with the NHS ester of the linker, whereby the label having a structure represented by formula (VII) is obtainable. The label having the structure represented by formula (VII) may alternatively be a commercially available label.

The label having a 3,4-dibromomaleimide group as the maleimide group may be prepared typically according to the reaction scheme below (see Morais M., et al., Bioconjugation, Methods and Protocols (Methods Mol. Biol., 2019, 2033, SpringerLink), Chapter 2, pp. 15-24). In the reaction scheme below, a label having a structure represented by formula (VIII) is obtainable as the label having a maleimide group. In the reaction scheme and formula (VIII) below, AcOH represents acetic acid, DBM-C₂-acid represents an intermediate, EEDQ represents 1-ethoxycarbonyl-2-ethoxy-1,2-dihydroquinoline as a condensing agent, MeCN represents acetonitrile, and Z represents the label.

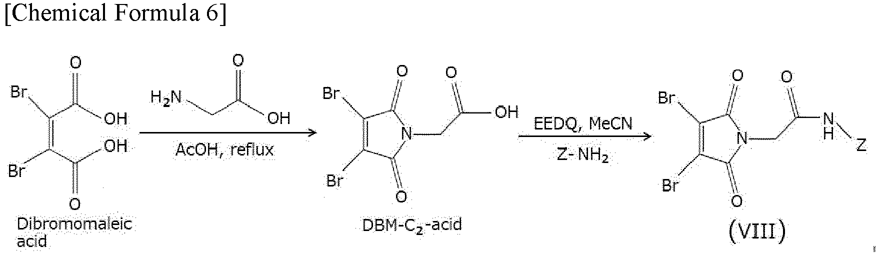

One 3,4-dibromomaleimide group can react with each of two thiol groups to form a thioether bond. Hence, use of the label having a 3,4-dibromomaleimide group typically enables one label molecule to bind two modified polypeptide molecules. That is, the modified polypeptide is dimerized while interposed by the 3,4-dibromomaleimide group, thereby obtaining a complex of two labeled polypeptide molecules. In an exemplary case where two modified polypeptide molecules are bound to one label molecule having the structure represented by formula (VIII), obtainable is a complex of the labeled polypeptide represented by formula (IX) below. In formula (IX), definitions of (C), X, Y, Z, and L are synonymous to those in formula (I).

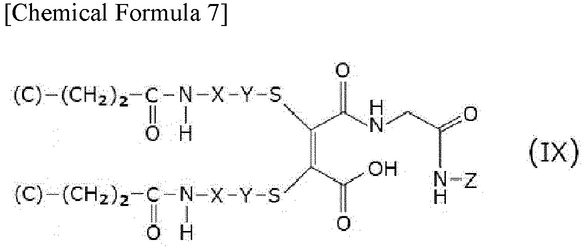

The modified polypeptide is preferably contacted with the label having a maleimide group, in a suitable aqueous medium. For example, mixing of a modified polypeptide solution, and a maleimide group-containing label solution can contact these components. The aqueous medium is preferably buffer, which is exemplified by triethanolamine buffer, PBS, Tris-HCl, and Good's buffer. pH of the aqueous medium is preferably 6.5 or higher and 7.5 or lower.

In such contact, the solution that contains the modified polypeptide and the label as a result of mixing is preferably incubated under conditions that the reaction between the thiol group and the maleimide group can proceed. Such conditions per se have been known. For example, the temperature is 5°C or higher and 37°C or lower, and preferably 20°C or higher and 30°C or lower. The incubation time is typically 2 hours or longer and 6 hours or shorter, although suitably selectable according to the temperature. The contact if conducted under low temperatures, such as 10°C or lower, is preferably kept for a longer time (16 hours or longer, for example). As a result of such contact, the thiol group of the modified polypeptide and the maleimide group of the label form a thioether bond, to produce the labeled polypeptide.

Then, the labeled polypeptide thus produced by the binding of the modified polypeptide and the label is acquired. For example, the solution that contains the modified polypeptide and the label after the enzymatic reaction may be purified by SEC with use of an appropriate column, to remove any unreacted component, thus acquiring the labeled polypeptide. The unreacted component is typically the label remained unbound to the modified polypeptide, or the modified polypeptide remained unbound to the label. The solution that contains the thus preparative labeled polypeptide may be desalted and concentrated as necessary.

In another embodiment, a label having a haloacetyl group may be used in place of the label having a maleimide group, to label the modified polypeptide (see Greg T. Hermanson, Bioconjugate Techniques Third Edition, Academic Press, Chapter 3 The reaction of Bioconjugation, pp. 240-241, 2. Thiol reactions, 2.1 Haloacetyl and alkyl halide derivatives). For example, in a case where the modified polypeptide and a label having a haloacetyl group represented by formula (X) (where, R³ represents a bromine atom or an iodine atom) are bound as represented by the reaction scheme below, obtainable is a labeled polypeptide represented by formula (XI) below. In the reaction scheme below, definitions of (C), X, Y, Z, and L are synonymous to those in formula (I).

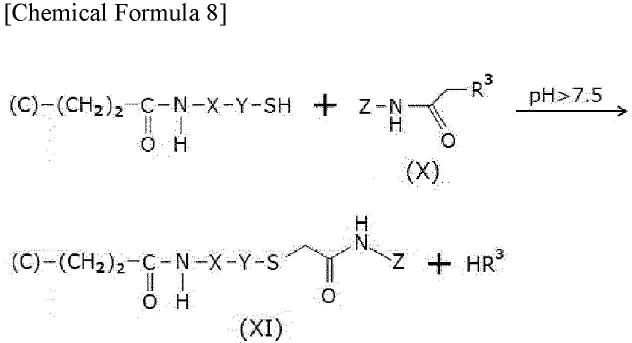

Yet another related aspect of the invention relates to a reagent that contains the labeled polypeptide or the modified polypeptide (also referred to as "reagent of the invention", hereinafter). In a case where the polypeptide is or comprises an antibody, the reagent according to the invention that contains the labeled polypeptide, is used for the measurement method of this embodiment described later. The reagent according to the invention that contains the modified polypeptide is used for a reaction for binding the modified polypeptide to any maleimide group-containing label, to obtain the labeled polypeptide. Details of the labeled polypeptide and the modified polypeptide are the same as those described above.

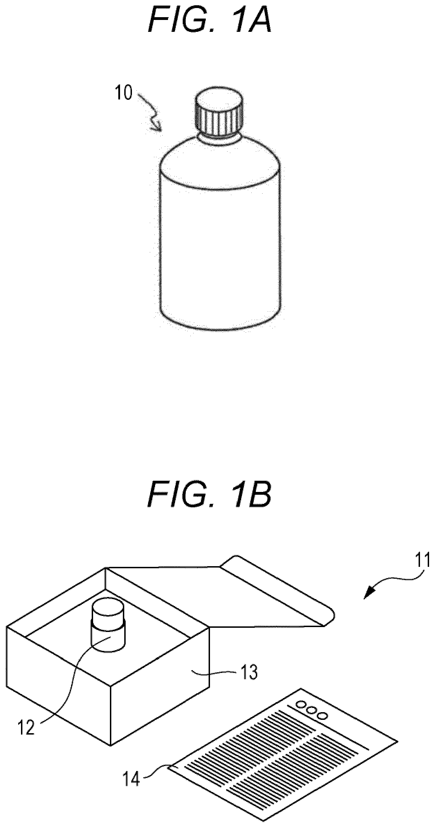

The reagent according to the invention may be provided to the user, while enclosing the labeled polypeptide or the modified polypeptide in a vial. An exemplary reagent is shown in Fig. 1A. Referring to Fig. 1A, reference sign 10 denotes a reagent enclosed in the vial. The labeled polypeptide or the modified polypeptide in the reagent may be solid (powder, crystal or freeze-dried product, for example), or may be liquid (solution, suspension or emulsion, for example). In a case where the labeled polypeptide or the modified polypeptide in the liquid form is contained in the reagent, the solvent is exemplified by the aforementioned aqueous media. Any stabilizer such as casein or BSA may be added to the aqueous medium, as necessary.

Yet another aspect relates to a reagent kit having the reagent that contains the labeled polypeptide or the modified polypeptide. In a case where the polypeptide is an antibody, the reagent kit, having the reagent that contains the labeled polypeptide, is used for the measurement method according to the invention described later. The reagent kit, having the reagent that contains the modified polypeptide, is used for a reaction of binding the modified polypeptide to any maleimide group-containing label, to obtain the labeled polypeptide. Details of the labeled polypeptide and the modified polypeptide are the same as those described above.

The reagent kit may be provided to the user, while packaging the vial that contains the labeled polypeptide or the modified polypeptide, in a box. The box may include a package insert. The package insert may contain descriptions on a reagent composition, a structure of the labeled polypeptide or modified polypeptide, use instructions of the reagent, and storage instructions of the reagent. An exemplary reagent kit is shown in Fig. 1B Referring now to Fig. 1B, reference sign 11 denotes a reagent kit according to the invention, reference sign 12 denotes a vial that contains the labeled polypeptide or the modified polypeptide, reference sign 13 denotes a packaging box, and reference sign 14 denotes a package insert.

Yet another aspect relates to a measurement method for a target substance with use of the labeled polypeptide according to the invention (also referred to as "measurement method according to the invention "). The labeled polypeptide used in the measurement method according to the invention is a fusion polypeptide that is composed of an antibody, and a peptide tag that contains the side chain-containing glutamine residue represented by formula (I) above (also referred to as "labeled antibody according to the invention "). The labeled antibody according to the invention is an antibody having a label, capable of specifically binding to a target substance, and is used as a detection antibody in the measurement method according to the invention. The detection antibody refers to an antibody that binds to a target substance, and provides a signal detectable with the aid of the label. The label is preferably a substance that catalyzes reaction of a signal generating substance or other substance, to induce signal generation. The label is more preferably enzyme, fluorescent dye, or fluorescent protein. Details of the labeled polypeptide according to the invention are the same as described above.

In the measurement method according to the invention, first, an immune complex of the labeled antibody and the target substance is formed. The target substance may be a substance recognizable by the labeled antibody, or may be a formed element that contains such substance. The substance recognizable by the labeled antibody is exemplified by protein, oligopeptide, nucleic acid, lipid, glycan, and hapten. The formed element that contains the substance recognizable by the labeled antibody is exemplified by cell, extracellular vesicle, microorganism, virus, and fragment thereof. The extracellular vesicle is exemplified by exosome, ectosome, microvesicle, microparticle, and apoptotic body. The microorganism is exemplified by bacteria and fungi.

The immune complex may be formed by mixing a sample that presumably contains a target substance, with the labeled antibody. Type of the sample is exemplified by, but not particularly limited to, biological samples such as blood, plasma, serum, lymph, and saliva; excreta such as urine and feces; and environmental samples such as river water, seawater, and soil. The immune complex is preferably formed in a solution. The sample that presumably contains the target substance is therefore preferably in a liquid form. The liquid sample is not limited to solution, and encompass suspension and sol. The sample, not in liquid form, may be converted to liquid form, typically by adding an appropriate aqueous medium to the sample. Any insoluble contaminant in the liquid sample may optionally be removed, typically by a known technique such as centrifugation or filtration. The liquid sample may optionally be diluted with the aforementioned aqueous medium. The labeled antibody is preferably in liquid form prepared in a suitable aqueous medium. The aqueous medium is as described above.

When forming the immune complex, the immune complex of the labeled antibody and the target substance is preferably formed on a solid phase. For example, the sample that presumably contains the target substance is mixed with the labeled antibody to form the immune complex, and the solution that contains the immune complex is then contacted with the solid phase capable of immobilizing thereon the labeled antibody or the target substance. The immune complex may be thus formed on the solid phase. Alternatively, the target substance may be preliminarily immobilized on the solid phase, and the solid phase may be contacted with the labeled antibody to form the immune complex on the solid phase.

Conditions for mixing, such as temperature and incubation time, are not particularly limited as long as they are suitable for the antigen-antibody interaction. Such conditions per se have been known. For example, the temperature is 4°C or higher and 40°C or lower, and preferably 20°C or higher and 37°C or lower. The incubation time is typically one minute or longer and 24 hours or shorter, although suitably selectable according to the temperature. The contact if conducted under low temperatures, such as 10°C or lower, is preferably kept for a longer time (1 hour or longer, for example).

In the measurement method according to the invention, a capture antibody that specifically binds to the target substance may be used in addition to the labeled antibody. The capture antibody refers to an antibody that is immobilized per se on the solid phase, and eventually captures the target substance on the solid phase. The capture antibody preferably recognizes an epitope which is different from that recognizable by the labeled antibody. In a case where the target substance has multiple same epitopes, the capture antibody and the labeled antibody may recognize the same epitope. With such additional use of the capture antibody, there is formed a sandwich immune complex of the labeled antibody, the target substance, and the capture antibody. The sandwich immune complex means a complex that contains the capture antibody, the target substance, and the labeled antibody, in which the capture antibody and the labeled antibody are bound to different sites on the target substance.

The sandwich immune complex may be formed by mixing the capture antibody, the sample that presumably contains the target substance, and the labeled antibody. In a preferred embodiment, the sandwich immune complex, formed of the capture antibody, the target substance, and the labeled antibody, is formed on the solid phase. For example, the sample that presumably contains the target substance, the capture antibody, and the labeled antibody are mixed to form the immune complex, and the solution that contains the immune complex is then contacted with the solid phase capable of immobilizing thereon the capture antibody. The sandwich immune complex may be thus formed on the solid phase. Alternatively employable is the capture antibody preliminarily immobilized on the solid phase. That is, the sandwich immune complex may be formed on the solid phase, by mixing the capture antibody preliminarily immobilized on the solid phase, the sample that presumably contains the target substance, and the labeled antibody. Conditions for mixing, such as temperature and incubation time, are as described above.

The solid phase may only be an insoluble carrier on which the target substance or the capture antibody may be immobilized. The solid phase is preferably an insoluble carrier on which the capture antibody may be immobilized. For example, the capture antibody may be immobilized on the solid phase, as a result of direct or indirect binding between the solid phase and the capture antibody. The direct binding between the solid phase and the capture antibody is exemplified by adsorption to the surface of the solid phase attributable to hydrophobic interaction, or formation of a covalent bond. In an exemplary case where the solid phase is an ELISA microplate, the capture antibody may be immobilized by adsorption, inside a well of the plate. In a case where the solid phase has a functional group on the surface, the capture antibody may be immobilized on the surface of the solid phase through a covalent bond with use of the functional group. In an exemplary case where the solid phase is a particle having a carboxy group, the carboxy group on the particle surface is activated with 1-[3-(dimethylamino)propyl]-3-ethylcarbodiimide (WSC), and then reacted with NHS to form an NHS ester. Upon contact of the particle having the NHS ester with the capture antibody, the NHS ester reacts with the amino group of the capture antibody, whereby the capture antibody is covalently immobilized on the particle surface.

The indirect binding between the solid phase and the capture antibody is exemplified by binding while interposed by a molecule that is specifically binds to the capture antibody. By preliminarily immobilizing such molecule on the surface of the solid phase, the capture antibody may be immobilized on the solid phase. The molecule that specifically binds to the capture antibody is exemplified by protein A, protein G, and antibody (secondary antibody) that specifically recognizes the capture antibody. The capture antibody and the solid phase may alternatively be bound, making use of combination of substances interposed between the capture antibody and the solid phase. Such combination of substances is exemplified by combination of biotins and avidins, or, hapten and anti-hapten antibody. In an exemplary case where the capture antibody is preliminarily DNP-modified, use of the solid phase, having an anti-DNP antibody immobilized thereon, can immobilize thereon such capture antibody.

Material for composing the solid phase is selectable typically from organic polymer compound, inorganic compound, and biopolymer. The organic polymer compound is exemplified by latex, polystyrene, polypropylene, styrene-methacrylate copolymer, styrene-glycidyl (meth)acrylate copolymer, styrene-styrene sulfonate salt copolymer, methacrylate polymer, acrylate polymer, acrylonitrile-butadiene-styrene copolymer, vinyl chloride-acrylate ester copolymer, and polyvinyl acetate-acrylate copolymer. The inorganic compound is exemplified by magnetic substance (iron oxide, chromium oxide, cobalt, ferrite, etc.), silica, alumina and glass. The biopolymer is exemplified by insoluble agarose, insoluble dextran, gelatin and cellulose. Two or more of them may be used in combination.

Shape of the solid phase is exemplified by, but not specifically limited to, particle, microplate, microtube and test tube. Among them, particle and microplate are preferred, and magnetic particle is particularly preferred. The solid phase, when given in the form of particle, may be used while being suspended in liquid, as the solid for formation of the immune complex. The solid phase, when given in the form of container such as a microplate, the immune complex may be formed in the container as the solid phase. When using the magnetic particles capable of immobilizing thereon the capture antibody, the measurement method according to the invention may be conducted with a commercially available fully automated immunoassay system such as HISCL (registered trademark) series (Sysmex Corporation).

Next, a signal generated by the label contained in the immune complex is detected. The label is owned by the labeled antibody bound to the target substance. The signal generated by the label in the immune complex therefore corresponds to the amount of the target substance. In this specification, the phrase "detecting a signal" encompasses qualitatively detecting presence or absence of a signal, quantifying the signal intensity, and semi-quantitatively detecting signal intensity. The phrase "semi-quantitatively detecting signal intensity" means that the signal intensity is detected stepwise as "no signal", "weak", "medium" and "strong". Preferably, the intensity of the signal generated by the label contained in the immune complex is quantified to obtain a measured value.

Results of detection of the signal may be used as results of measurement of the target substance in the sample. When quantifying the signal intensity, the measured value of the signal intensity per se, or a value acquired from the measured value, may be used as the measured value of the target substance. The value acquired from the measured value of the signal intensity is exemplified by a value obtained by subtracting a measured value of a negative control, or a background value, from the measured value. The negative control is properly selectable, which is exemplified by a buffer solution free of the target substance.

The measured value of the signal intensity may be fitted on an analytical curve, to determine the amount or concentration of the target substance in the sample. The analytical curve may be created on the basis of measured values of multiple calibrators. The measured values of the calibrators are obtainable by measuring the calibrators according to the measurement method according to the invention, in the same way as for the sample. The analytical curve may be prepared by plotting the measured values of the plurality of calibrators on an X-Y chart, having concentration of the target substance in the calibrator scaled on the X-axis, and the measured value (signal intensity, for example) scaled on the Y-axis, and by fitting thereto a straight line or a curve according to a known technique such as the least square method. The calibrators may be prepared typically by adding any known concentrations of an isolated or synthesized target substance into a buffer free of the target substance. The calibrator free of the target substance may be a buffer per se which is free of the target substance.

The measurement method according to the invention may be conducted with a commercially available fully automated immunoassay system. The fully automated immunoassay system is an instrument that automatically starts, upon sample setting and entry of measurement start command by the user, preparation and immunoassay of a measurement sample, and outputs measurement results of the target substance. This sort of fully automated immunoassay system is exemplified by those in HISCL Series (Sysmex Corporation), such as HISCL (registered trademark)-5000, and HISCL-2000i. Measurement with the HISCL Series system relies upon the sandwich ELISA method, with use of a magnetic particle as the solid phase.

In this embodiment, B/F (bound/free) separation for removing any free component that remains unreacted may be interposed between formation of the sandwich immune complex and the signal detection. The free component that remains unreacted refers to a component that does not constitute the immune complex. This is exemplified by excessive capture antibody and detection antibody according to the invention, not having been bound to the target substance. Technique for the B/F separation is not specially limited. With the solid phase given a particle form, the B/F separation may be enabled by centrifugation, by which only the solid having the immune complex captured thereon is collectable. With the solid phase given as a container such as a microplate or a microtube, the B/F separation may rely upon removal of a liquid that contains the unreacted free component. With the solid phase given as a magnetic particle, the B/F separation may rely upon removal of a liquid that contains the unreacted free component, under suction through a nozzle, while magnetically restraining the magnetic particle with use of a magnet. The method is preferred from the viewpoint of automatization. After removing the unreacted free component, the solid phase having the immune complex captured thereon may be washed with a suitable aqueous medium, such as PBS.

With the label of the labeled antibody given as a fluorescent dye or fluorescent protein, the measurement method according to the invention may rely upon a flow cytometer. The "flow cytometer" in this specification encompasses an imaging flow cytometer (IFC). The IFC is a sort of flow cytometer equipped with an imaging unit such as a CCD camera. A flow cytometer not equipped with such imaging unit is structured to irradiate light on the individual formed elements in a liquid that flow through a flow cell, and to detect scattered light and/or fluorescence emitted from the individual formed elements as an optical signal. With the formed element given as cells, the IFC can typically measure the size of each cell, and distribution of the amounts of molecules on the cell surface or inside the cells. The IFC is an instrument capable of acquiring images of the individual formed elements in the liquid that flow through the flow cell. The IFC can quantitatively measure the formed elements within a time as short as several seconds to several minutes, by acquiring fluorescence signal, scattered light signal, fluorescence image, or bright field image (also referred to as a transmitted light image) from each of several thousands to several millions of formed elements. The image processing also enables extraction of information from the individual formed elements.

The measurement method according to the invention, with use of the flow cytometer, may be targeted at the formed element that contains the substance recognizable by the labeled antibody. When measured with the flow cytometer, the immune complex may be formed by mixing the sample that presumably contains the target substance, and the labeled antibody. In this way, the labeled antibody can bind to the target substance that resides on the formed element, thereby forming the immune complex on the formed element. Conditions for mixing, such as temperature and incubation time, are as described above.

The flow cytometer takes part in detection of a signal ascribed to the label contained in the immune complex. That is, the signal is detected by the flow cytometer, upon introduction of the immune complex into the flow cell of the flow cytometer. In the flow cytometer, each of the formed element having the immune complex formed thereon is irradiated with light, while passing through the flow cell. The flow cytometer then detects fluorescence signal emitted from the formed element. Since the measurement method according to the invention with use of the flow cytometer employs the fluorescent dye or the fluorescent protein as the label, so that detectable is a fluorescent signal emitted from the fluorescent dye or the fluorescent protein. Fluorescence information is then acquired according to the detected fluorescence signal. A scattered light signal may alternatively be detected from the formed element as necessary, and scattered light information may be acquired on the basis of the scattered light signal. The scattered light is exemplified by forward scattered light (scattered light typically at a light receiving angle of 0° to approximately 20°), and side scattered light (scattered light typically at a light receiving angle of approximately 20° to approximately 90°).

Light source of the flow cytometer is not particularly limited, and is typically selectable from those having a wavelength suitable for exciting the fluorescent dye or the fluorescent protein. The light source usable here may typically be semiconductor laser source, argon laser source, He-Ne laser light source, or mercury lamp.

The fluorescence information is exemplified by fluorescence pulse height, pulse width, pulse area, transmittance, Stokes shift, ratio, temporal change, and values correlated thereto. The scattered light information is exemplified by scattered light pulse height, pulse width, pulse area, transmittance, Stokes shift, ratio, temporal change, and values correlated thereto.

With the flow cytometer given as an IFC, the fluorescence information may typically be a fluorescent signal-based value acquired from an image captured by the IFC. In a fluorescence image of the particle captured by the IFC, each pixel that constitutes an area demonstrating the fluorescence signal has a pixel value that corresponds to the fluorescence signal intensity. Hence, a value based on the optical signal, acquired from the image, may typically be a value based on the pixel that constitutes the area demonstrating the fluorescence signal. Such value is exemplified by fluorescence intensity, maximum fluorescence intensity, total fluorescence signal intensity, and fluorescence signal area value. The "fluorescence intensity" is an average value of the pixel values of the pixels that constitute the area demonstrating the fluorescence signal, in the fluorescence image of the formed element that contains the immune complex. The "maximum fluorescence intensity" is the maximum value from among the pixel values of the pixels that constitute the area demonstrating the fluorescence signal, in the fluorescence image of the formed element that contains the immune complex. The "total fluorescence intensity" is the total of the pixel values of the pixels that constitute the area demonstrating the fluorescence signal, in the fluorescence image of the formed element that contains the immune complex. The "fluorescence signal area value" is the number of pixels that constitute the area demonstrating the fluorescence signal, in the fluorescence image of the formed element that contains the immune complex.

The formed element that contains the immune complex may also be detected, on the basis of the acquired fluorescence information. With the fluorescence information given as information indicated by numerical values such as the pulse peak of fluorescence, or the fluorescence intensity acquired from the fluorescence image, the fluorescence information of the formed element that contains the immune complex will have a value larger than the value of the fluorescence information of the formed element having no immune complex formed thereon. Hence, the measurement data of the formed element that contains the immune complex may be extracted, referring to a result of comparison between the value of the acquired fluorescence information and a predetermined threshold value. For example, a formed element whose value of the acquired fluorescence information is equal to or larger than the predetermined threshold value, may be detected as the formed element that contains the immune complex. The predetermined threshold value may be freely determined without special limitation. For example, a value of fluorescence information may be acquired by measuring a buffer solution free of the target substance, or a sample that contains the formed element already known to contain no target substance, by the measurement method according to the invention with use of the flow cytometer. The thus obtained value may be used as the predetermined threshold.

The present invention will be further detailed referring to EXAMPLES, to which the present invention is by no means limited.

### EXAMPLES

### [Materials and Methods]

Materials and methods used in Test Examples described later will be described below. Details of the method will also be described in the individual Test Examples.

### 1. Antibodies

### (1.1) Preparation of Q-tag-Attached Anti-p24 Fab Antibody

### (1.1.1) Establishment of Anti-p24 Antibody (sF001-5, sF028-22 and sF001-25)-Producing Hybridoma

An anti-p24 antibody (sF001-5, sF028-22, sF001-25)-producing hybridoma was established, while using a mouse (ddY; 5 weeks old, female) as a host, and recombinant HIV p24 protein as an antigen. That is, a mouse (ddY; 5 weeks old, female) was immunized with recombinant HIV p24 protein, with use of Freund's complete adjuvant (FCA) and incomplete Freund's adjuvant (FIA) as adjuvants. The mouse, after confirming increase in the blood antibody titer, was euthanized, and the spleen was then extracted by necropsy. The extracted spleen cells were fused with mouse myeloma cells by the PEG method, and hybridomas were selected on a HAT medium. The antibody secreted into the culture supernatant of each hybridoma was assayed by ELISA, to establish hybridomas that produce each of sF001-5, sF028-22 and sF001-25, as an anti-p24 antibody.

### (1.1.2) Preparation of sF001-5, sF028 -22 and sF001-25

sF001-5, sF028-22 and sF001-25 derived from the mouse hybridomas were prepared. That is, the thus established sF001-5-, sF028-22-, and sF001-25-producing hybridoma cells were cultured in an SFM medium to induce secretion of sF001-5, sF028-22, and sF001-25 into the medium, respectively. The culture supernatant, filtered through a filter to remove the cells, was subjected to column chromatography with use of Protein G carrier, to obtain each of purified sF001-5, sF028-22, and sF001-25 antibodies.

### (1.1.3) Preparation of Q-Tag-Attached Anti-p24 Fab Antibodies

Chimeric sF001-5 (abbreviated as "csF001-5", hereinafter), chimeric sF028-22 (abbreviated as "csF028 -22", hereinafter), and chimeric sF001-25 (abbreviated as "csF001-25", hereinafter) Fab antibodies were obtained by linking the hypervariable regions of sF001-5, sF028-22, and sF001-25, derived from the mouse hybridoma, with the constant region of human IgG1 κ isotype Fab. A Q-tag sequence was attached to the heavy chain C terminal of each of these chimeric Fab antibodies, to obtain tagged anti-p24 Fab antibodies (also referred to as "csF001-5Fab-Qtag", "csF028-22Fab-Qtag", and "csF001-25Fab-Qtag", hereinafter). Amino acid sequences of the constant region including the inserted Q-tag sequence, and base sequences that encode the same are listed below (with the Q-tag sequence underlined).

### Amino Acid Sequence of Light Chain

TVAAPSVFIFPPSDEQLKSGTASVVCLLNNFYPREAKVQWKVDNALQS
GNSQESVTEQDSKDSTYSLSSTLTLSKADYEKHKVYACEVTHQGLSSPVTKSFN
RGEC (SEQ ID NO: 4)

### Base Sequence of Light Chain

ACCGTCGCGGCGCCGTCGGTCTTTATCTTCCCGCCGAGCGATGAACA
ACTGAAATCTGGTACCGCGAGTGTGGTTTGTCTGCTGAACAATTTTTATCCG
CGTGAAGCGAAAGTCCAGTGGAAGGTGGACAACGCCCTGCAGTCTGGCAAT
AGTCAAGAATCCGTGACCGAACAAGATTCAAAAGACTCGACGTACAGCCTG
AGTTCCACCCTGACGCTGAGCAAGGCAGATTATGAAAAACATAAGGTGTAC
GCTTGCGAAGTTACCCACCAGGGTCTGTCCAGTCCGGTTACGAAATCATTCA
ACCGCGGCGAATGT (SEQ ID NO: 5)

### Amino Acid Sequence of Heavy Chain

ASTKGPSVFPLAPSSKSTSGGTAALGCLVKDYFPEPVTVSWNSGALTSG

VHTFPAVLQSSGLYSLSSVVTVPSSSLGTQTYICNVNHKPSNTKVDKKVEPKSC
GVLNLAQSP (SEQ ID NO: 6)

### Base Sequence of Heavy Chain

GCTAGCACAAAAGGGCCCAGCGTGTTTCCACTGGCTCCAAGCTCCA
AATCTACTTCTGGGGGCACCGCCGCACTGGGCTGTCTGGTCAAGGACTACTT
CCCAGAGCCCGTCACCGTGTCATGGAACTCCGGCGCACTGACTTCCGGAGT
CCACACCTTTCCAGCCGTGCTGCAGTCCAGCGGACTGTACAGCCTGTCTAGT
GTGGTCACAGTGCCTTCATCCAGCCTGGGAACTCAGACCTATATCTGCAACG
TGAATCACAAGCCATCAAATACTAAAGTCGACAAGAAAGTGGAACCCAAG
AGCTGTGGCGTCCTTAACCTCGCACAAAGTCCA (SEQ ID NO: 7)

Polynucleotides that encode the light chains of csF001-5Fab-Qtag, csF028-22Fab-Qtag, and csF001-25Fab-Qtag were obtained by individually amplifying the variable regions of the light chains of sF001-5, sF028-22, and sF001-25, and the constant region of the human IgG light chain κ, and then by linking the individual fragments by overlap-PCR. The polynucleotides that encode the heavy chain was obtained by overlap-PCR, with use of the CH1 region of human IgG1 as a template, in which fragments each having a Q-tag sequence attached to the C-terminal while mediated by a primer was coupled with fragments of the variable domains of the heavy chains of sF001-5, sF028-22, and sF001-25 amplified by PCR. Each of the thus prepared PCR products was introduced into pcDNA3.4 (Thermo Fisher Scientific) by TA cloning, to construct plasmids that encode csF001-5_LC (light chain in chimerized sF001-5), csF028-22_LC, and csF001-25_LC. Next, constructed were plasmids that encode csF001-5_HC_Fab-Qtag (chimeric sF001-5 whose Fab heavy chain has Qtag attached to the C-terminal), csF028-22_HC_Fab-Qtag, and csF001-25_HC_Fab-Qtag. Next, the plasmids that encode each of the heavy chain and the light chain were co-transfected into Expi-293 cell (Thermo Fisher Scientific), so as to express recombinant proteins csF001-5Fab-Qtag, csF028-22Fab-Qtag, and csF001-25Fab-Qtag in the culture supernatant. Each of csF001-5Fab-Qtag, csF028-22Fab-Qtag, and csF001-25 Fab-Qtag thus expressed in the supernatant was purified through Protein G column.

### (1.2) Preparation of Q-Tag-Attached Anti-HBs628 Fab Antibody

### (1.2.1) Establishment of Anti-HBs628 Antibody-Producing Hybridoma

A hybridoma that produces mouse anti-HBs628 antibody was produced according to the Kohler-Milstein method (Kohler G. and Milstein C., Nature, 256, 495-497 (1975)), with use of HBs628 antigen.

### (1.2.2) Preparation of Q-Tag-Attached Anti-HBs628 Fab Antibody

Q-tag-attached HBs628 Fab antibody (also referred to as "HBs628Fab-Qtag", hereinafter) was obtained by attaching the Q-tag sequence to the heavy chain C-terminal of the Fab moiety of HBs628 derived from mouse hybridoma. Amino acid sequences of the constant region including the inserted Q-tag sequence, and base sequences that encode the same are listed below (with the Q-tag sequence underlined).

### Amino Acid Sequence of Light Chain

QPKSSPSVTLFPPSSEELETNKATLVCTITDFYPGVVTVDWKVDGTPVT
QGMETTQPSKQSNNKYMASSYLTLTARAWERHSSYSCQVTHEGHTVEKSLSR
ADCS (SEQ ID NO: 8)

### Base Sequence of Light Chain

CAGCCCAAGTCTTCGCCATCAGTCACCCTGTTTCCACCTTCCTCTGA
AGAGCTCGAGACTAACAAGGCCACACTGGTGTGTACGATCACTGATTTCTA
CCCAGGTGTGGTGACAGTGGACTGGAAGGTAGATGGTACCCCTGTCACTCA
GGGTATGGAGACAACCCAGCCTTCCAAACAGAGCAACAACAAGTACATGGC
TAGCAGCTACCTGACCCTGACAGCAAGAGCATGGGAAAGGCATAGCAGTTA
CAGCTGCCAGGTCACTCATGAAGGTCACACTGTGGAGAAGAGTTTGTCCCG
TGCTGACTGTTCC (SEQ ID NO: 9)

### Amino Acid Sequence of Heavy Chain

AKTTPPSVYPLAPGSAAQTNSMVTLGCLVKGYFPEPVTVTWNSGSLSS
GVHTFPAVLQSDLYTLSSSVTVPSSTWPSETVTCNVAHPASSTKVDKKIVPRDC
GLLQGP (SEQ ID NO: 10)

### Base Sequence of Heavy Chain

GCCAAAACGACACCCCCATCTGTCTATCCACTGGCCCCTGGATCTGC
TGCCCAAACTAACTCCATGGTGACCCTGGGATGCCTGGTCAAGGGCTATTTC
CCTGAGCCAGTGACAGTGACCTGGAACTCTGGATCCCTGTCCAGCGGTGTG
CACACCTTCCCAGCTGTCCTGCAGTCTGACCTCTACACTCTGAGCAGCTCAG
TGACTGTCCCCTCCAGCACCTGGCCCAGCGAGACCGTCACCTGCAACGTTGC
CCACCCGGCCAGCAGCACCAAGGTGGACAAGAAAATTGTGCCCAGGGATTG
TGGCCTGCTCCAGGGCCCCTGATAA (SEQ ID NO: 11)

A polynucleotide that encodes the light chain of HBs628Fab-Qtag was obtained by cloning from hybridoma RNA according to 5'-RACE, and then by amplifying it by PCR. A polynucleotide that encodes the heavy chain of HBs628Fab-Qtag was obtained by cloning from hybridoma RNA according to 5'-RACE, and a fragment having a Q-tag attached to the C-terminal mediated by a primer was amplified by PCR. Each of the thus prepared PCR products was introduced into pcDNA3.4 (Thermo Fisher Scientific) by TA cloning, to construct a plasmid that encodes HBs628Fab-Qtag. Next, the plasmid was transfected into Expi-293 cell (Thermo Fisher Scientific), so as to express recombinant protein HBs628Fab-Qtag in the culture supernatant. HBs628Fab-Qtag thus expressed in the supernatant was purified through CaptureSelect LC-Lambda (mur) affinity column (Thermo Fisher Scientific).

### (1.3) Preparation of Q-Tag-Attached Anti-CD20 Fab Antibody

Q-tag-attached anti-CD20 Fab antibody (also referred to as "anti-CD20Fab-Qtag", hereinafter) was obtained by attaching the Q-tag sequence to the heavy chain C-terminal of the Fab moiety of human anti-CD20 Fab. Amino acid sequences of the hypervariable region and the constant region that contains the Q-tag inserted thereto, and base sequences that encode them were listed below (with the Q-tag sequence underlined).

### Amino Acid Sequence of Light Chain

QIVLSQSPAILSASPGEKVTMTCRASSSVSYIHWFQQKPGSSPKPWIYAT
SNLASGVPVRFSGSGSGTSYSLTISRVEAEDAATYYCQQWTSNPPTFGGGTKLEI
KRTVAAPSVFIFPPSDEQLKSGTASVVCLLNNFYPREAKVQWKVDNALQSGNS
QESVTEQDSKDSTYSLSSTLTLSKADYEKHKVYACEVTHQGLSSPVTKSFNRGE
C (SEQ ID NO: 12)

### Base Sequence of Light Chain

CAAATCGTGCTTAGCCAGTCACCAGCGATATTGTCCGCTAGTCCTGG
AGAGAAGGTGACCATGACCTGTAGGGCATCTTCCAGCGTTTCCTACATTCAC
TGGTTTCAGCAGAAGCCAGGGTCCAGCCCTAAACCCTGGATCTATGCCACC
AGTAACCTTGCCTCAGGGGTACCTGTGCGATTCAGCGGAAGCGGTAGTGGC
ACCTCATACTCCCTGACCATTAGCCGCGTTGAAGCTGAAGATGCGGCTACCT
ACTACTGCCAACAGTGGACGTCTAACCCACCCACATTTGGTGGCGGCACTA
AACTGGAGATCAAGCGGACTGTGGCTGCACCATCCGTATTCATCTTTCCGCC
CTCTGATGAGCAGCTGAAATCTGGCACAGCTAGCGTCGTGTGCCTGCTGAA
CAACTTCTACCCTAGAGAGGCAAAAGTGCAGTGGAAGGTCGACAATGCCCT
GCAGTCTGGCAATTCACAGGAATCAGTCACCGAACAGGACTCTAAGGATAG
CACGTACTCCTTGAGCAGCACACTCACTCTCTCTAAAGCCGACTATGAGAAG
CACAAGGTCTATGCCTGTGAGGTGACTCATCAAGGGCTGAGTAGTCCCGTC
ACAAAGTCCTTCAATCGTGGAGAA (SEQ ID NO: 13)

### Amino Acid Sequence of Heavy Chain

QVQLQQPGAELVKPGASVKMSCKASGYTFTSYNMHWVKQTPGRGLE
WIGAIYPGNGDTSYNQKFKGKATLTADKSSSTAYMQLSSLTSEDSAVYYCARS
TYYGGDWYFNVWGAGTTVTVSAASTKGPSVFPLAPSSKSTSGGTAALGCLVK
DYFPEPVTVSWNSGALTSGVHTFPAVLQSSGLYSLSSVVTVPSSSLGTQTYICNV
NHKPSNTKVDKKAEPKSCDKTHTGLLQG (SEQ ID NO: 14)

### Base Sequence of Heavy Chain

CAGGTGCAGTTGCAGCAACCTGGGGCAGAACTCGTGAAGCCTGGGG
CATCCGTTAAGATGTCCTGTAAAGCAAGCGGCTATACTTTCACTTCCTACAA

CATGCATTGGGTGAAACAGACTCCTGGCAGAGGCTTGGAGTGGATAGGAGC
CATCTACCCAGGGAATGGTGACACATCTTACAACCAGAAGTTCAAGGGCAA
AGCCACCCTCACTGCCGACAAAAGTAGCTCTACAGCCTATATGCAGCTGTC
ATCCCTGACGAGCGAGGATTCAGCCGTGTATTATTGCGCTAGGTCTACCTAC
TATGGAGGCGATTGGTACTTCAATGTGTGGGGTGCCGGAACTACCGTGACT
GTGTCTGCTGCGAGCACCAAGGGTCCAAGTGTCTTTCCCCTTGCACCGTCCA
GCAAATCCACAAGCGGAGGTACTGCTGCTCTTGGCTGTCTGGTGAAGGACT
ATTTTCCAGAGCCAGTGACGGTTAGCTGGAATTCAGGCGCCCTTACATCTGG
GGTACACACGTTTCCTGCCGTTCTGCAAAGCTCAGGACTGTACAGCCTGTCT
AGTGTGGTCACAGTCCCCTCAAGCAGTCTCGGCACCCAGACATACATCTGC
AATGTCAACCACAAACCCTCCAACACCAAGGTCGACAAGAAGGCAGAACCC
AAAAGTTGCGATAAGACCCATACAGGGCTGCTCCAA (SEQ ID NO: 15)

The polynucleotide that encodes the light chain Fab, and the polynucleotide having the Q-tag sequence attached to the heavy chain Fab C-terminal were individually inserted into pcDNA3.4 (Thermo Fisher Scientific), to construct, by total synthesis, expression plasmids that encode anti-CD20Fab-LC and anti-CD20Fab-HC-Qtag, respectively. Next, the plasmids were transfected into Expi-293 cell (Thermo Fisher Scientific), so as to express recombinant protein anti-CD20Fab-Qtag in the culture supernatant. Anti-CD20Fab-Qtag thus expressed in the supernatant was purified through CaptureSelect (registered trademark) Kappa XL pre-packed column (Thermo Fisher Scientific).

### 2. PEG Linker Having Amino Group and Thiol Group (SH-PEG-NH₂ linker)

SH-PEG-NH₂ linkers purchased include (a) HS-PEG2K-NH₂, HCl salt, average Mn 2000 (Sigma-Aldrich), (b) SH-PEG-NH₂, MW2K (Biopharma PEG Scientific Inc.), (c) HS-PEG-NH₂, MW2K (Creative PEGWorks), (d) HS-PEG-NH₂, HCl salt, average Mn 3500 (Sigma-Aldrich), (e) HS-PEG-NH₂, HCl salt, average Mn 5000 (Sigma-Aldrich), (f) Thiol PEG-NH₂, average Mn 400 Da (Nanocs Inc.) and (g) HS-PEG-NH₂, MW 1 kDa (Creative PEGWorks). Also purchased was HS-PEG2K-NH₂, HCl salt, average Mn 2000 (Sigma-Aldrich), but from a lot different from the linker (a) (also referred to as linker (a'), hereinafter). The linkers (a), (a'), (b) and (c) were sold as products with a molecular weight indication of 2000. Each of the linkers (a), (a'), (b) and (c) will also be referred to as "SH-PEG-NH₂ linker (2K)". The linker (d) was sold as a product with a molecular weight indication of 3500. The linker (d) will also be referred to as "SH-PEG-NH₂ linker (3.5K)". The linker (e) was sold as a product with a molecular weight indication of 5000. The linker (e) will also be referred to as "SH-PEG-NH₂ linker (5K)". The linker (f) was sold as a product with a molecular weight indication of 400. The linker (f) will also be referred to as "SH-PEG-NH₂ linker (400Da)". The linker (g) was sold as a product with a molecular weight indication of 1000. The linker (g) will also be referred to as "SH-PEG-NH₂ linker (1K)". The linkers (a), (a'), (b), (d), and (e) were available as hydrochlorides, and the linkers (c), (f), and (g) were available in the free form. Structure of the individual linkers are listed below.

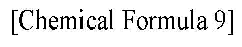

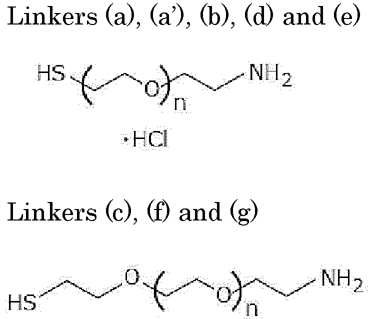

Fab antibody having the SH-PEG-NH₂ linker bound thereto will also be referred to as "SH-PEG-Fab". In particular, each Fab antibody having any of the SH-PEG-NH₂ linkers (a), (a'), (b), (c) bound thereto will also be referred to as "SH-PEG(2K)-Fab". Meanwhile, The Fab antibodies having the SH-PEG-NH₂ linkers (d), (e), (f) and (g) bound thereto will also be referred to as "SH-PEG(3.5K)-Fab", "SH-PEG(5K)-Fab", "SH-PEG(400Da)-Fab" and "SH-PEG(1K)-Fab", respectively.

### 3. Crosslinking Agent Having Maleimide Group and NHS Ester (EMCS Reagent)

EMCS reagent (Product name: EMCS, CAS No.: 55750-62 -5) was purchased from Dojindo Laboratories. The molecular formula and molecular weight were C₁₄H₁₆N₂O₆ and 308.29, respectively, with a structure given below.

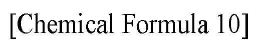

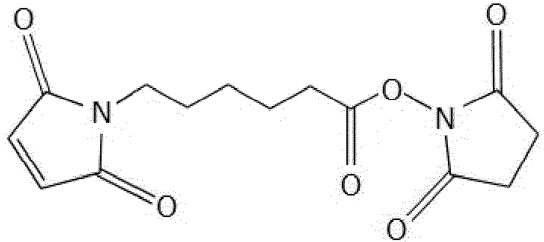

### 4. Labeling

### (4.1) Biotin Having Maleimide Group

Biotin-PEAC5-maleimide (product name: Biotin-PEAC₅-maleimide, CAS No.: 374592-98-0) was purchased from Dojindo Laboratories. The molecular formula and molecular weight were C₂₆H₄₁ClN₆O₅S and 585.16, respectively, with a structure given below.

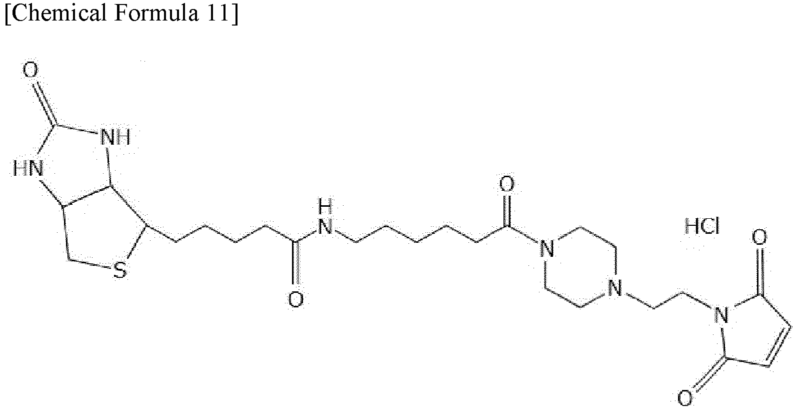

### (4.2) Fluorescent Dye Having Maleimide Group

Alexa 488-maleimide (product name: Alexa Fluor (registered trademark) 488 C5 maleimide, CAS No.: 500004-82-0) was purchased from Thermo Fisher Scientific. The molecular formula and molecular weight were C₃₀H₂₅N₄NaO₁₂S₂ and 720.66, respectively, with a structure given below.

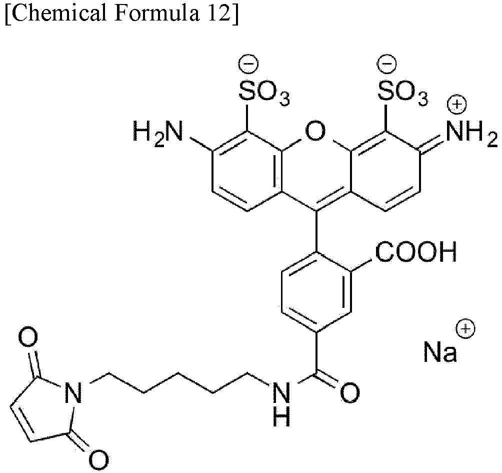

### (4.3) R-Phycoerythrin (R-PE)

R-PE (product name: OB1) was purchased from One Biotech.

### (4.4) Alkaline Phosphatase (ALP)

ALP derived from bovine small intestine (product name: ALP-55) was purchased from Oriental Yeast Co., Ltd.

### 5. Transglutaminase (TG)

TG employed herein was Activa (registered trademark) KS-CT (Ajinomoto Corporation), which is a microorganism-derived transglutaminase, used after purified. The thus purified enzyme will also be referred to as "BTGase", hereinafter.

### 6. Mass Spectrometry based on Infusion Scheme (Infusion MS)

The aforementioned SH-PEG-NH₂ linkers (a), (a'), (b), (c), (d) and (e) were analyzed by Infusion MS, in order to find the molecular weight distributions and occurrence of dimerization. An MS analyzer employed herein was Q Exactive (Thermo Fisher Scientific). The ionization mode was set to positive. The measurement sample was prepared by dissolving each of the aforementioned SH-PEG-NH₂ linkers in a BTGase reaction solution (50 mM Tris, 2 mM EDTA, pH 8.2; or 20 mM Tris, 2 mM EDTA, 150 mM NaCl, pH 8.2), followed by 100-fold dilution with a 30% acetonitrile solution containing 0.1% formic acid. Result of mass analysis for each SH-PEG-NH₂ linker having a repeating structure was examined to find an isotope peak whose intensity is highest of all peaks having an S/N of 5 or larger, and from the molecular weight and intensity of such most intense peak, the weight average molecular weight of each linker was determined. The MS peak was also used to determine the distribution range of molecular weight.

### 7. Quantification of Target Protein

The individual Fab antibodies and derivatives thereof were quantified on the basis of Abs280 peak area in SEC. Reference used herein was a recombinant human interleukin 6 (rhIL-6) (absorption coefficient 0.43).

### 8. Analysis of Non-Reducing SDS-PAGE

The individual Fab antibodies and derivatives thereof were analyzed by non-reducing SDS-PAGE under the conditions below.

10 to 20% Polyacrylamide gel: e-PAGEL (ATTO Corporation)
Electrophoretic device: PageRun (ATTO Corporation)
Electrophoresis conditions: 20 mA, 80 minutes
Sample buffer: NuPAGE LDS Sample Buffer (Invitrogen)

### Test Example 1: BTGase-Catalyzed Modification of Fab Antibodies with SH-PEG-NH₂ Linker (2K)

### (1) Binding of SH-PEG-NH₂ Linker (2K) with Fab Antibodies

The aforementioned (a) SH-PEG-NH₂ linker (2K) (Sigma-Aldrich) was bound to each of csF001-5Fab-Qtag, csF028-22Fab-Qtag, csF001-25Fab-Qtag and HBs628Fab-Qtag, while catalyzed by BTGase. Also the aforementioned (a') SH-PEG-NH₂ linker (2K) (Sigma-Aldrich) was bound to anti-CD20Fab-Qtag. Operational details were as follows. csF001-5Fab-Qtag or HBs628Fab-Qtag was dissolved in MES buffer solution (50 mM MES, 2 mM EDTA, pH 7.0) so as to adjust the concentration to 10 µM, and the solution was incubated with 50 equivalents of SH-PEG-NH₂ linker (2K) and 0.1 U/mL of BTGase at 25°C for 3 hours. The activity of BTGase was assayed by the hydroxamate method (Folk J.E. and Cole P.W., J.Biol.Chem. 241, 5518-5525

### (1966)). Progress of the reaction was monitored by SEC.

Enhancement of introduction efficiency of the SH-PEG-NH₂ linker (2K) was examined in a Tris buffer (20 mM Tris, 2 mM EDTA, 150 mM NaCl, pH 8.2 or pH 8.5), by adding 50 equivalents of the linker to 10 µM of each of csF001-5Fab-Qtag, csF028-22Fab-Qtag, and csF001-25Fab-Qtag, and by reacting the mixture at room temperature for 5 hours. Results of the SEC analysis of the reaction products obtained at pH 8.2 and pH 8.5, and the reaction product obtained at pH 7.0 were then compared.

The reaction condition (pH 8.2 or 8.5) aimed at enhanced introduction efficiency was applied to all reactions between the Fab-Qtags and the SH-PEG-NH₂ linkers. Among the reaction solutions, the reaction solution of csF001-5Fab-Qtag was desalted and concentrated with Amicon 10K (Merck), to remove the SH-PEG-NH₂ linker. The reaction solution of csF028-22Fab-Qtag was fractionated with Superdex 200 Increase 10 × 300 mm, and concentrated with Amicon 10K (Merck). The reaction solution of HBs628Fab-Qtag was prepared through Superdex 75 Increase 10 × 300 mm, and concentrated with Amicon 10K (Merck). The reaction solutions of csF001-25Fab-Qtag and anti-CD20Fab-Qtag were desalted and purified through Protein G column, and then concentrated with Amicon 10K (Merck).

### (2) SEC Analysis

The reaction solutions of csF001-5Fab-Qtag, csF028-22Fab-Qtag, csF001-25Fab-Qtag, each reacted with the SH-PEG-NH₂ linker (2K) (Sigma-Aldrich), were analyzed by SEC. The reaction solution of csF028-22Fab-Qtag and SH-PEG-NH₂ linker (2K) was also fractionated. Conditions were as follows.

Column: Superdex 200 Increase 10×300 mm (Cytiva)
HPLC apparatus: Chromaster (registered trademark) (Hitachi High-Tech Science Corporation)
Analysis temperature: room temperature
Solvent: Arg-SEC Mobile Phase (Standard, Nacalai Tesque, Inc.)
Flow rate: 0.8 ml/min
Detection: UV 280 nm, FL (Ex: 295 nm/Em: 335 nm)

### (3) SEC Analysis and Fractionation

The reaction solution of HBs628Fab-Qtag and the SH-PEG-NH₂ linker (2K) (Sigma-Aldrich) was analyzed and isolated by fractionation according to the conditions below.

Column: Superdex 75 Increase 10×300 mm (Cytiva)
HPLC apparatus: Chromaster (registered trademark) (Hitachi High-Tech Science Corporation)
Analysis temperature: room temperature
Solvent: Arg-SEC Mobile Phase (Strong, Nacalai Tesque, Inc.)
Flow rate: 0.8 ml/min
Detection: UV 280 nm, FL (Ex: 295 nm/Em: 335 nm)

### (4) Fractionation of SH-PEG-Fab through Protein G

SH-PEG (2K)-Fab derived from each of csF001-25 Fab-Qtag and anti-CD 20Fab-Qtag was isolated by fractionation under the following conditions.

Column: HiTrap (trademark) Protein G HP Column (1 mL, Cytiva)
Solvent A: 20 mM phosphate buffer, 150 mM NaCl, pH 7.0
Solvent B: 0.1 M glycine, pH 2.7
Flow rate: 0.8 ml/min
Detection: UV 280 nm, FL (Ex: 295 nm/Em: 335 nm)

### Test Example 2: Biotin Labeling of SH-PEG-Fab Derived from HBs628Fab-Qtag

### (1) Biotin Labeling of SH-PEG-Fab

SH-PEG(2K)-Fab from HBs628Fab-Qtag was labeled with biotin, as described below. The BTGase-catalyzed reaction solution of HBs628Fab-Qtag and the (a) SH-PEG-NH₂ linker (2K) (Sigma-Aldrich) was desalted and concentrated with Amicon 10K (Merck), to obtain a concentrated solution that contains SH-PEG-Fab. In a 50 mM MES-2 mM EDTA buffer solution (pH 7.0), added was Biotin-PEAC5-maleimide, whose amount was adjusted to 100 equivalents relative to 10 µM of SH-PEG-Fab, and the mixture was reacted at 5°C overnight. The reaction solution was purified by SEC. The reaction product of SH-PEG-Fab and maleimide-labeled biotin will also be referred to as "biotin-PEG-Fab", hereinafter. The biotin-PEG-Fab was found to have the structure represented by formula (I) (wherein X represents ethylene, Y represents a PEG chain, Z represents biotin, and L represents a spacer).

### (2) SEC Analysis and Fractionation of Biotin-PEG-Fab

Biotin-PEG-Fab derived from HBs628Fab-Qtag was analyzed and fractionated.

Column: Superdex 75 Increase 10×300 mm
HPLC apparatus: Chromaster (registered trademark) (Hitachi High-Tech Science Corporation)
Analysis temperature: room temperature
Solvent: Arg-SEC Mobile Phase (Strong, Nacalai Tesque, Inc.)
Flow rate: 0.8 ml/min
Detection: UV 280 nm, FL (Ex: 295 nm/Em: 335 nm)

### (3) Analysis by Western Blotting (WB)

HBs628Fab-Qtag, SH-PEG(2K)-Fab thereof, and biotin-PEG-Fab were separated by non-reducing SDS-PAGE, and then analyzed by WB as follows. Fab (5 to 10 ng equivalent) was separated by SDS-PAGE (10 to 20% polyacrylamide gel), and then transferred to a PVDF membrane (Invitrogen) with use of iBlot2 dry blotting system (Thermo Fisher Scientific). The PVDF membrane after the transfer was blocked with 1% skim milk in TBST (10 mM Tris, 150 mM NaCl, 0.05% Tween 20, pH 7.4) at room temperature for one hour. The PVDF membrane after the blocking was washed with TBST (10 min, three times). HRP-conjugated streptavidin (Thermo Fisher Scientific) diluted 1: 20,000 with 1% skim milk-containing TBST was added to the PVDF membrane, and the membrane was left still at room temperature for one hour for reaction. The PVDF membrane was then washed with TBST (10 min, three times). ECL Prime Western Blotting Detection Reagent (Cytiva) was added to the PVDF membrane, and the presence or absence of biotin labeling was detected on Amersham Imager 680 (Cytiva).

### (4) LC-MS Analysis

SH-PEG-Fab derived from HBs628Fab-Qtag was analyzed by MS according to two types of method below. That is, non-reduced and reduced samples were analyzed. The samples were reduced by adding an excessive amount of 85 mM TCEP (tris(2-carboxyethyl) phosphine) to 10 µg of Fab, the mixture was left overnight at 5°C, and then subjected to LC-MS analysis. Analytical conditions were as follows.

Column: Develosil C18 (3 µm, 2 mm ID × 100 mmL, Nomura Chemical Co., Ltd.)
Analysis temperature: 25°C
Solvent A: 0.1% formic acid
Solvent B: 0.1% formic acid, 100% acetonitrile
Gradient: from B10% to B60% over one minute
Flow rate: 100 µl/min
LC apparatus: LC-20A (Shimadzu Corporation)
MS analyzer: Q Exactive (Thermo Fisher Scientific)
Ionization mode: positive

### Test Example 3: ALP Labeling of SH-PEG-Fab

### (1) Maleimide Modification of ALP

ALP was modified with maleimide, aiming at use for ALP labeling of SH-PEG(2K)-Fab derived from each of csF001-5Fab-Qtag, csF028-22Fab-Qtag, and HBs628Fab-Qtag. ALP was dissolved in a 25 mM triethanolamine buffer (1 mM MgCl₂, 0.1 mM ZnCl₂, 150 mM NaCl, pH 7.0), so as to adjust the concentration to 10 µM. Twenty equivalents of EMCS reagent was then added to the ALP solution, and the mixture was incubated at 37°C for one hour. The reaction solution was desalted through PD10 column (Cytiva), and then concentrated with Amicon 10K.

### (2) ALP Labeling of SH-PEG(2K)-Fab

### (2.1) Labeling of SH-PEG(2K)-Fab Derived from csF001-SFab-Qtag

SH-PEG(2K)-Fab derived from csF001-5Fab-Qtag was labeled with ALP, as described below. Twenty micromoles of SH-PEG(2K)-Fab and 10 µM of maleimide-modified ALP were incubated in a 25 mM triethanolamine buffer (1 mM MgCl₂, 0.1 mM ZnCl₂, 150 mM NaCl, pH7.0) at 5°C overnight. After analyzing the reaction solution by HPLC with use of Superdex 200 Increase (Cytiva), each of ALP-labeled Fab having Fab bound to one maleimide group owned by one ALP molecule (referred to as "Fab-PEG-ALP", hereinafter); ALP-labeled Fab having Fab bound to each of two maleimide groups owned by one ALP molecule (referred to as "(Fab-PEG)₂-ALP", hereinafter); and ALP-labeled Fab having Fab bound to each of three maleimide groups owned by one ALP molecule (referred to as "(Fab-PEG)₃-ALP", hereinafter) were individually purified and fractionated. Note that Fab-PEG-ALP was a complex of one ALP molecule and one Fab molecule, (Fab-PEG)₂-ALP was a complex of one ALP molecule and two Fab molecules, and (Fab-PEG)₃-ALP was a complex of one ALP molecule and three Fab molecules. Each of Fab-PEG-ALP, (Fab-PEG)₂-ALP, and (Fab-PEG)₃-ALP was found to have the structure represented by formula (I) (wherein X represents ethylene, Y represents a PEG chain, Z represents ALP, and L represents a spacer).

### (2.2) Labeling of SH-PEG(2K)-Fab Derived from csF028-22Fab-Qtag

SH-PEG(2K)-Fab derived from csF028-22Fab-Qtag was labeled with ALP, as described below. SH-PEG(2K)-Fab was concentrated with Amicon 10K (Merck), and the buffer was exchanged to a 25 mM triethanolamine buffer (1 mM MgCl₂, 0.1 mM ZnCl₂, 150 mM NaCl, pH 7.0). Twenty micromoles of the thus obtained reaction solution and 6.7 µM of maleimide-modified ALP were incubated in a 25 mM triethanolamine buffer (1 mM MgCl₂, 0.1 mM ZnCl₂, 150 mM NaCl, pH7.0) at 5°C overnight. After analyzing the reaction solution by HPLC with use of Superdex 200 Increase (Cytiva), Fab-PEG-ALP, (Fab-PEG)₂-ALP, and (Fab-PEG)₃-ALP were individually purified and fractionated. Each ALP-labeled Fab was quantified according to the aforementioned quantification method for the target protein. BSA (Proliant) was added to the solution of each ALP-labeled Fab, so as to adjust the final concentration to 0.1%.

### (2.3) Labeling of SH-PEG(2K)-Fab Derived from HBs628Fab-Qtag

SH-PEG(2K)-Fab derived from HBs628Fab-Qtag was labeled with ALP, as described below. SH-PEG(2K)-Fab and the maleimide-modified ALP (also referred to as "(Mal)n-ALP", hereinafter) were dissolved according to the molar proportion listed in Table 1 into a 25 mM triethanolamine buffer (1 mM MgCl₂, 0.1 mM ZnCl₂, 150 mM NaCl, pH7.0), and incubated at 5°C overnight. After analyzing the reaction solution by HPLC with use of Superdex 200 Increase (Cytiva), Fab-PEG-ALP, (Fab-PEG)₂-ALP, and (Fab-PEG)₃-ALP were individually purified and fractionated. Note that (Mal)ₙ-ALP represents one ALP molecule to which n (n is an integer of 1 or larger) maleimide groups are added.

**[Table 1]**

| Reaction condition | SH-PEG-Fab (*µ* M) | (Mal)ₙ-ALP (*µ* M) |
|---|---|---|
| 1 | 10 | 5 |
| 2 | 10 | 10 |
| 3 | 10 | 20 |

### (3) SEC Analysis and Fractionation of Fab-PEG-ALP, (Fab-PEG)₂-ALP and (Fab-PEG)₃-ALP

### (3.1) Analytical Conditions for ALP-PEG-Fab Derived from SH-csF001-5Fab-Qtag, and HBs628Fab-Qtag

Column: Superdex 200 Increase 10×300 mm
HPLC apparatus: Chromaster (registered trademark) (Hitachi High-Tech Science Corporation)
Analysis temperature: room temperature
Solvent: Arg-SEC Mobile Phase (Standard, Nacalai Tesque, Inc.)
Flow rate: 0.8 ml/min
Detection: UV 280 nm, FL (Ex: 295 nm/Em: 335 nm)

### (3.2) Conditions for Analysis and Fractionation of Fab-PEG-ALP, (Fab-PEG)₂-ALP and (Fab-PEG)₃-ALP

Column: Superdex 200 Increase 10×300 mm
HPLC apparatus: Chromaster (registered trademark) (Hitachi High-Tech Science Corporation)
Analysis temperature: room temperature
Solvent: 25 mM triethanolamine buffer (1 mM MgCl₂, 0.1 mM ZnCl₂, 150 mM NaCl, pH 7.0)
Flow rate: 0.8 ml/min
Detection: UV 280 nm, FL (Ex: 295 nm/Em: 335 nm)

### Test Example 4: ALP Labeling of Fab' by Random Labeling

### (1) Maleimide Modification of ALP

Intended to be used for ALP labeling of Fab' free of Q-tag, ALP was modified with maleimide as follows. ALP was dissolved in D-PBS(-) (pH 7.4), so as to adjust the concentration to 20 µM. Thirty equivalents of EMCS reagent was then added to the ALP solution, and the mixture was incubated at 37°C for one hour. The reaction solution was desalted through PD10 column (Cytiva), and the buffer was exchanged to a 0.1 M triethanolamine buffer (1 mM MgCl₂, 0.1 mM ZnCl₂, 150 mM NaCl, pH 7.0). The solution was then concentrated with Amicon 50K.

### (2) Preparation of F(ab')₂ from Full-Length Antibody, and SEC Fractionation

sF001-5 and pepsin (Sigma) were dissolved into McIlvaine buffer (pH 3.8) so as to adjust the concentrations to 6.67 µM and 0.95 µM, respectively, and the mixture was incubated at 37°C for 3 hours. Then 10 v% of 1 M Tris-HCl (pH 8.5) was added for neutralization, thereby terminating the reaction. The product was concentrated with Amicon 10K, and F(ab')₂ was then purified and fractionated through Superdex 200 Increase (Cytiva). The thus obtained F(ab')₂ solution was again concentrated with use of Amicon 10K. Note that the reactivity of the antibody, per unit amount of substance, was not found to decline, even after thus processed. Conditions for fractionation were as follows.

Column: Superdex 200 Increase 10×300 mm
Purification apparatus: ÄKTA go (Cytiva)
Analysis temperature: room temperature
Solvent: 0.1 M phosphate buffer (1 mM EDTA 2Na, pH 6.0)
Flow rate: 0.5 ml/min
Detection: UV 280 nm

### (3) Preparation of Fab' from F(ab')₂, and SEC Fractionation

F(ab')₂ thus prepared in (2) above was dissolved in a 0.1 M phosphate buffer (1 mM EDTA 2Na, pH 6.0), so as to adjust the concentration to 20 µM. To the F(ab')₂ solution, 1500 equivalents of 2-mercaptoethylamine reagent (Nacalai Tesque, Inc.) was added, and the mixture was incubated at 37°C for 90 minutes. Only a fraction that contains the produced sF001-5Fab' was fractionated and purified through Superdex 200 Increase (Cytiva), and concentrated with Amicon 10K. Conditions for fractionation were as follows.

Column: Superdex 200 Increase 10×300 mm
Purification apparatus: ÄKTA go (Cytiva)
Analysis temperature: room temperature
Solvent: 0.1 M phosphate buffer (1 mM EDTA 2Na, pH 6.0)
Flow rate: 0.5 ml/min
Detection: UV 280 nm

### (4) ALP Labeling of Fab' (Random Labeling)

Fab' was labeled with ALP, by coupling of the cysteine residue in Fab', with maleimide-modified ALP. Operational details were as follows. Forty-four micromoles of sF001-5Fab' and 8.8 µM of maleimide-modified ALP were incubated in a 0.1 M triethanolamine buffer (1 mM MgCl₂, 0.1 mM ZnCl₂, 150 mM NaCl, pH7.0) at 5°C overnight. The thus produced coupled product (referred to as "(Fab')ₙ-ALP", hereinafter) was then fractionated and purified through Superdex 200 Increase (Cytiva). Conditions for fractionation were as follows. Note that (Fab')ₙ-ALP represents a complex in which Fab' is bound to each of n (n is an integer of 1 or more) maleimide groups owned by ALP.

Column: Superdex 200 Increase 10×300 mm
Purification apparatus: ÄKTA go (Cytiva)
Analysis temperature: room temperature
Solvent: 0.1 M triethanolamine buffer (1 mM MgCl₂, 0.1 mM ZnCl₂, 150 mM NaCl, pH 7.0)
Flow rate: 0.5 ml/min
Detection: UV 280 nm

The sample that contains purified (Fab')ₙ-ALP was analyzed by non-reducing SDS-PAGE and SEC. Analytical conditions for SEC were as follows.

Column: Superdex 200 Increase 10×300 mm
HPLC apparatus: Chromaster (registered trademark) (Hitachi High-Tech Science Corporation)
Analysis temperature: room temperature
Solvent: Arg-SEC Mobile Phase (Standard, Nacalai Tesque, Inc.)
Flow rate: 0.8 ml/min
Detection: UV 280 nm, FL (Ex: 295 nm/Em: 335 nm)

### Test Example 5: Fluorescence Labeling of SH-PEG-Fab with Alexa 488-Maleimide

### (1) Coupling of SH-PEG-Fab with Alexa 488-Maleimide

SH-PEG(2K)-Fab derived from csF001-25Fab-Qtag was labeled with a fluorescent dye, as described below. SH-PEG(2K)-Fab was concentrated with Amicon 10K (Merck), and the buffer was exchanged to a 50 mM sodium phosphate buffer (2 mM EDTA, pH 7.0). Six micromoles of reaction solution and 120 µM of Alexa 488-maleimide were incubated in the 50 mM sodium phosphate buffer (2 mM EDTA, pH 7.0) overnight at 5°C. The Alexa 488-labeled Fab (Alexa 488 -PEG-Fab) in the reaction solution was fractionated and analyzed by HPLC with use of Superdex 200 Increase (Cytiva). The reaction product of SH-PEG-Fab and Alexa 488-maleimide will also be referred to as "Alexa 488-PEG-Fab", hereinafter. The Alexa 488-PEG-Fab was found to have the structure represented by formula (I) (wherein X represents ethylene, Y represents a PEG chain, Z represents Alexa 488, and L represents a spacer).

### (2) Conditions for SEC Fractionation

Column: Superdex 200 Increase 10×300 mm
HPLC apparatus: Chromaster (registered trademark) (Hitachi High-Tech Science Corporation)
Analysis temperature: room temperature
Solvent: 50 mM phosphate buffer, 2 mM EDTA, pH 7.0
Flow rate: 0.8 ml/min
Detection: UV 280 nm

### (3) Analytical Conditions for SEC

Column: Superdex 200 Increase 10×300 mm
HPLC apparatus: Chromaster (registered trademark) (Hitachi High-Tech Science Corporation)
Analysis temperature: room temperature
Solvent: Arg-SEC Mobile Phase (Standard, Nacalai Tesque, Inc.)
Flow rate: 0.8 ml/min
Detection: UV 280 nm
FL (Ex. 495 nm/Em. 519 nm) PMT Super Low

### (4) LC-MS Analysis

SH-PEG(2K)-Fab derived from csF001-25Fab-Qtag, and Alexa 488-PEG-Fab were analyzed by LC-MS as described below. The samples were measured by LC-MS, after reduced with 200 equivalents of TCEP in 1 M Tris (pH 7.5) at 37°C for 2 hours. Analytical conditions were as follows.

Column: PLRP-1000 column (2 µm × 100 mmL, PL Laboratory)
Analysis temperature: 25°C
Solvent A: 0.1% formic acid
Solvent B: 0.1% formic acid, 100% acetonitrile
Gradient: from B10% to B60% over one minute
Flow rate: 100 µl/min
LC apparatus: LC-20A (Shimadzu Corporation)
MS analyzer: Q Exactive (Thermo Fisher Scientific)
Ionization mode: positive

### Test Example 6: Fluorescence Labeling of SH-PEG-Fab with R-PE

### (1) Maleimide Modification of R-PE

R-PE was modified with maleimide, aiming at use for R-PE labeling of SH-PEG(2K)-Fab derived from each of csF001-25Fab-Qtag and anti-CD20Fab-Qtag. R-PE was dialyzed against a 50 mM sodium phosphate buffer (2 mM EDTA, pH 7.0) to replace the buffer. Then, 400 µM of EMCS reagent was added to the 5 µM R-PE solution, and the mixture was incubated at 37°C for one hour. The reaction solution was desalted through PD 10 column (Cytiva), and then concentrated with Amicon 3K. The thus obtained maleimide-modified R-PE is also referred to as "(Mal)ₙ-R-PE", hereinafter. (Mal)ₙ-R-PE represents one R-PE molecule to which n (n is an integer of 1 or larger) maleimide groups are added.

### (2) Coupling of SH-PEG-Fab with Maleimide-modified R-PE

SH-PEG(2K)-Fab derived from each of csF001-25Fab-Qtag and anti-CD20Fab-Qtag was labeled with a fluorescent dye, as described below. SH-PEG(2K)-Fab was concentrated with Amicon 10K (Merck), and the buffer was exchanged to a 50 mM sodium phosphate buffer (2 mM EDTA, pH 7.0). Six micromole of the reaction solution and 2 µM of (Mal)ₙ-R-PE were incubated in a 50 mM sodium phosphate buffer (2 mM EDTA, pH 7.0) overnight at 5°C. L-cysteine was added to the reaction solution to adjust the final concentration to 0.1 mM, so as to cap the unreacted maleimide groups. The reaction solution was then analyzed by HPLC with Superdex 200 Increase (Cytiva). R-PE-labeled Fab having Fab bound to one maleimide group owned by one R-PE molecule (referred to as "Fab-PEG-R-PE", hereinafter); R-PE-labeled Fab having Fab bound to each of two maleimide groups owned by one R-PE molecule (referred to as "(Fab-PEG)₂-R-PE", hereinafter); and R-PE-labeled Fab having Fab bound to each of three maleimide groups owned by one R-PE molecule (referred to as "(Fab-PEG)₃-R-PE", hereinafter) were individually purified and fractionated. These fractions were also analyzed by SEC. Conditions for the fractionation and analysis were as follows. Note that Fab-PEG-R-PE was a complex of one R-PE molecule and one Fab molecule, (Fab-PEG)₂-R-PE was a complex of one R-PE molecule and two Fab molecules, and (Fab-PEG)₃-R-PE was a complex of one R-PE molecule and three Fab molecules. Each of Fab-PEG-R-PE, (Fab-PEG)₂-R-PE, and (Fab-PEG)₃-R-PE was found to have the structure represented by formula (I) (wherein X represents ethylene, Y represents a PEG chain, Z represents R-PE, and L represents a spacer).

### (3) Conditions for SEC Fractionation

Column: Superdex 200 Increase 10×300 mm
HPLC apparatus: Chromaster (registered trademark) (Hitachi High-Tech Science Corporation)
Analysis temperature: room temperature
Solvent: 50 mM phosphate buffer, 2 mM EDTA, pH 7.0
Flow rate: 0.8 ml/min
Detection: UV 280 nm

### (4) Analytical Conditions for SEC

Column: Superdex 200 Increase 10×300 mm
HPLC apparatus: Chromaster (registered trademark) (Hitachi High-Tech Science Corporation)
Analysis temperature: room temperature
Solvent: Arg-SEC Mobile Phase (Standard, Nacalai Tesque, Inc.)
Detection: UV 280 nm
FL (Ex. 565 nm/Em. 574 nm) PMT Super Low

### Test Example 7: Measurement of ALP Activity of ALP-Labeled Antibody

ALP activity of Fab-PEG-ALP, (Fab-PEG)₂-ALP and (Fab-PEG)₃-ALP derived from csF028-22Fab-Qtag prepared in Test Example 3 were measured as follows. The ALP activity of the calibrator (ALP-55 (Oriental Yeast Co., Ltd.) whose activity value was assigned with use of biochemistry automatic analyzer; Hitachi 7170), and the individual ALP-labeled antibody samples whose concentrations were adjusted within a range of analytical curve, were measured, while using CDP-star (Thermo Fisher Scientific) as a substrate. The ALP activity of the individual ALP-labeled antibodies was calculated by multiplying the measured ALP activity of the individual measurement samples by the dilution rate. Specific activities relative to unmodified ALP were compared.

### Test Example 8: Performance Evaluation of Reagents that Contain Fab-PEG-ALP and (Fab')ₙ-ALP

HIV p24 antigen was assayed by immunological assay that individually uses, as the detection antibodies, Fab-PEG-ALP derived from csF001-5 Fab-Qtag prepared in Test Example 3, and (Fab')ₙ-ALP prepared in Test Example 4. Measured results were compared to examine the performance of the reagents that individually contain Fab-PEG-ALP and (Fab')ₙ-ALP. The measurement employed a fully automated immunoassay system HISCL (registered trademark) 2000i (Sysmex Corporation).

### (1) Preparation of Reagents

Reagents used here were R1 reagent (biotin-labeled antibody), R2 reagent (streptavidin-immobilized magnetic particle), R4 reagent (chemiluminescent substrate diluent), and R5 reagent (chemiluminescent substrate), all contained in HIV-1 p24 antigen/HIV antibody kit "HISCL (registered trademark) HIV Ag+Ab Reagent" (Sysmex Corporation); or reagents prepared by a method according to the method for producing these reagents, while excluding R3 reagent which is a detection antibody reagent. The R3 reagent was prepared as follows, with use of Fab-PEG-ALP or (Fab')ₙ-ALP. Each of Fab-PEG-ALP and (Fab')ₙ-ALP was diluted with a 0.1 M triethanolamine buffer (3% BSA, 0.5% sodium caseinate, 1 mM MgCl₂, 0.1 mM ZnCl₂, 150 mM NaCl, pH 6.5), and the concentration was adjusted to the level summarized in Table 2. Each antibody solution was filtered through Millex-GS 0.22 µm (Merck) to obtain R3 reagent. The ALP activity of each antibody was estimated in the same way as described in Test Example 7.

**[Table 2]**

| ALP-labeled antibody | Concentration (U/mL) | |
|---|---|---|
| (Fab')ₙ-ALP | | 0.15 |
| | | 0.50 |
| | | 1.0 |
| | | 1.5 |
| Fab-PEG-ALP | | 0.15 |
| | | 0.50 |
| | | 1.0 |
| | | 1.5 |

### (2) Preparation of Samples

The HIV p24 antigen (Abeam plc) was diluted with each of D-PBS (0.1% BSA, pH 7.4) and human pool serum (Nissui Pharmaceutical Co., Ltd.), to prepare samples with antigen concentrations of 10 pg/mL, 100 pg/mL and 1000 pg/mL. D-PBS (0.1% BSA, pH 7.4) and human pooled serum per se were used as antigen-free (0 pg/mL) samples.

### (3) Assay

The R1 to R5 reagents described in (1) above were set on HISCL-2000i (Sysmex Corporation), and eight types of samples prepared in (2) above were assayed. Measurement procedures with use of HISCL-2000i were as follows. The sample (20 µL) and R1 reagent (50 µL) were mixed, and reagent R2 (30 µL) was then added. The magnetic particle in the resultant mixed liquid was magnetically collected to remove the supernatant, and the magnetic particle was washed by adding HISCL washing solution (300 µL). The supernatant was removed, and R3 reagent (100 µL) was added to the magnetic particle, followed by mixing. The magnetic particle in the resultant mixed liquid was magnetically collected to remove the supernatant, and the magnetic particle was washed by adding HISCL washing solution (300 µL). The supernatant was removed, R4 reagent (50 µL) and R5 reagent (100 µL) were added to the magnetic particle, and chemiluminescence intensity was then measured.

### Test Example 9: Evaluation of Antigen Binding Capacity of Alexa 488-Labeled Antibody

Antigen binding capacity of SH-PEG(2K)-Fab derived from csF001-25Fab-Qtag, and Alexa 488-PEG-Fab, prepared in Test Example 5, was measured by surface plasmon resonance (SPR) analysis with use of Biacore (registered trademark) T200 (Cytiva). For comparison, also csF001-25 Fab-Qtag was measured. Operational details were as follows. Human Fab Binder attached to Human Fab Capture Kit (Cytiva) was immobilized on a sensor chip CM5 (Cytiva), by amine coupling. SH-PEG(2K)-Fab, Alexa 488 PEG-Fab, and unlabeled Fab (csF001-25Fab-Qtag) were individually bound as ligands. Recombinant protein HIV-1 p24 (Prospec) was reacted as an analyte. The interaction was analyzed according to a 1:1 binding reaction model, with use of Biacore T 200 Evaluation software. Apparatus, reagents and reaction conditions employed here were as follows.

### [Apparatus and Reagents]

Apparatus: Biacore (registered trademark) T200 (Cytiva)
Sensor chip: Sensor chip CM5 (Cytiva)
Capture kit: Human Fab Capture Kit (Cytiva)
Coupling kit: Amine Coupling Kit (Cytiva)
Buffer: HBS-EP+ Buffer (Cytiva)

### [Ligand Capture]

Ligand: SH-PEG(2K)-Fab, Alexa 488 PEG-Fab, and unlabeled Fab (csF001-25Fab-Qtag)
Flow rate: 30 µl/min
Addition time (Contact time): 60 sec

### [Sample]

Analyte: HIV-1 p24 (Prospec) (2.5 to 80 nM)
Flow rate: 30 µl/min
Addition time (Contact time): 30 sec
Dissociation time: 300 sec

### [Regeneration]

Regeneration buffer: 10 mM glycine-HCl pH 2.1
Flow rate: 30 µl/min
Addition time (Contact time): 30 sec

### Test Example 10: Antigen-Binding Capacity of R-PE-Labeled Antibody

Antigen binding capacity of Fab-PEG-R-PE, (Fab-PEG)₂-R-PE, and (Fab-PEG)₃-R-PE derived from csF001-25Fab-Qtag prepared in Test Example 6 was measured by ELISA, as follows. Each of Fab-PEG-R-PE, (Fab-PEG)₂-R-PE, and (Fab-PEG)₃-R-PE was diluted so as to give equivalent fluorescence intensity of R-PE, to prepare each detection reagent. His-tagged recombinant HIV-1 p24, used as an antigen (Prospec), was inoculated in a black 96-well microplate having anti-His tag antibody immobilized thereon, and left for reaction at room temperature for one hour. Also anti-His tag antibody-immobilized microplate was prepared for comparison, without adding the antigen. Each well was then washed with a washing solution (0.05% Tween 20 in saline). Each detection reagent was added to each well of the microplate, and left for reaction at room temperature for one hour. After washing each well with the washing solution, the R-PE-labeled antibody in the microplate well was detected with a fluorescence plate reader (excitation wavelength: 488 nm, fluorescence wavelength: 578 nm).

### Test Example 11: Performance Evaluation of Reagents That Contain R-PE-Labeled Antibody

CD20-expressing cell was measured by flow cytometry (FCM), while using each of Fab-PEG-R-PE, (Fab-PEG)₂-R-PE, and (Fab-PEG)₃-R-PE derived from anti-CD20 Fab-Qtag prepared in Test Example 6, as a detection antibody. Measurement results were compared to examine performances of the reagents that contain the individual R-PE-labeled antibodies. Specific procedures for measurement were as follows. Each of Fab-PEG-R-PE, (Fab-PEG)₂-R-PE, and (Fab-PEG)₃-R-PE was diluted with a diluting buffer (2% FBS, 2 mM EDTA/D-PBS (pH 7.4)), so as to give equivalent fluorescence intensity of R-PE, to prepare each detection reagent. CD20-expressing Ramos cell was suspended in each detection reagent, and left for reaction at 4°C for 30 minutes. The cell was pelletized by centrifugation, and then washed with a dilution buffer. Each R-PE-labeled antibody bound to CD20 on the surface of Ramos cell was detected with BD Accuri (registered trademark) C6 Plus flow cytometer (Becton, Dickinson and Company).

### Test Example 12: Modification of Fab Antibody with SH-PEG-NH₂ Linker (3.5 K) and SH-PEG-NH₂ Linker (5 K)

### (1) Coupling of SH-PEG-NH₂ Linker (3.5K) and SH-PEG-NH₂ Linker (5K) with Fab Antibody

Each of (d) SH-PEG-NH₂ linker (3.5K) (Sigma-Aldrich), and (e) SH-PEG-NH₂ linker (5K) (Sigma-Aldrich) was bound to csF001-25Fab-Qtag, while catalyzed by BTGase. Operational details were as follows. csF001-25Fab-Qtag was dissolved in Tris buffer (20 mM Tris, 2 mM EDTA, 150 mM NaCl, pH 8.2) so as to adjust the concentration to 10 µM, and the solution was incubated with 100 equivalents of SH-PEG-NH₂ linker (3.5K) or SH-PEG-NH₂ linker (5K), and with 0.1 U/mL of BTGase at 5°C overnight. Activity of BTGase was assayed by the hydroxamate method. Progress of the reaction was monitored by SEC. The reaction solutions of the individual linkers were desalted and purified through Protein G column, and then concentrated with Amicon 10K (Merck). Analytical conditions for SEC were as follows.

### (2) Analytical Conditions for SEC

Column: Superdex 200 Increase 10×300 mm (Cytiva)
HPLC apparatus: Chromaster (registered trademark) (Hitachi High-Tech Science Corporation)
Analysis temperature: room temperature
Solvent: Arg-SEC Mobile Phase (Standard, Nacalai Tesque, Inc.)
Flow rate: 0.8 ml/min
Detection: UV 280 nm, FL (Ex: 295 nm/Em: 335 nm)

### Test Example 13: Fluorescence Labeling of SH-PEG-Fab with Alexa 488-Maleimide

### (1) Fluorescence Labeling of SH-PEG-Fab

SH-PEG(3.5K)-Fab and SH-PEG(5K)-Fab derived from csF001-25Fab-Qtag were labeled with a fluorescent dye, as described below. Each SH-PEG-Fab was concentrated with Amicon 10K (Merck), and the buffer was exchanged to a 25 mM triethanolamine buffer (1 mM MgCl₂, 0.1 mM ZnCl₂, 150 mM NaCl, pH 7.0). Six micromoles of reaction solution and 120 µM of Alexa 488-maleimide were incubated in a 25 mM triethanolamine buffer (1 mM MgCl₂, 0.1 mM ZnCl₂, 150 mM NaCl, pH7.0) at 5°C overnight. Alexa 488-PEG-Fab in the reaction solution was assayed by HPLC with use of Superdex 200 Increase (Cytiva). Analytical conditions for SEC were as follows.

### (2) Analytical Conditions for SEC

Column: Superdex 200 Increase 10×300 mm (Cytiva)
HPLC apparatus: Chromaster (registered trademark) (Hitachi High-Tech Science Corporation)
Analysis temperature: room temperature
Solvent: Arg-SEC Mobile Phase (Standard, Nacalai Tesque, Inc.)
Flow rate: 0.8 ml/min
Detection: UV 280 nm
FL (Ex. 565 nm/Em. 574 nm) PMT Super Low

### Test Example 14: Modification of Fab Antibody with SH-PEG-NH₂ Linker (400Da)

### (1) Coupling of SH-PEG-NH₂ Linker (400Da) with Fab Antibody

The aforementioned (f) SH-PEG-NH₂ linker (400Da) (Nanocs Inc.) was bound to csF001-5Fab-Qtag, while catalyzed by BTGase. Operational details were as follows. csF001-5Fab-Qtag was dissolved in Tris buffer (20 mM Tris, 2 mM EDTA, 150 mM NaCl, pH 8.2) so as to adjust the concentration to 10 µM, and the solution was incubated with 50 equivalents of SH-PEG-NH₂ linker (400Da) and 0.1 U/mL of BTGase, at 5°C overnight. Activity of BTGase was assayed by the hydroxamate method. The reaction solution was desalted and purified through Superdex 200 Increase (Cytiva), and concentrated with Amicon 10K (Merck).

### (2) ALP Labeling of SH-PEG (400 Da)-Fab

Ten micromoles of SH-PEG(400Da)-Fab and 5 µM of maleimide-modified ALP were incubated in a 25 mM triethanolamine buffer (1 mM MgCl₂, 0.1 mM ZnCl₂, 150 mM NaCl, pH7.0) at 5°C overnight. The reaction solution was analyzed by HPLC with Superdex 200 Increase (Cytiva). Analytical conditions for SEC were as follows.

### (3) Analytical Conditions for SEC

Column: Superdex 200 Increase 10×300 mm (Cytiva)
HPLC apparatus: Chromaster (registered trademark) (Hitachi High-Tech Science Corporation)
Analysis temperature: room temperature
Solvent: Arg-SEC Mobile Phase (Standard, Nacalai Tesque, Inc.)
Flow rate: 0.8 ml/min
Detection: UV 280 nm
FL (Ex. 295 nm/Em. 335 nm)

### Test Example 15: Modification of Fab Antibody with SH-PEG-NH₂ Linker (1K)

### (1) Coupling of SH-PEG-NH₂ Linker (1K) with Fab Antibody

The aforementioned (g) SH-PEG-NH₂ linker (1K) (Creative PEGWorks) was bound to HBs628Fab-Qtag, while catalyzed by BTGase. Operational details were as follows. HBs628Fab-Qtag was dissolved in MES buffer solution (50 mM MES, 2 mM EDTA, pH 7.0) so as to adjust the concentration to 10 µM, and the solution was incubated with 50 equivalents of SH-PEG-NH₂ linker (1K) and 0.1 U/mL of BTGase at 25°C for 3 hours. Progress of the reaction was monitored by SEC. Activity of BTGase was assayed by the hydroxamate method. The reaction solution was desalted, concentrated, and purified with Amicon 10K (Merck).

### (2) ALP Labeling of SH-PEG(1K)-Fab

Ten micromoles of SH-PEG(1K)-Fab and 5 µM of maleimide-modified ALP were incubated in a 25 mM triethanolamine buffer (1 mM MgCl₂, 0.1 mM ZnCl₂, 150 mM NaCl, pH7.0) at 5°C overnight. The reaction solution was analyzed by HPLC with Superdex 200 Increase (Cytiva). Analytical conditions for SEC were as follows.

### (3) Analytical Conditions for SEC

Column: Superdex 200 Increase 10×300 mm (Cytiva)
HPLC apparatus: Chromaster (registered trademark) (Hitachi High-Tech Science Corporation)
Analysis temperature: room temperature
Solvent: Arg-SEC Mobile Phase (Standard, Nacalai Tesque, Inc.)
Flow rate: 0.8 ml/min
Detection: UV 280 nm
FL (Ex. 295 nm/Em. 335 nm)

### Results

### Results of the above tests will be described below.

### 1. Analytical Results of SH-PEG-NH₂ Linker (2K) by Infusion MS

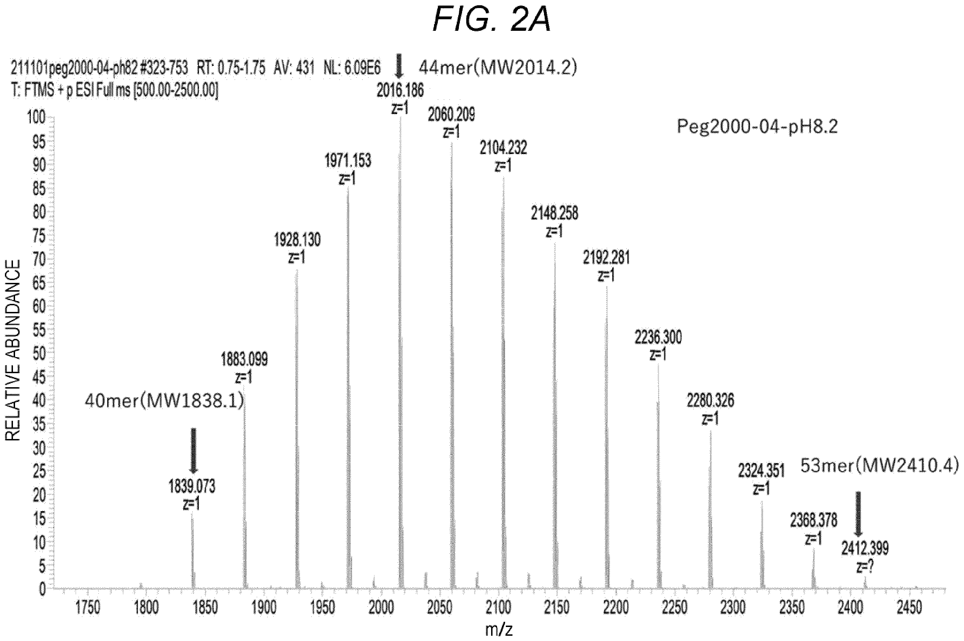

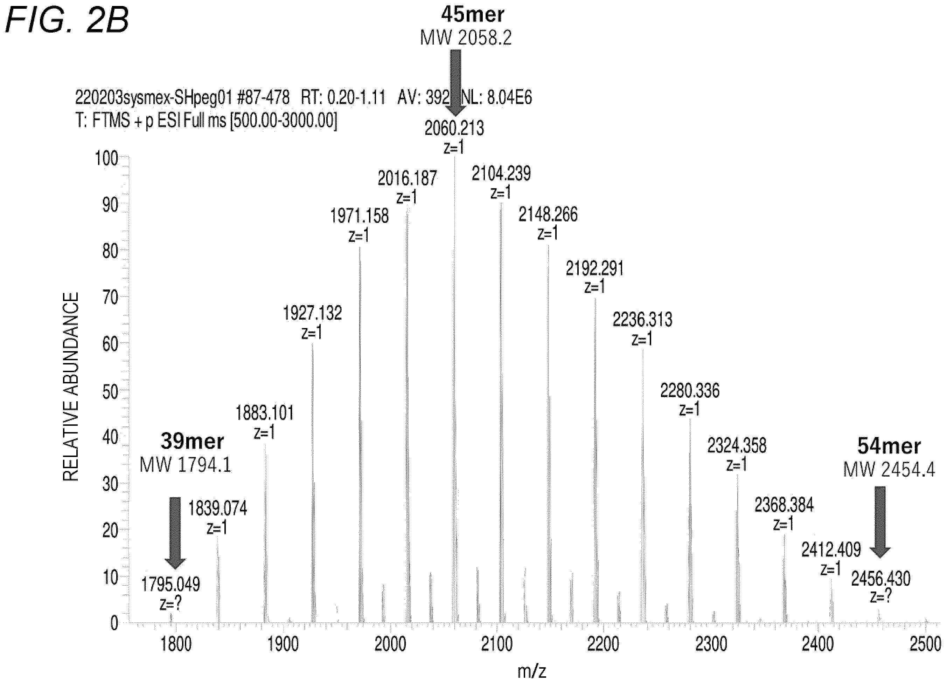

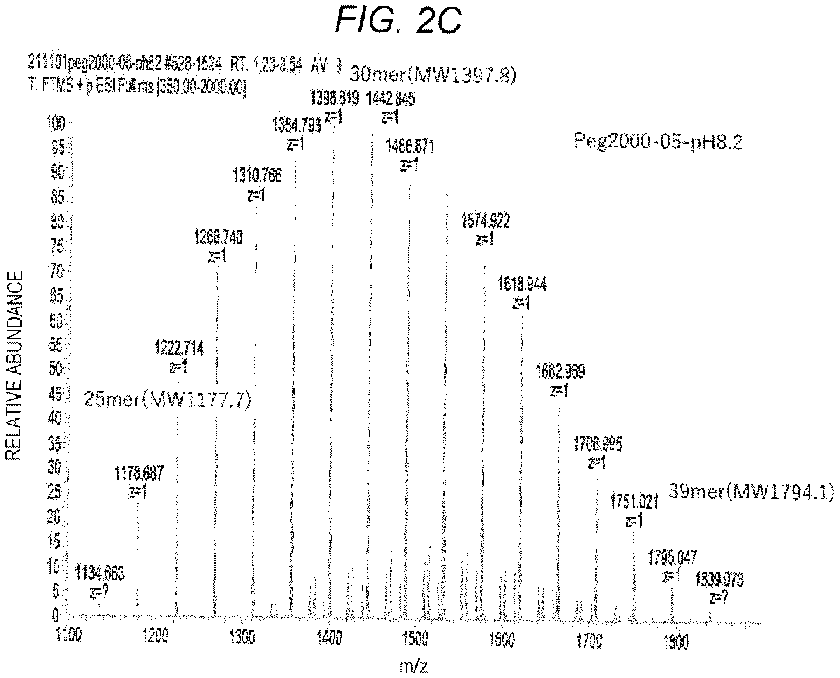

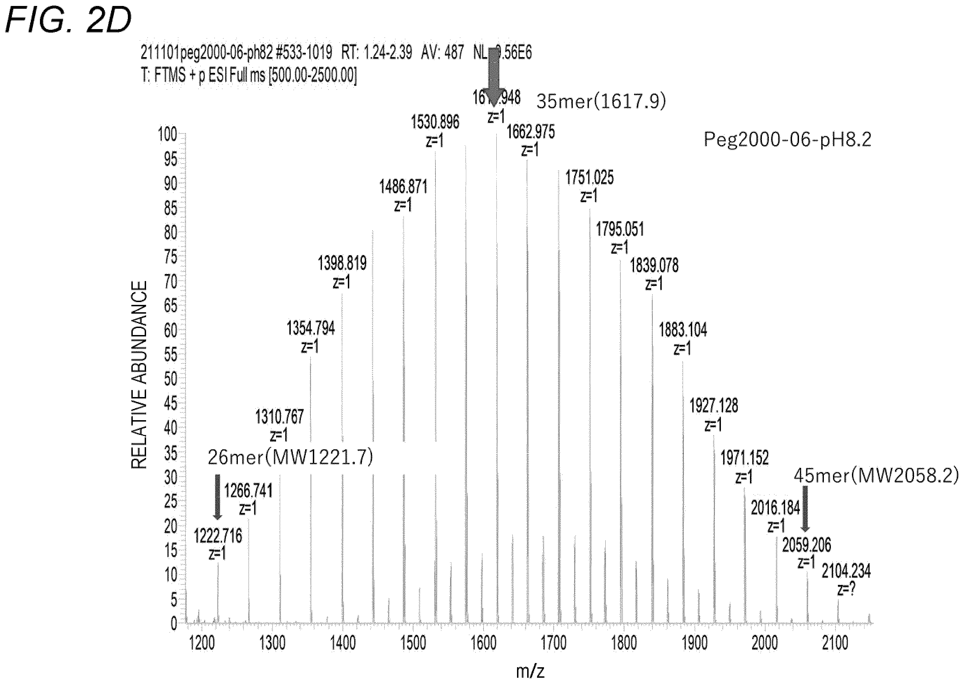

Analytical results of the individual SH-PEG-NH₂ linkers (2K) (a), (a'), (b) and (c) obtained by Infusion MS were shown in Figs. 2A, 2B, 2C and 2D. In Figs. 2A, 2B, and 2D, arrows indicate a peak corresponded to the minimum value of the molecular weight, a peak corresponded to the most abundant molecular weight, and a peak corresponded to the maximum value of the molecular weight. The most abundant molecular weight of the linkers of (a), (a'), (b) and (c) were 2014.2, 2058.2, 1397.8 and 1617.9, respectively. Also weight average molecular weight of the PEG chain was estimated from the analytical results. As can be seen from Figs. 2A, 2B, 2C, and 2D, the individual linkers demonstrated different weight-average molecular weights and molecular weight distributions. This will be detailed below. Note that the weight-average molecular weight was estimated from MS peaks with an S/N of 5 or larger for the linkers other than (a'); meanwhile estimated from peaks with an S/N of 1.5 or larger for the linker (a').

- Linker (a): weight average molecular weight 2095.2, molecular weight distribution 1838.1 to 2410.4
- Linker (a'): weight average molecular weight 2029.5, molecular weight distribution 1794.1 to 2454.4
- Linker (b): weight average molecular weight 1460.0, molecular weight distribution 1177.7 to 1794.1
- Linker (c): weight average molecular weight 1611.9, molecular weight distribution 1221.7 to 2058.2

In all linkers, the PEG chain moiety, exclusive of the functional groups (SH-CH₂CH₂- and -NH₂) was found to have a molecular weight of 1100 or larger. The weight-average molecular weight of the PEG chain moiety, subtracted the weight ofthe functional groups, was found to be 1300 or larger. The SH-PEG-NH₂ linker (2K) after adding the BTGase reaction solution (no NaCl added, pH 8.2) was not found to dimerize while interposed by a disulfide (S-S) bond, and only the MS spectrum of the monomer, correlated with the molecular weight distribution of the PEG chain, was detected. The results demonstrated that none of the SH-PEG-NH₂ linkers (2K) (a), (a'), (b) and (c) caused dimerization in the BTGase reaction solution.

### 2. BTGase-Catalyzed Modification of csF001-5Fab-Qtag with SH-PEG-NH₂ Linker (2K) (Test Example 1)

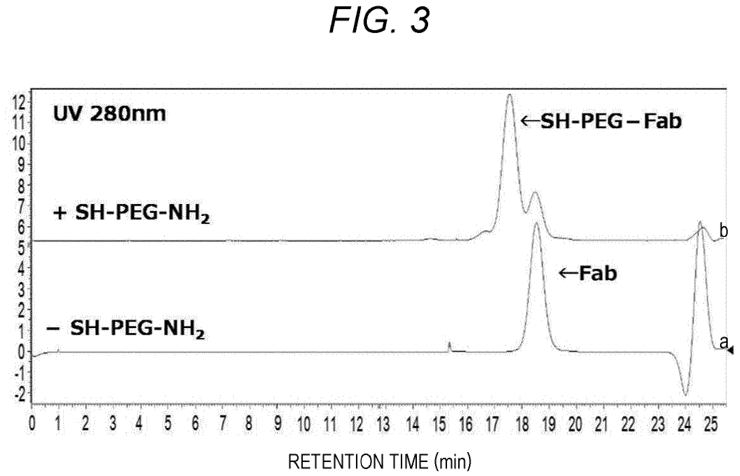

SEC analytical results of a BTGase-catalyzed reaction product formed between csF001-5Fab-Qtag and the (a) SH-PEG-NH₂ linker (2K) (Sigma-Aldrich) were shown in Fig. 3. The chart is specifically a chromatogram of the desalted and concentrated reaction solution. In the chart, "+ SH-PEG-NH₂" relates to an analytical result of the reaction solution with addition of the SH-PEG-NH₂ linker (2K), and "- SH-PEG-NH₂" relates to an analytical result of a reaction solution without addition of the SH-PEG-NH₂ linker (2K). As seen in Fig. 3, the case without addition of the linker demonstrated no change in the Fab peak. This indicates that there was no intermolecular dimerization of Fabs occurred between the Gln residue of the Q-tag and the Lys residue of the Fab. On the other hand, in the case with addition of the linker, the peak assignable to the unmodified Fab decreased at around a retention time of 18.5 minutes, meanwhile a new peak shifted towards the higher molecular weight region up to around a retention time of 17.5 minutes was confirmed. This indicates that the SH-PEG-Fab, having one straight chain SH-PEG-NH₂ linker molecule bound while interposed by the Q-tag, appeared to increase the apparent size, and was observed as a separate peak. In fact, the reason why the peak is broader than the unmodified Fab peak is that the PEG chain in the linker has a wide molecular weight distribution as shown in Figs. 2A to 2D. Reaction efficiency estimated from the peak area was approximately 73%. This tendency was similar to that in a prior case of BTGase-catalyzed modification with high molecular weight PEG linker (see Sato H., et al., Biochemistry, 35 (40) 13072 -13080 (1996)).

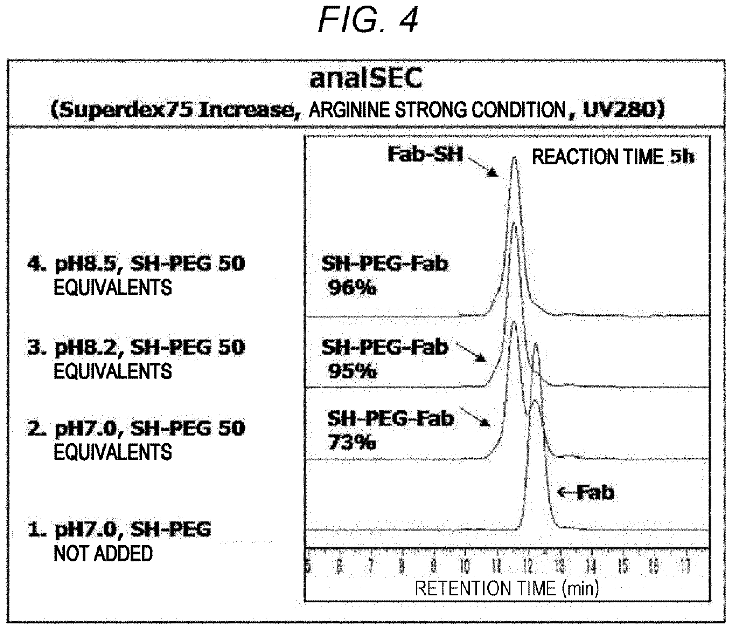

Fig. 4 illustrates analytical results obtained while pH was changed from 7.5 to 8.2 or 8.5, aiming at enhancing the introduction efficiency catalyzed by BTGase. In the chart, "SH-PEG" represents the aforementioned (a) SH-PEG-NH₂ linker (2K) (Sigma-Aldrich). It was confirmed from Fig. 4 that the amino group of the SH-PEG-NH₂ linker demonstrated enhanced nucleophilicity against a thioether bond intermediate formed between the active SH group of BTGase and the Gln side chain of Fab-Qtag, thereby improving the reaction efficiency from 73% to approximately 95% and 96%, respectively, proving feasibility of quantitative introduction. Judging from that the reaction efficiency at pH 7.0 remained approximately 73% as before, elevation of pH in the BTGase-catalyzed reaction up to around 8 to 8.5 was found to enable quantitative introduction of the SH-PEG-NH₂ linker.

### 3. BTGase-Catalyzed Modification of csF028-22Fab-Qtag with SH-PEG-NH₂ Linker (2K) (Test Example 1)

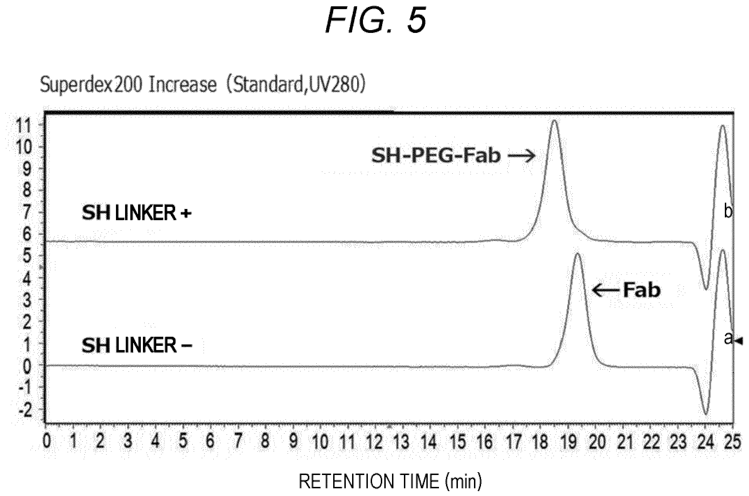

An SEC analytical result of a product of the BTGase-catalyzed reaction between csF028-22Fab-Qtag and the aforementioned (a) SH-PEG-NH₂ linker (2K) (Sigma-Aldrich), under conditions aimed at enhanced introduction efficiency (pH 8.2 or 8.5), was shown in Fig. 5. In the chart, "SH linker +" relates to an analytical result of the reaction solution with addition of the SH-PEG-NH₂ linker (2K), and "SH Linker -" relates to an analytical result of a reaction solution without addition of the SH-PEG-NH₂ linker (2K). As seen in Fig. 5, the case without addition of the linker demonstrated no change in the Fab peak, similarly to the case with csF001-5Fab-Qtag. This indicates that there was no intermolecular dimerization of Fabs occurred between the Gln residue of the Q-tag and the Lys residue of the Fab. On the other hand, in the case with addition of the linker, the peak assignable to the unmodified Fab decreased at around a retention time of 19.3 minutes, meanwhile a new broad peak shifted towards the higher molecular weight region up to around a retention time of 18.2 minutes was confirmed. This indicates that the SH-PEG-Fab, having one straight chain SH-PEG-NH₂ linker molecule bound while interposed by the Q-tag, appeared to increase the apparent size, and was observed as a separate peak, although not as a completely separated peak. The reason why the peak is broader than the peak of the unmodified Fab is ascribed to a wide molecular weight distribution of the PEG chain in the linker. The reaction efficiency was estimated to be 96%, due to incomplete separation of the peak from that of the unmodified Fab. In fact, the peak after fractionation appeared to be a single broad peak.

### 4. BTGase-Catalyzed Modification of csF001-5Fab-Qtag with SH-PEG-NH₂ Linker (3.5K) (Test Example 12)

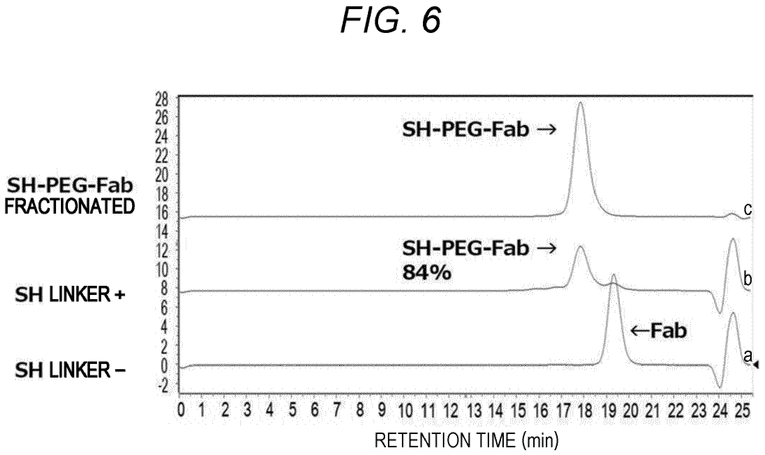

An SEC analytical result of a product of the BTGase-catalyzed reaction between csF001-5Fab-Qtag and the aforementioned (d) SH-PEG-NH₂ linker (3.5K) (Sigma-Aldrich), under conditions aimed at enhanced introduction efficiency, was shown in Fig. 6. In the chart, "SH linker +" relates to an analytical result of the reaction solution with addition of the SH-PEG-NH₂ linker (3.5K), and "SH Linker -" relates to an analytical result of a reaction solution without addition of the SH-PEG-NH₂ linker (3.5K). The case without addition of the linker demonstrated no change in the Fab peak, similarly to the case with csF001-5Fab-Qtag. This indicates that there was no intermolecular dimerization of Fabs occurred between the Gln residue of the Q-tag and the Lys residue of the Fab. On the other hand, in the case with addition of the linker, the peak assignable to the unmodified Fab decreased at around a retention time of 19.3 minutes, meanwhile a new broad peak shifted towards the higher molecular weight region up to around a retention time of 17.8 minutes was confirmed. This indicates that the SH-PEG-Fab, having one straight chain SH-PEG-NH₂ linker molecule bound while interposed by the Q-tag, appeared to increase the apparent size, and was observed as a separate peak, although not as a completely separated peak. The reason why the peak is broader than the peak of the unmodified Fab is ascribed to a wide molecular weight distribution of the PEG chain in the linker. The reaction efficiency was approximately 84%, which was slightly lower than that in the reaction with use of the SH-PEG-NH₂ linker (2K). This was presumably because the SH-PEG-NH₂ linker (3.5 K) has the molecular chain longer than that of the SH-PEG-NH₂ linker (2 K), thus slightly reducing the substrate reactivity of BTGase. In fact, the peak after fractionation appeared to be a single broad peak.

### 5. ALP Labeling of SH-PEG-Fab Derived from csF001-5Fab-Qtag (Test Example 3)

### (1) SEC Analysis of Coupling Reaction Solution

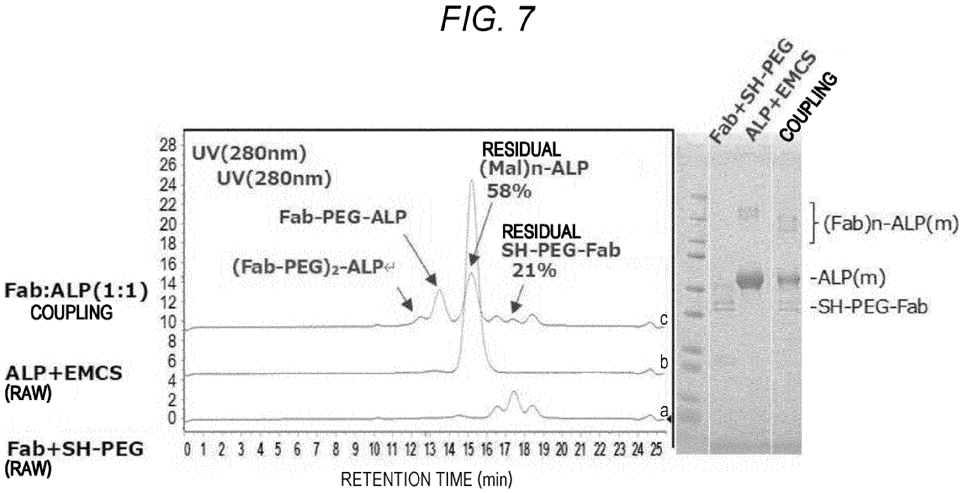

Analytical results of SH-PEG(2K)-Fab derived from csF001-5Fab-Qtag, maleimide-modified ALP ((Mal)ₙ-ALP), and a coupling reaction solution of them, analyzed by SEC and non-reducing SDS-PAGE were shown in Fig. 7. In the chart, "Fab+SH-PEG" represents an analytical result of the solution of SH-PEG(2K)-Fab, "ALP+EMCS" represents an analytical result of the solution that contains the maleimide-modified ALP, and "Fab:ALP (1: 1) coupling" and "Coupling" represent analytical results of the coupling reaction solution of SH-PEG(2K)-Fab and maleimide-modified ALP. Despite only a small mixing ratio of Fab and ALP such as 1:1, only approximately 20% of the peak assignable to SH-PEG (2K)-Fab (at a retention time of around 17.5 minutes) remained, and also the peak assignable to (Mal)ₙ-ALP (at a retention time around 15.3 minutes) decreased. A main peak presumably assignable to a complex having one ALP molecule and one Fab molecule bound thereto (Fab-PEG-ALP, at a retention time around 13.5 min), and a sub-peak presumably assignable to a complex having two Fab molecules bound thereto ((Fab-PEG)₂-ALP, at a retention time around 12.5 min), were confirmed. In fact, also referring to the results of non-reducing SDS-PAGE of the individual raw materials and reaction solutions (corresponding to the bands assignable to the ALP monomer (ALP(m), derived from homodimer structure of ALP molecule), the peak separability of the Fab-coupled product from the unmodified ALP was very high. This was ascribed to the increased apparent size, as a result of binding of the Fab molecule to ALP while interposed by the straight chain PEG molecule.

### (2) SEC Fractionation of Coupling Reaction Peaks, and SEC Analysis of Preparative Fractions

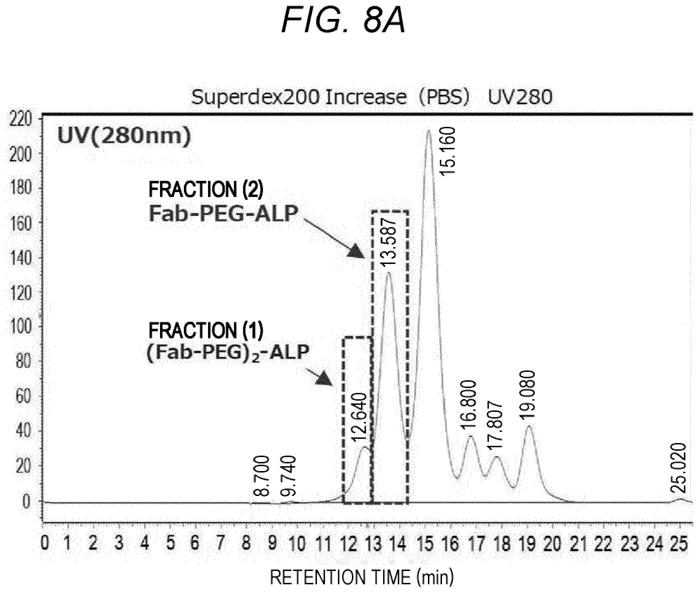

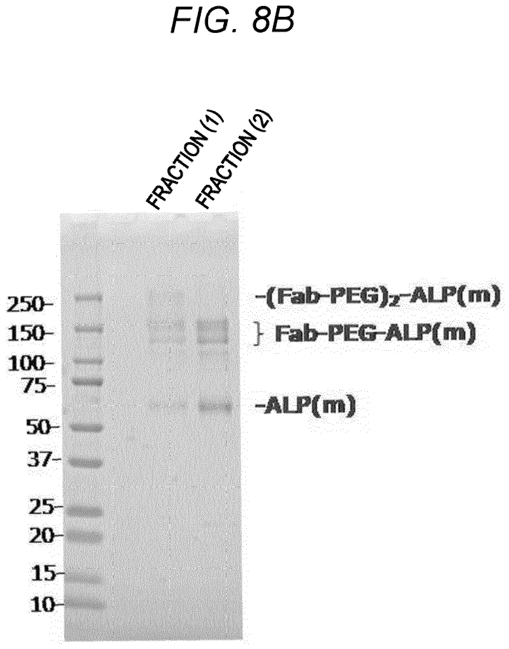

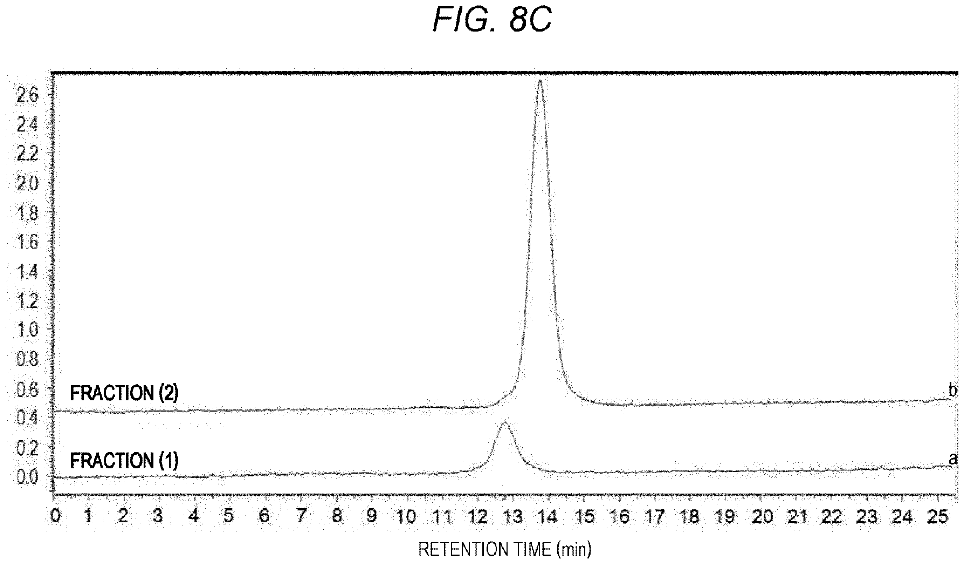

A coupling reaction solution that contains SH-PEG(2K)-Fab derived from csF001-5Fab-Qtag, and the maleimide-modified ALP was fractioned by SEC, and the preparative fractions were analyzed by non-reducing SDS-PAGE and SEC. Results were shown in Figs. 8A to 8C. In Fig. 8A, two peaks surrounded by the broken line, in the sequential order from earlier to later retention time, were concluded to be ALP-labeled Fab having ALP and two Fab molecules bound thereto ((Fab-PEG)₂-ALP); and ALP-labeled Fab having one ALP molecule and one Fab molecule bound thereto (Fab-PEG-ALP). In fact, Fab-PEG-ALP was detected in Fig. 8B, as a band attributable to ALP(m) (molecular weight approx. 60000), and as two bands that reside in the range of molecular weight from approximately 100000 to 130000, presumably attributable to products with one Fab molecule bound thereto. The two bands were detected for ALP (m), presumably because the maleimide modification of the ALP molecule occurred randomly to the side chain of the Lys residue of the ALP molecule, so that apparently two sizes of Fab-ALP monomer conjugate species were produced, depending on the binding site of SH-PEG-Fab to ALP, conjugatedby SH-PEG. There has been an actual case reporting that a random single molecular conjugate of a straight chain high-molecular-weight PEG chain was confirmed as two bands in electrophoresis (see Sato H., Advanced Drug Delivery Reviews, 54, 487-504 (2002)). This was presumably ascribed to an influence of random modification of ALP molecule, by the EMCS linker on ALP bound to Fab while interposed by SH-PEG. A band attributable to the unmodified ALP was almost not observed for (Fab-PEG)₂-ALP in the electrophoresis. The main component was Fab-PEG-ALP that appeared as two bands with the molecular weight ranged from approximately 100000 to 130000. One additional band was detected at around a molecular weight of 200000, which was presumably attributable to (Fab-PEG)₂-ALP. These results were reasonable. Among them, Fab-PEG-ALP was selected for performance evaluation. As shown in Fig. 8C, both purified products demonstrated a single peak in the SEC analysis, proving their high purity. The reason why the molecular weight estimated from the electrophoretic band of the purified product was detected to be larger than the sum of the molecular weights of the individual proteins, was presumably because the conjugate species had increased apparent size as a result of binding while interposed by the straight chain high-molecular-weight PEG (2K).

### 6. ALP Labeling of SH-PEG-Fab Derived from csF028-22Fab-Qtag (Test Example 3)

### (1) SEC Analysis of Coupling Reaction Solution

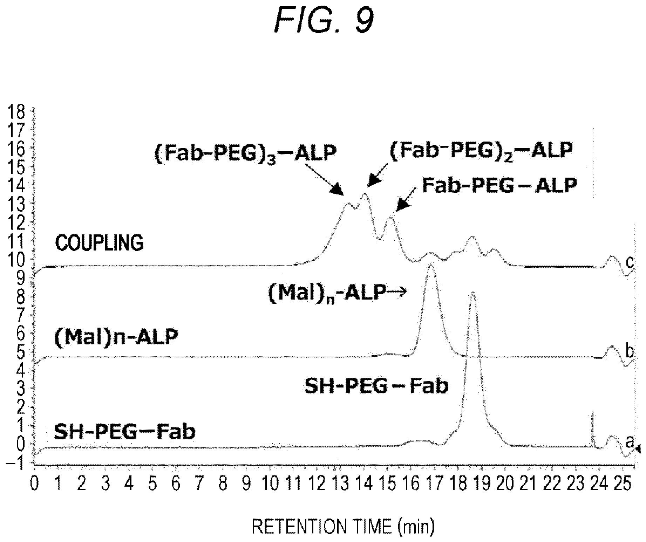

Analytical results of SH-PEG(2K)-Fab derived from csF028-22Fab-Qtag, maleimide-modified ALP ((Mal)ₙ-ALP), and a coupling reaction solution of them, analyzed by SEC were shown in Fig. 9. In the chart, "Coupling" represents an analytical result of the coupling reaction solution of SH-PEG(2K)-Fab and maleimide-modified ALP. Despite only a small mixing ratio of Fab and ALP such as 3:1, only approximately 30% of the peak assignable to SH-PEG(2K)-Fab (at a retention time of around 18.4 minutes) remained, and also the peak assignable to (Mal)ₙ-ALP (at a retention time around 15.3 minutes) was found to remain only as much as 10%. The reaction efficiency was found to be high, similarly to as in other ALP coupling. A main peak presumably assignable to a complex having one ALP molecule and three Fab molecules bound thereto ((Fab-PEG)₃-ALP, at a retention time around 12.8 minutes) was confirmed, in addition to a sub-peak presumably assignable to Fab-PEG-ALP (at a retention time around 14.8 minutes), and a sub-peak presumably assignable to (Fab-PEG)₂-ALP (at a retention time around 13.8 minutes).

### (2) SEC Fractionation of Coupling Reaction Peaks, and SEC Analysis of Preparative Fractions

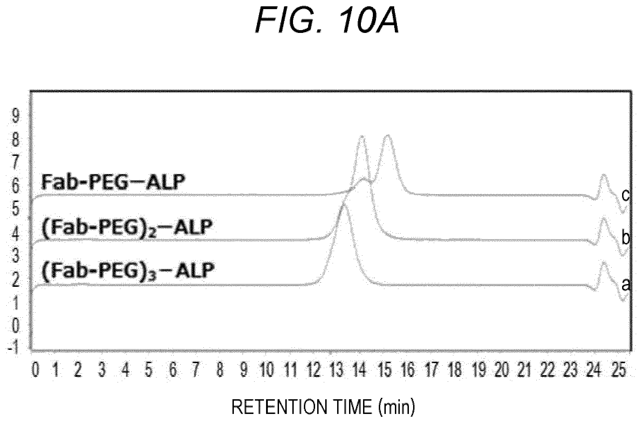

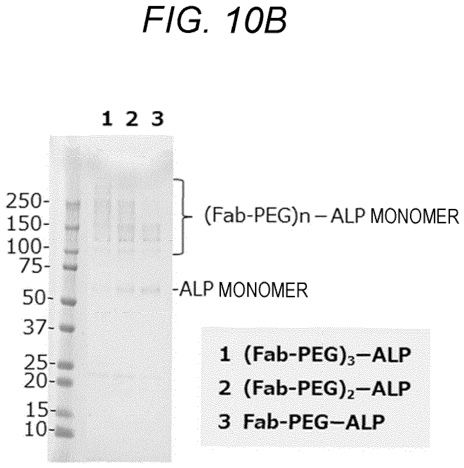

A coupling reaction solution that contains SH-PEG(2K)-Fab derived from csF028-22Fab-Qtag, and the maleimide-modified ALP ((Mal)ₙ-ALP) was fractioned by SEC, and the preparative fractions were analyzed by non-reducing SDS-PAGE and SEC. Results were shown in Figs. 10A and 10B. Referring to Fig. 10B, each of the thus obtained fractions demonstrated bands that correspond to the profile of the ALP monomer-modified product, according to the presumed number of bindings of Fab. The individual peaks in Fig. 10A were concluded to be (Fab-PEG)₃-ALP, (Fab-PEG)₂-ALP, and Fab-PEG-ALP, in the sequential order from earlier to later retention time. (Fab-PEG)₃-ALP and (Fab-PEG)₂-ALP demonstrated high purity, meanwhile Fab-PEG-ALP demonstrated slightly lower purity. All fractions were, however, confirmed to contain the main products in the main peaks.

### 7. Coupling Reaction between sF001-5 Fab' and Maleimide-Modified ALP (Test Example 4)

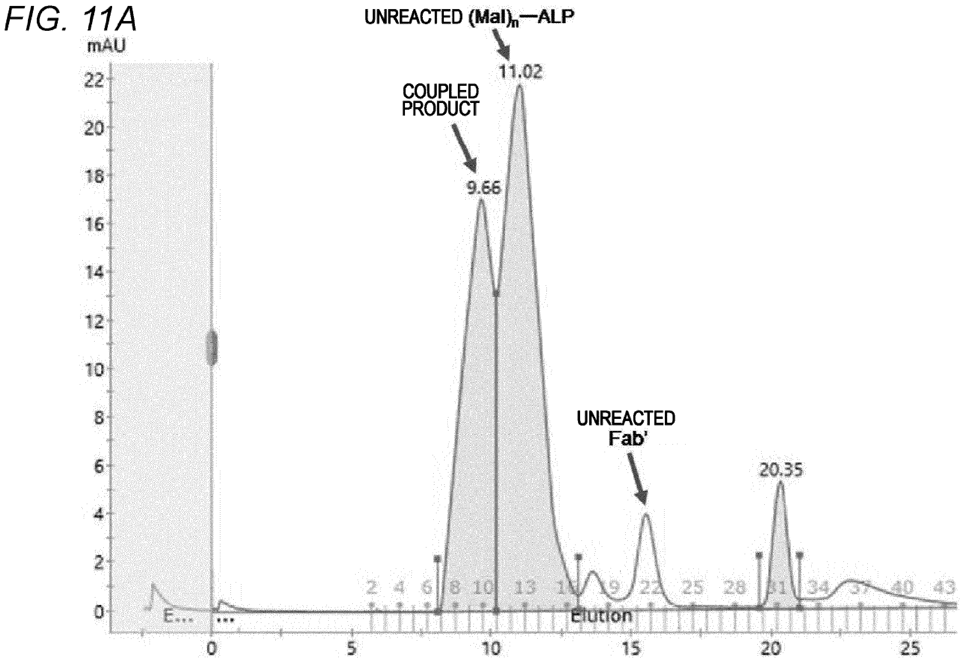

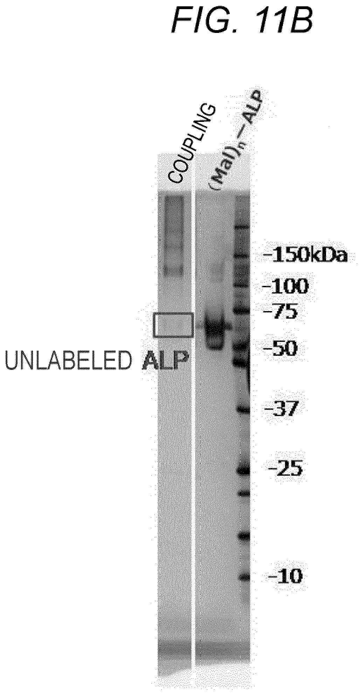

A chromatogram obtained in SEC fractionation of the coupling reaction solution that contains sF001-5 Fab' and (Mal)ₙ-ALP, and analytical results of the preparative individual fractions analyzed by non-reducing SDS-PAGE were shown in Figs. 11A and 11B. While the ALP labeling of SH-PEG-Fab derived from csF001-5Fab employed a mole proportion of SH-PEG-Fab and (Mal)ₙ-ALP of 1:1 (see Test Example 3), the present reaction employed a coupling proportion Fab':ALP of 5:1. Hence as shown in Fig. 11A, the peak area for unreacted (Mal)ₙ-ALP was larger than the peak area for the coupled product. This means that the coupling efficiency of the SH group in the side chain of Cys residue in sF001-5 Fab' with (Mal)ₙ-ALP was lower than the coupling efficiency of SH-PEG-Fab derived from csF001-5 Fab with (Mal)ₙ-ALP. In fact, the maleimide modification rates of ALP used in the SH-PEG linker method and the random labeling method, given in terms of the amount of addition of the EMCS reagent, were found to be 20 equivalents for the former, meanwhile as high as 30 equivalents for the latter. Judging from multiple SDS-PAGE bands actually observed as a result of the EMCS modification, also the coupling reaction according to the random labeling was considered to demonstrate high modification rate of ALP. These results proves that the SH-PEG linker method demonstrated a remarkably high coupling efficiency between SH-PEG-Fab and maleimide-modified ALP, as compared with the random labeling method.

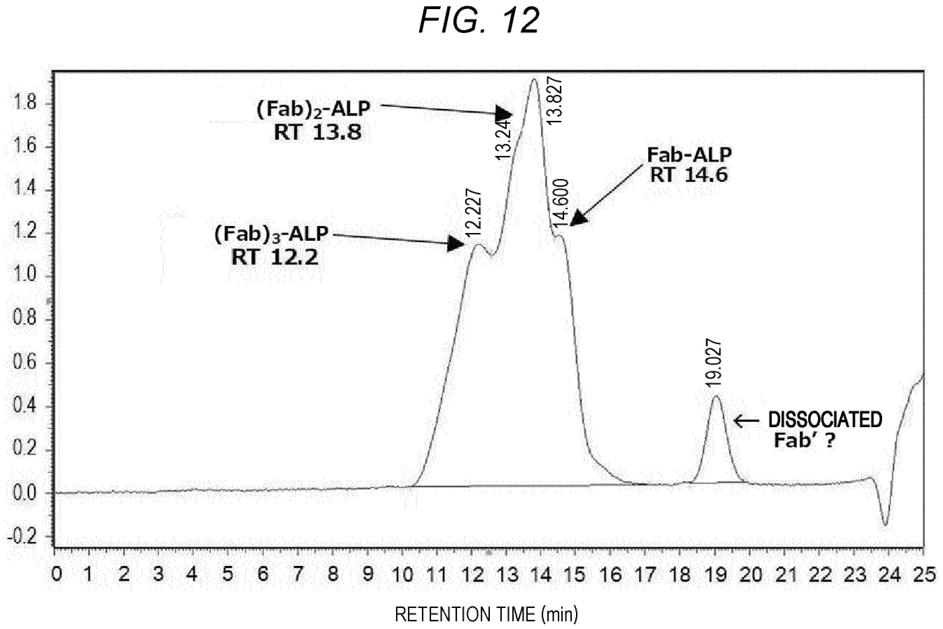

Although ALP normally exists as a 150-kDa dimer having two 75-kDa molecules combined therein, SDS-PAGE now demonstrated ALP as a monomer, with a band appeared at around 70 kDa, as shown in Fig. 11B. On the other hand, the coupled product demonstrated multiple bands at or above 100 kDa. Unlike the conjugate formed with the aid of the PEG-SH linker, the coupled product was considered to demonstrate bands assignable to one-Fab-conjugate, two-Fab-conjugate, and multiple-Fab-conjugate, reflecting the apparent sizes. The coupled products having higher molecular weights were also suspected to contain those having the ALP monomer bound to two Fab' molecules, while partially leaving the S-S bonds between the H chain and the L chain in a reduced form, since the product has not gone through any oxidation process after reduced. (Fab)ₙ-ALP to be used in the following evaluation was, however, found to contain almost no unlabeled ALP, since almost no band attributable to the unlabeled ALP was detected from the preparative fraction. A result of SEC analysis of the purified (Fab)ₙ-ALP sample is shown in Fig. 12. Unlike the result of SEC analysis of a homogeneous complexes of SH-PEG-Fab derived from csF001-5Fab with ALP ((Fab-PEG)₂-ALP and Fab-PEG-ALP) (see Fig. 5), Fig. 12 demonstrated a broad SEC peak assignable to (Fab)ₙ-ALP, indicating low peak separability among the number of bindings of Fabs. The reason why is that the former increased the apparent size due to binding of Fab and ALP while interposed with the PEG chain, thereby enhancing the SEC separability, meanwhile (Fab)ₙ-ALP, being a coupled product having EMCS as a short-chain linker, did not increase the apparent molecular weight, thus leaving the separability low. The broadness of the individual peaks for (Fab-PEG)₂-ALP and Fab-PEG-ALP, with different numbers of binding of Fab, is ascribed to that the coupled product of the random labeling has (Fab)s bound to multiple sites such as Cys residue in the hinge moiety, or Cys residues between the chains, thus making the structure of ALP coupled product more heterogeneous, unlike Fab-PEG which is a coupled product as a result of hinge-moiety selective binding only to the terminal SH group of the PEG chain.

### 8. Measurement of ALP Activity of ALP-Labeled Antibody (Test Example 7)

Specific ALP activity of ALP molecule per se; and Fab-PEG-ALP, (Fab-PEG)₂-ALP and (Fab-PEG)₃-ALP derived from csF028-22Fab-Qtag, were summarized in Table 3. As summarized in Table 3, the individual ASP-labeled antibodies did not decrease the specific activity even with increase in the number of binding of (Fab-PEG)s to the ALP molecule. The preparation method of this embodiment was thus proved to prepare the ALP-labeled polypeptide with the ALP activity well maintained. The specific activities exceeding 100% were presumably ascribed to influences of purity of the fractionated samples.

**[Table 3]**

| | ALP | Fab-PEG-ALP | (Fab-PEG)₂-ALP | (Fab-PEG)₃-ALP |
|---|---|---|---|---|
| Molecular weight | 150,000 | 202,000 | 254,000 | 306,000 |
| ALP specific activity (U/nmol) | 785 | 902 | 857 | 797 |
| ALP specific activity (percentage to ALP) | 100% | 115% | 109% | 102% |

### 9. Performance Evaluation of Reagents that Contain Fab-PEG-ALP and (Fab')ₙ-ALP (Test Example 8)

### (1) Reactivity of R3 Reagent

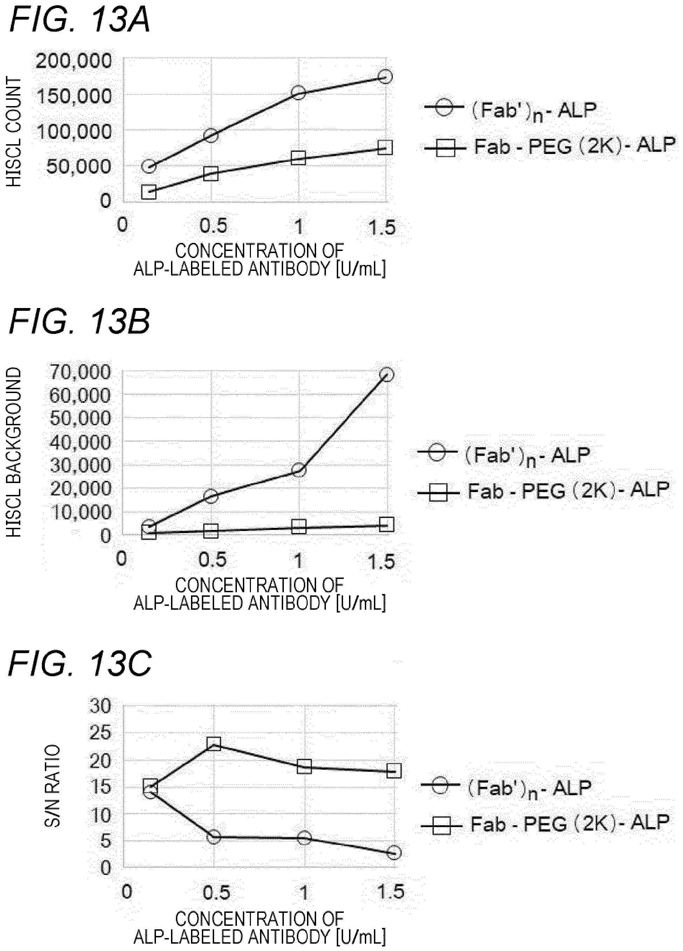

Samples in D-PBS (0.1% BSA, pH 7.4) were analyzed with use of each of Fab-PEG-ALP from csF001-5Fab-Qtag and (Fab')ₙ-ALP obtained by random labeling as R3 reagent, and the analytical results were shown in Figs. 13A to 13C, while itemizing them into signal value, noise (also referred to as background), and signal/noise (S/N). In the charts, the antibody concentration scaled on the abscissa represents the activity of ALP bound to each antibody. The measured value (count) of HISCL (registered trademark) HIV Ag+Ab reagent increased depending on the antigen concentration, thus indicating in Fig. 13A that the higher the signal value, the higher the reactivity with the antigen. Fig. 13B conversely indicated that the lower the noise value, the higher the specificity. (Fab')ₙ-ALP demonstrated relatively large signal value, but accompanied by increased noise. (Fab')ₙ-ALP therefore demonstrated, as shown in Fig. 13C, low S/N that represents the sensitivity of detection reagent. On the other hand, Fab-PEG-ALP derived from csF001-5Fab-Qtag demonstrated the signal value and noise, both smaller than those for (Fab')ₙ-ALP. In particular, the noise was suppressed very low as compared with (Fab')ₙ-ALP. As a result, Fab-PEG-ALP demonstrated the S/N, that indicates the sensitivity, four times or more as large as that of (Fab')ₙ-ALP, at 0.5 U/mL. The reason why (Fab')ₙ-ALP demonstrated higher signal values as compared with that of Fab-PEG-ALP at the same ALP activity was presumably attributable to binding of multiple Fab molecules to one ALP molecule on average. From these results, use of the enzyme-labeled antibody obtained by the production method according to the invention is expected to improve the sensitivity of an in vitro diagnostic agent.

### (2) Suppression of Increase of Background due to Serum-Derived Component

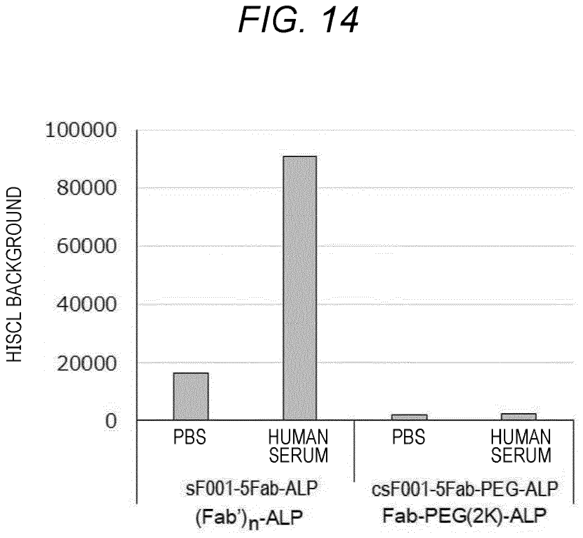

For comparison of the background, D-PBS (0.1% BSA, pH 7.4) and human pool serum were analyzed with use of each of Fab-PEG-ALP derived from csF001-5Fab-Qtag, and (Fab')ₙ-ALP as R3 reagent, and the analytical results were shown in Fig. 14. Fab-PEG-ALP derived from csF001-5Fab-Qtag demonstrated a lower background than that demonstrated by (Fab')ₙ-ALP as described above, when the sample in PBS was measured. Such difference of the background was found to be further remarkable, when the sample in human serum was measured, as shown in Fig. 14. That is, (Fab')ₙ-ALP in the serum elevated the background 5.6 times, whereas Fab-PEG-ALP demonstrated only a 1.26-fold elevation which was almost minor. Due to various proteins and lipids contained in serum, the sample in the serum often demonstrates the background higher than in the buffer. Referring to the results above, a possible reason why Fab-PEG-ALP having the SH-PEG linker interposed therein could suppress the serum-derived background from elevating, in contrast to the structurally nonuniform (Fab')ₙ-ALP, could be that Fab-PEG-ALP is structurally uniform, and is a molecular species constituted by the straight chain PEG linker whose hydrophilicity is high due to hydration. Although not shown, as a result of lowering of the background, Fab-PEG-ALP demonstrated approximately 30-fold at most elevation of the sensitivity, over the entire concentration range of p24 antigen.

### 10. Preparation of SH-PEG-Fab Derived from HBs628Fab-Qtag, and Biotin Labeling Thereof (Test Examples 1 and 2)

### (1) BTGase-Catalyzed Modification of HBs628Fab-Qtag with SH-PEG-NH₂ Linker (2K)

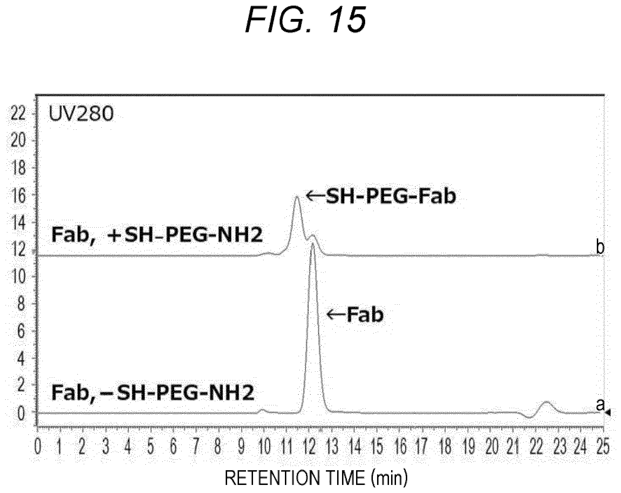

SEC analytical results of a BTGase-catalyzed reaction product formed between HBs628Fab-Qtag and the (a) SH-PEG-NH₂ linker (2K) (Sigma-Aldrich) were shown in Fig. 15. In the chart, "+ SH-PEG-NH₂" relates to an analytical result of the reaction solution with addition of the SH-PEG-NH₂ linker (2K), and "- SH-PEG-NH₂" relates to an analytical result of a reaction solution without addition of the SH-PEG-NH₂ linker (2K). As seen in Fig. 15, the case without addition of the linker demonstrated no change in the Fab peak, similarly to the case with csF001-5Fab-Qtag. This indicates that there was no intermolecular dimerization of Fabs occurred between the Gln residue of the Q-tag and the Lys residue of the Fab. On the other hand, in the case with addition of the linker, the peak assignable to the unmodified Fab decreased at around a retention time of 12.3 minutes, meanwhile a new peak shifted towards the higher molecular weight region up to around a retention time of 11.5 minutes was confirmed. This indicates that the SH-PEG-Fab, having one straight chain SH-PEG-NH₂ linker molecule bound while interposed by the Q-tag, appeared to increase the apparent size, and was observed as a separate peak. The reason why the peak is broader than the peak of the unmodified Fab is ascribed to a wide molecular weight distribution of the PEG chain in the linker. Reaction efficiency estimated from the peak area was approximately 72%, which was comparable to the result of csF001-5 Fab-Qtag.

### (2) Biotin Labeling of SH-PEG-Fab Derived from HBs628Fab-Qtag

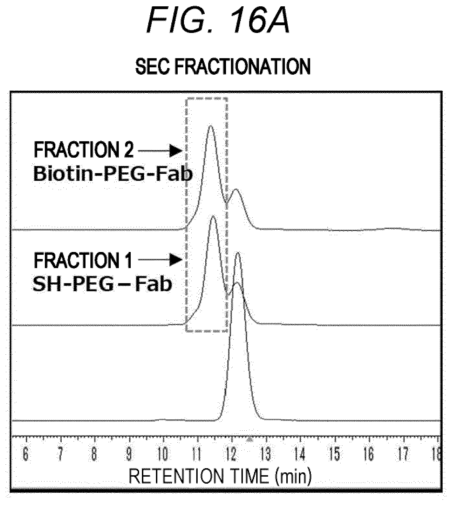

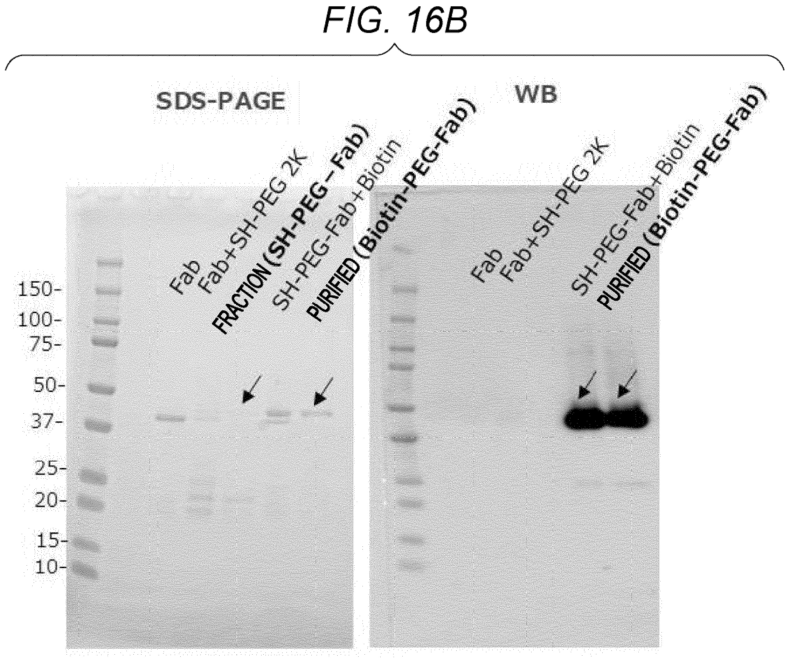

Analytical results of fractions of a reaction solution that contains csF001-5Fab-Qtag and the (a) SH-PEG-NH₂ linker (2K) (Sigma-Aldrich), collected from a peak at a retention time of 11.5 minutes in SEC analysis; and, analytical results of a reaction solution that contains SH-PEG-Fab-containing concentrated solution and Biotin-PEAC5-maleimide fractioned from a peak in SEC analysis, were shown in Figs. 16A and 16B. Peaks of the individual reaction solutions fractionated by the SEC analysis were surrounded by a broken line in Fig. 16A. Referring now to Fig. 16B that illustrates analytical results of non-reducing SDS-PAGE of the reaction solution that contains csF001-5Fab-Qtag and the SH-PEG-NH₂ linker (2K), and the fractionated sample thereof, the thus produced SH-PEG-Fab was found to slightly shift towards the higher molecular weight region at around 40 kDa (band indicated by ->), from the band of the unreacted Fab, suggesting that one SH-PEG-NH₂ linker molecule was bound. Note that in the electrophoresis, two bands were occasionally detected at around 20 kDa, suggesting that the H chain and L chain were left unbound. This was because the packing structure of Fab changed upon binding of the SH-PEG-NH₂ linker (2K), and was partially reduced by the SH group of the linker.

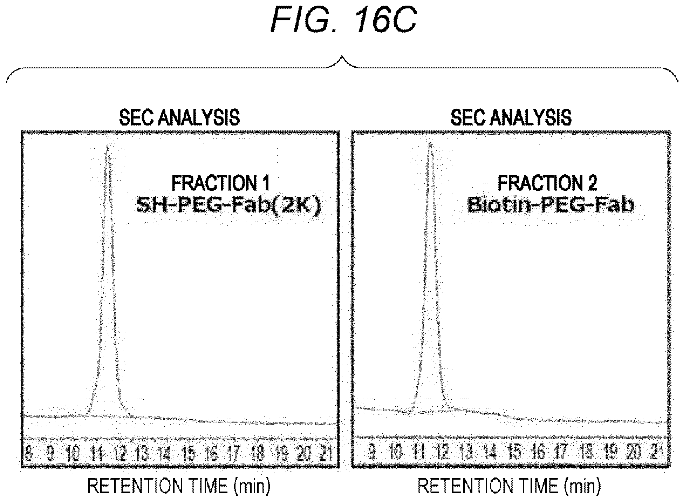

In fact, even if Fab was kept with the SH-PEG-NH₂ linker (2K) in a reaction solution in the absence of BTGase, the Fab did not cause reduction of the S-S bond between the H chain and the L chain (data not shown). As seen in Fig. 16C, each of the purified samples demonstrated a single peak in SEC, proving high purity.

Referring now to Fig. 16B that illustrates analytical results of non-reducing SDS-PAGE of the reaction solution that contains SH-PEG-Fab and Biotin-PEAC5-maleimide, and the fractionated sample thereof, there was detected one band slightly shifted from the band of the unmodified Fab, at around 40 kDa, similarly to the case with SH-PEG-Fab. The band intensity was found to reflect the peak intensity ratio of the modified fractions in the SEC fractionation chromatogram of the reaction solution. Bands assignable to the H chain and the L chain, having been observed for SH-PEG-Fab, were hardly detected, confirming that the S-S bond of Fab has been reconstructed. This was presumably because the modification with Biotin-PEAC5-maleimide stabilized the packing structure of SH-PEG-Fab, and constituted the inter-chain S-S bond having been partially cleaved. Only the reaction solution of such biotin-modified product and the fractions thereof demonstrated intense bands at the position of the product in non-reducing SDS-PAGE, indicating that SH-PEG-Fab was labeled with biotin.

### 11. LC-MS Analysis of SH-PEG Fab Derived from HBs628Fab-Qtag, and Biotin-PEG-Fab (Test Example 2)

### (1) Results of LC-MS Analysis of HBs628Fab-Qtag

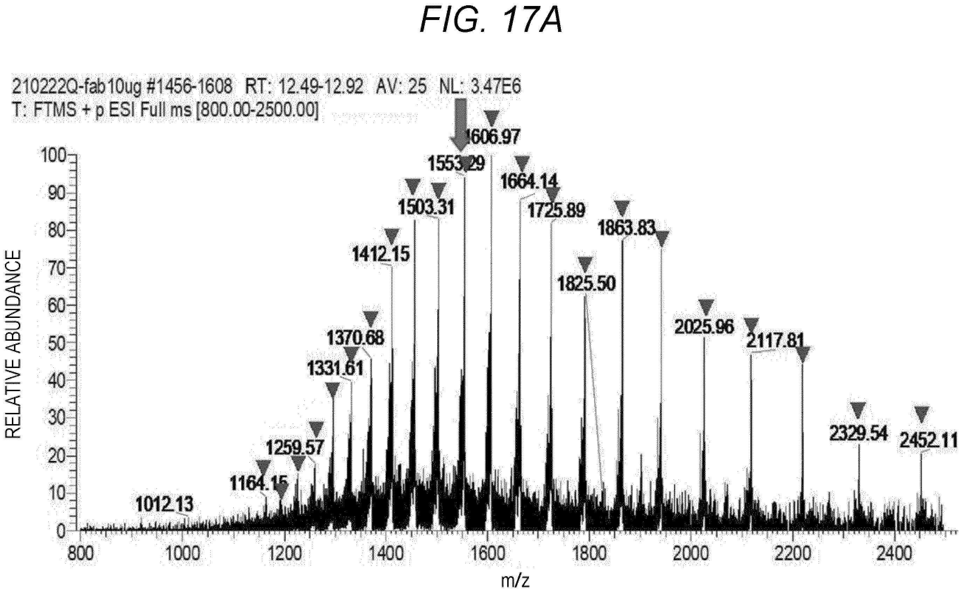

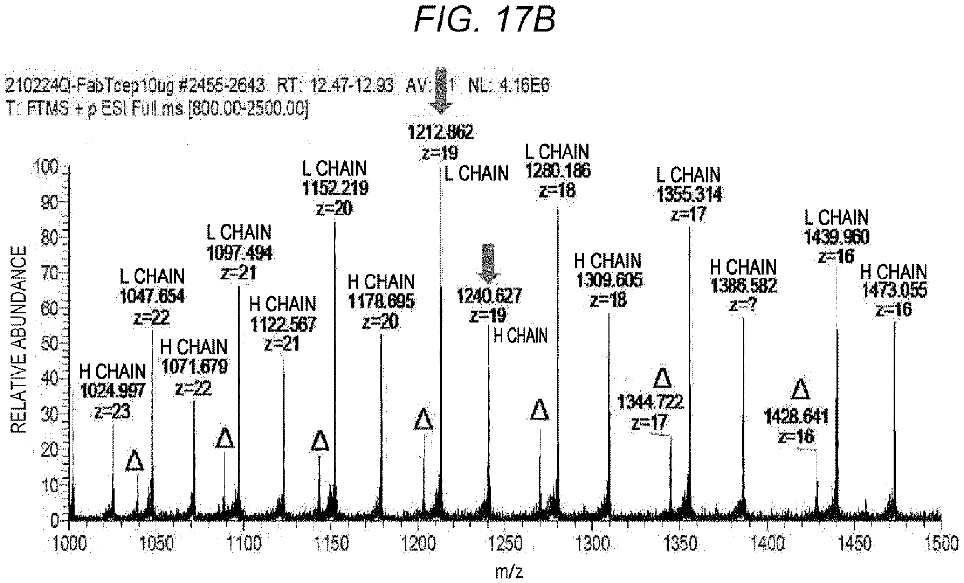

LC-MS spectra of non-reduced and reduced samples of HBs628Fab-Qtag were shown in Figs. 17A and 17B. As seen in Fig. 17A, multivalent ions from +20 to +41 valences of HBs628Fab-Qtag were observed in LC-MS of the non-reduced sample. The spectrum was found to reflect the measured molecular weight, although Cys residue would be partially reduced. On the other hand, as seen in Fig. 17B, multivalent ions of the L chain and the H chain were observed in LC-MS of the reduced sample. The L chain was observed to generate a product from which two residues at the N-terminal were removed. The L chain and the H chain were confirmed to have framework structures as designed, although pyroglutamylated at the N-termini.

### (2) Results of LC-MS Analysis of SH-PEG Fab and Biotin-PEG-Fab

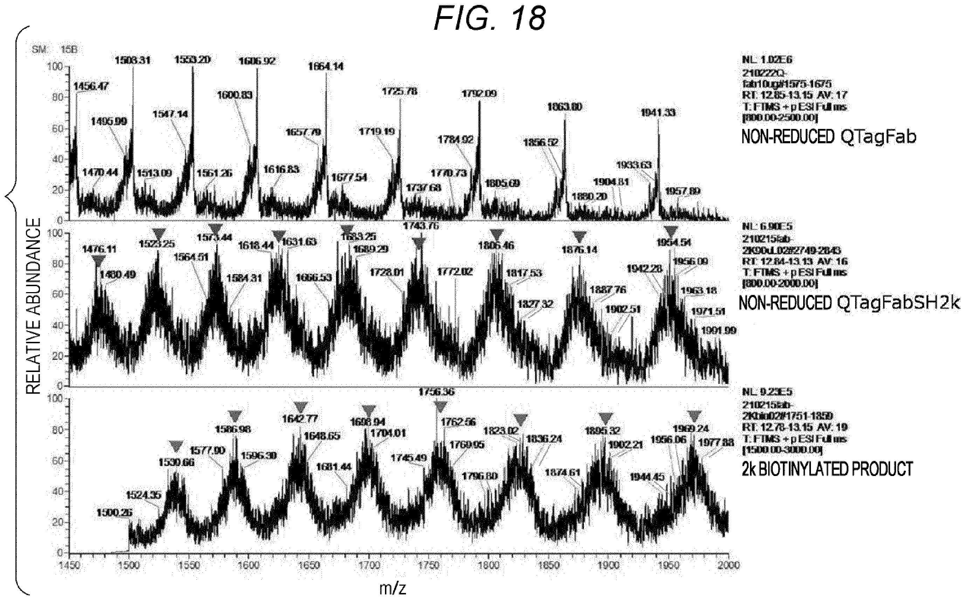

LC-MS spectra of HBs628Fab-Qtag, SH-PEG-Fab thereof, and biotin-PEG-Fab were shown in Fig. 18. In the spectra of SH-PEG-Fab and biotin-PEG-Fab respectively in the middle and lower tiers in Fig. 18, multivalent ions indicated by ▼ were observed. The signals observed were broad, reflecting the molecular weight distribution of the PEG chain. Measured average molecular weights (number average molecular weights) of the non-reduced SH-PEG-Fab and the biotin-PEG-Fab were 48710 and 49223, respectively. The differences from the measured average molecular weight (number average molecular weight) of unmodified HBs628Fab-Qtag were 2141 and 2654, respectively. This suggests that SH-PEG-Fab and biotin-PEG-Fab have, on average, one SH-PEG chain (2K) and one biotin-PEG chain (2K) bound thereto, respectively.

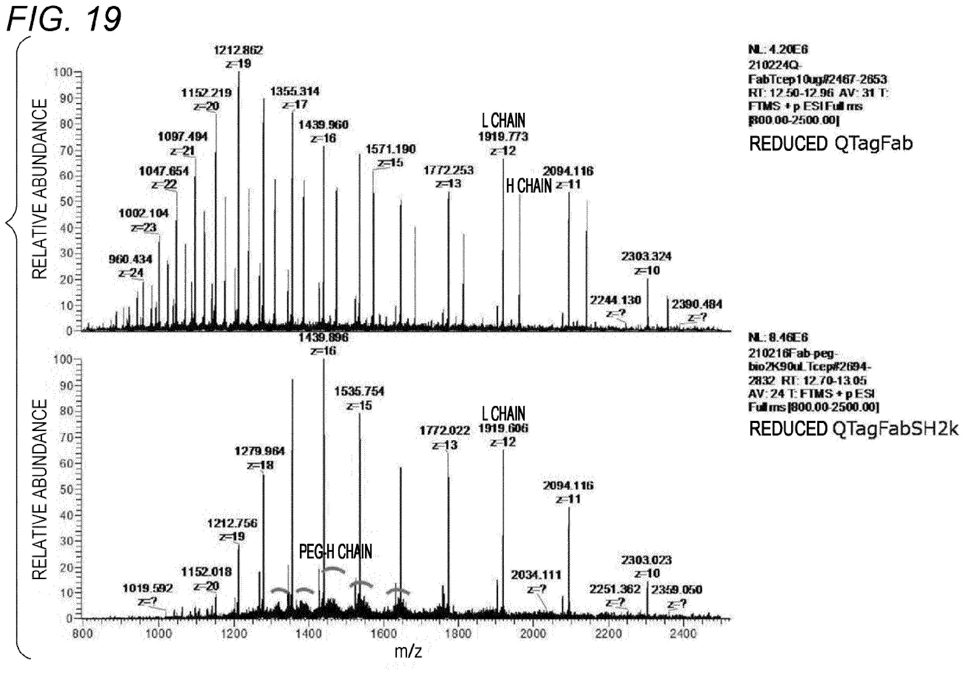

LC-MS spectra of reduced samples of HBs628Fab-Qtag and SH-PEG-Fab thereof were shown in Fig. 19. Multivalent ions of the L chain and the H chain were observed as a result of reduction. Spectral pattern of the L-chain of SH-PEG-Fab was the same as that of HBs628Fab-Qtag. On the other hand, the H chain of SH-PEG-Fab was observed as a broad signal (see areas tied with "arc line"), reflecting the molecular weight distribution of the PEG chain. The results indicate that one (a) SH-PEG-NH₂ linker (2K) (Sigma-Aldrich) molecule was bound to the H chain of HBs628Fab-Qtag, as a result of the BTGase-catalyzed reaction. Now, endogenous Gln residues in antibody, exclusive of Gln295 after removal of the glycan in the Fc region, has been known not to serve as a substrate for BTGase (see WO 2012/059882, and Jeger S., et al., Angew. Chem. Int. Ed. Engl., 49, 9995-9997 (2010)). It was therefore suggested that one SH-PEG-NH₂ linker (2K) molecule was bound to the Gln residue in the Q-tag of HBs628Fab-Qtag.

### 12. ALP Labeling of SH-PEG-Fab Derived from HBs628Fab-Qtag (Test Example 3)

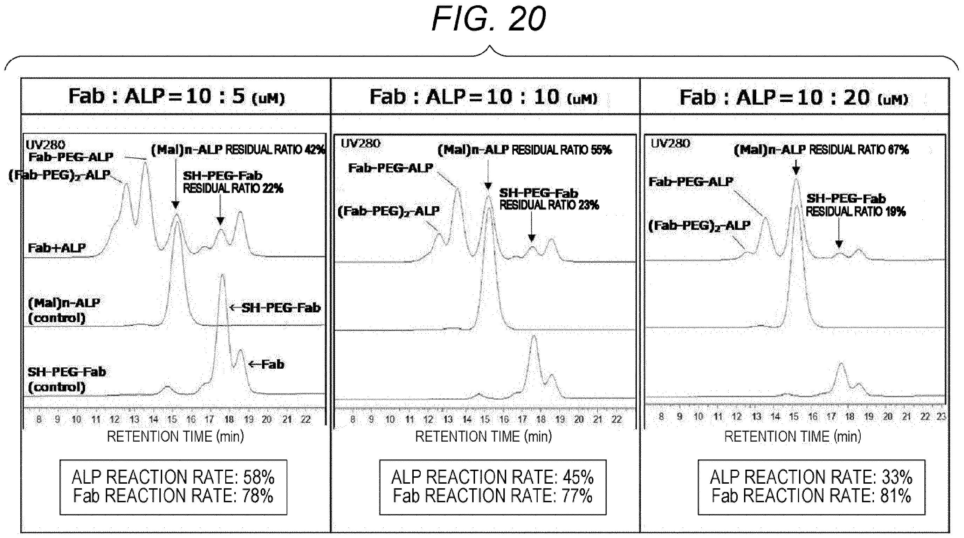

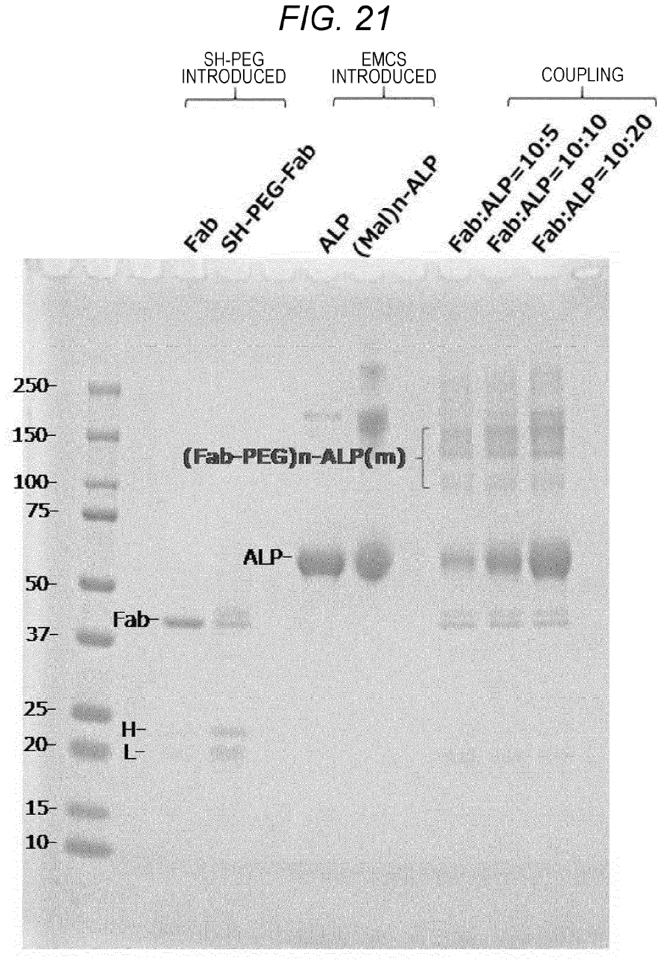

A coupling reaction solution that contains SH-PEG(2K)-Fab derived from HBs628Fab-Qtag, and the maleimide-modified ALP ((Mal)ₙ-ALP) was fractioned by SEC, and the preparative fractions were analyzed by SEC. Results were shown in Fig. 20. Analytical results of the coupling reaction solution and the individual raw materials analyzed by non-reducing SDS-PAGE were shown in Fig. 21. As seen in Fig. 20, there was no large difference in the residual ratio of SH-PEG-Fab among the reaction solutions whose Fab-Alp mixing ratios were 10 : 5, 10 : 10, and 10 : 20, when calculated from the area ratio of SEC analytical peaks. Almost 80% of the SH-PEG-Fab was found to react. The higher the mole ratio of added SH-PEG-Fab relative to (Mal)ₙ-ALP, the higher the area ratio of a peak at around a retention time of 12.3 minutes assignable to (Fab-PEG)₂-ALP. Therefore, it was demonstrated that the reaction efficiency of SH-PEG-Fab with maleimide-modified ALP was enhanced by the PEG linker effect, similarly to the result of csF001-5 Fab-Qtag. The results also demonstrated that the binding ratio of Fab to ALP is adjustable by controlling the mole ratio of reaction. When compared among such three types of reaction conditions, the higher the mole ratio of SH-PEG-Fab to be added, the lower the residual ratio of the peak assignable to (Mal)ₙ-ALP, proving it to be reasonable. As seen in Fig. 21 that illustrates the results of non-reduced SDS-PAGE of the individual raw materials and reaction solutions (corresponding to the bands assignable to the dimerized products with the ALP monomer), the number of bands presumably assignable to coupling of SH-PEG-Fab to the ALP monomer increased as the addition ratio of SH-PEG-Fab to (Mal)ₙ-ALP increased. Also results of non-reduced SDS-PAGE indicated that the coupling ratio between proteins such as Fab and ALP is adjustable, on the basis of increased ratio of bands in the higher molecular weight region.

### 13. Preparation of SH-PEG-Fab Derived from csF001-25Fab-Qtag, and Fluorescence Labeling Thereof (Test Examples 1 and 5)

### (1) BTGase-Catalyzed Modification of csF001-25Fab-Qtag with SH-PEG-NH₂ Linker (2K)

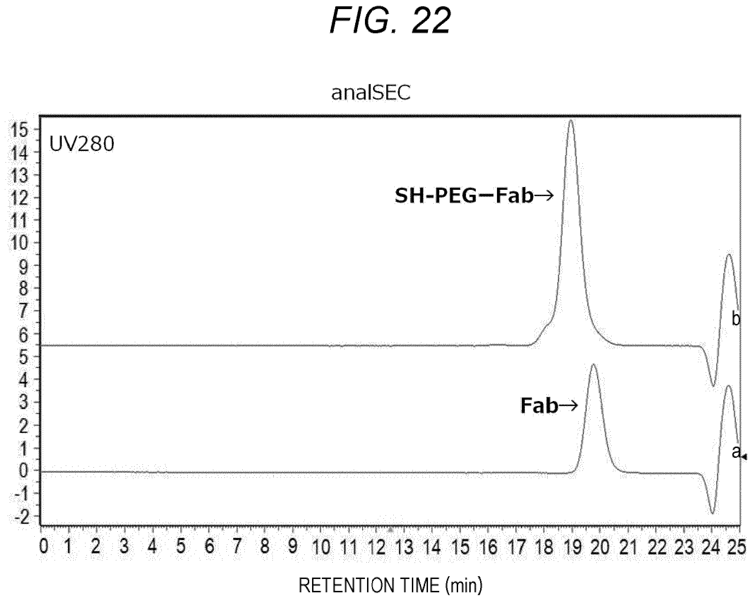

An SEC analytical result of a reaction solution, desalted and concentrated after the BTGase-catalyzed reaction between csF001-25Fab-Qtag and the aforementioned (a) SH-PEG-NH₂ linker (2K) (Sigma-Aldrich), under conditions aimed at enhanced introduction efficiency, was shown in Fig. 22. In the chart, "Fab" corresponds to an analytical result of the reaction solution without addition of the SH-PEG-NH₂ linker (2K). A peak at around a retention time of 19.8 minutes assignable to the unmodified Fab almost disappeared, meanwhile a new peak shifted towards the higher molecular weight region up to around a retention time of 18.9 minutes was confirmed. Reaction efficiency was estimated to be 93%, from the peak area.

### (2) Fluorescence Labeling of SH-PEG-Fab with Alexa 488-Maleimide

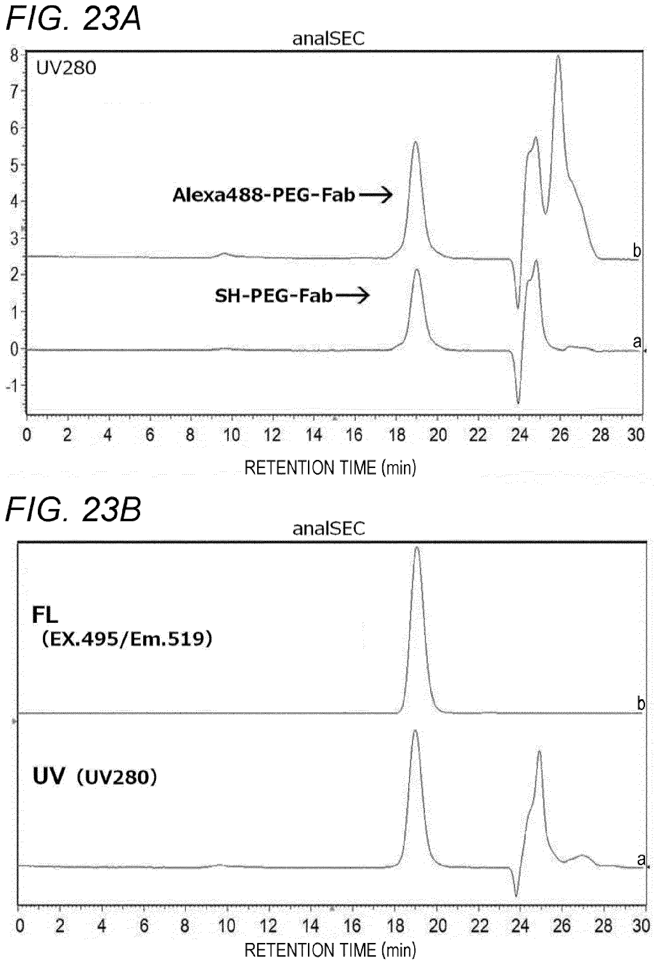

Results of SEC analysis of a reaction solution that contains SH-PEG(2K)-Fab derived from csF001-25Fab-Qtag, and Alexa 488-maleimide were shown in Figs. 23A and 23B. In Fig. 23A, the upper chromatogram corresponds to the analytical result of the reaction solution with addition of 20 equivalents of Alexa 488-maleimide, and the lower chromatogram corresponds to the analytical result of the reaction solution without addition of Alexa 488- maleimide. As seen in Fig. 23A, no difference was observed in the retention time, indicating absence, for example, of aggregation due to the labeling with the fluorophore. The lower chromatogram that corresponds to the reaction solution without addition of Alexa 488-maleimide also did not demonstrate a peak assignable to dimerization of the SH-PEG chain linkers, indicating absence of the dimerization. SEC analysis, based on UV and fluorescence absorption, of the desalted and purified fraction of the reaction solution with addition of Alexa 488-maleimide demonstrated a fluorescence absorption assignable to Alexa 488, as shown in Fig. 23B. Hence, the results indicated production of Alexa 488-PEG-Fab, based on 1:1 binding of SH-PEG-Fab and Alexa 488-maleimide.

### 14. LC-MS Analysis of SH-PEG Fab Derived from csF001-25Fab-Qtag, and Alexa488-PEG-Fab (Test Example 5)

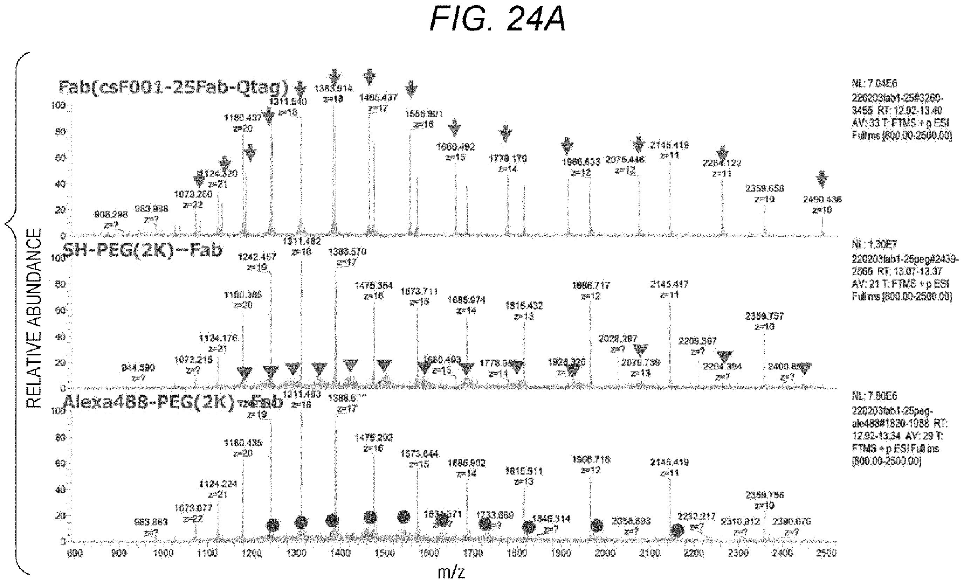

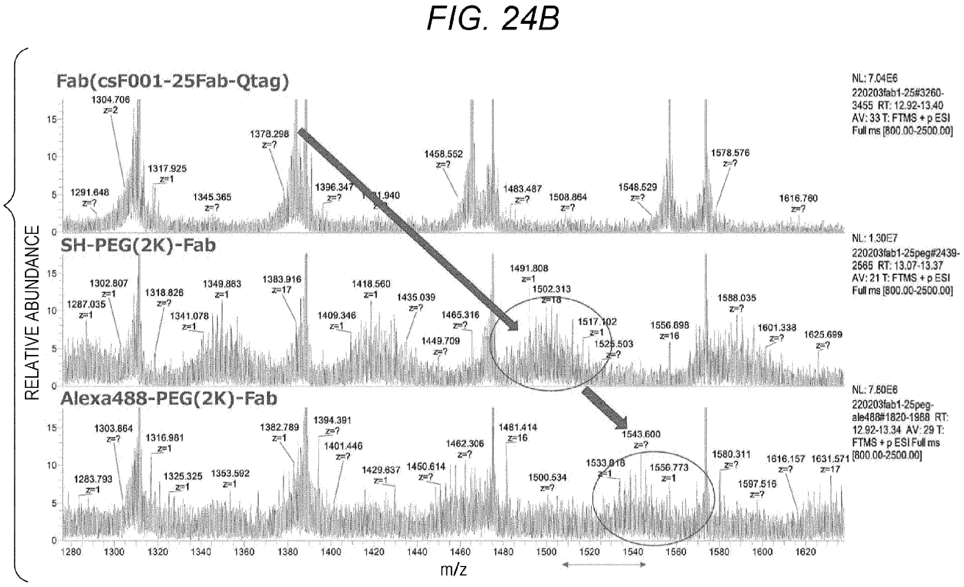

LC-MS spectra of reduced samples of csF001-25Fab-Qtag, SH-PEG-Fab thereof, and Alexa 488-PEG-Fab were shown in Figs. 24A and 24B. Fig. 24B is an enlarged chart of the Fig. 24A. Spectral pattern of the L-chain of SH-PEG-Fab remained unchanged from that of csF001-25Fab-Qtag, even after binding of the SH-PEG-NH₂ linker (2K). On the other hand, multivalent ions of the H chain of SH-PEG-Fab (see the points marked with i) were observed as broad signals (see the points marked with ▼), reflecting the molecular weight distribution of the PEG chain. The results indicate that one SH-PEG-NH₂ linker (2K) molecule was bound to the H chain of csF001-25Fab-Qtag, as a result of the BTGase-catalyzed reaction. Since the endogenous Gln residues in antibody, exclusive of Gln295 after removal of the glycan in the Fc region, has been known not to serve as a substrate for BTGase as described previously, the results suggested that the one (a) SH-PEG-NH₂ linker (2K) (Sigma-Aldrich) molecule was bound to the Gln residue in the Q-tag of HBs628Fab-Qtag. Upon binding of Alexa 488-maleimide, the spectra assignable to the multivalent ions of the H chain, broadened due to PEG chain, almost disappeared, instead a shift that reflects the binding of one Alexa 488 molecule was observed (see the points marked with ●). The results indicated that Alexa 488-PEG-Fab is a Fab having one Alexa 488 molecule bound thereto, while interposed by the SH-PEG linker.

### 15. BTGase-Catalyzed Modification of Anti-CD20 Fab-Qtag with SH-PEG-NH₂ Linker (2K) (Test Example 1)

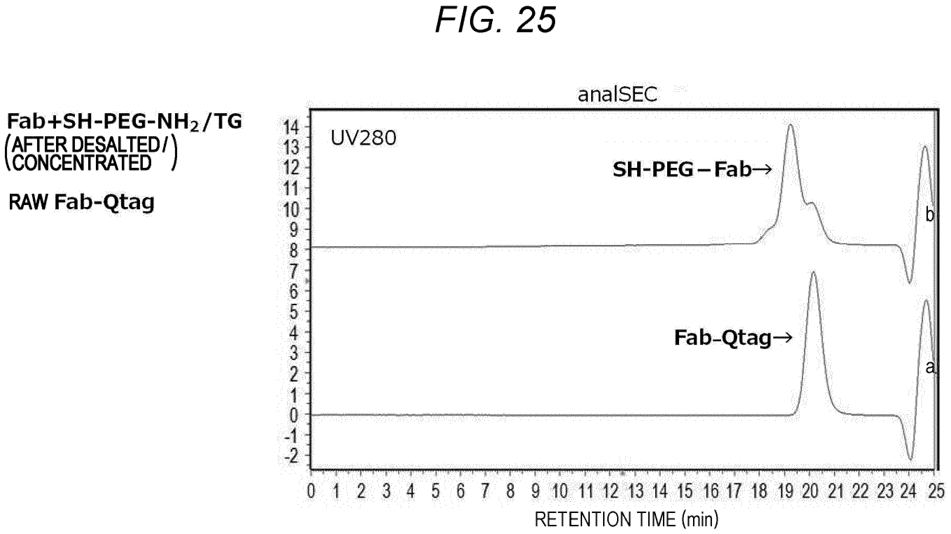

An SEC analytical result of a reaction solution, desalted and concentrated after the BTGase-catalyzed reaction between anti-CD20 Fab-Qtag and the aforementioned (a') SH-PEG-NH₂ linker (2K) (Sigma-Aldrich), under conditions aimed at enhanced introduction efficiency, was shown in Fig. 25. In the chart, "Fab-Qtag" relates to an analytical result of the reaction solution without addition of the SH-PEG-NH₂ linker (2K). A peak at around a retention time of 20.2 minutes assignable to the unmodified Fab almost disappeared, meanwhile a new peak shifted towards the higher molecular weight region up to around a retention time of 19.0 minutes was confirmed. Reaction efficiency was estimated to be 77%, from the peak area. The reason why the reaction efficiency was lower than that of other Fab-Q-tags, despite under the conditions aimed at enhanced introduction efficiency, is presumably because the anti-CD20 Q-tag had no proline residue at the C-terminal and was thus susceptible to peptidase, so that the raw anti-CD20 Fab-Qtag would contain a fragment thereof.

### 16. R-PE Labeling of SH-PEG-Fab (Test Example 6)

### (1) Maleimide Modification of R-PE

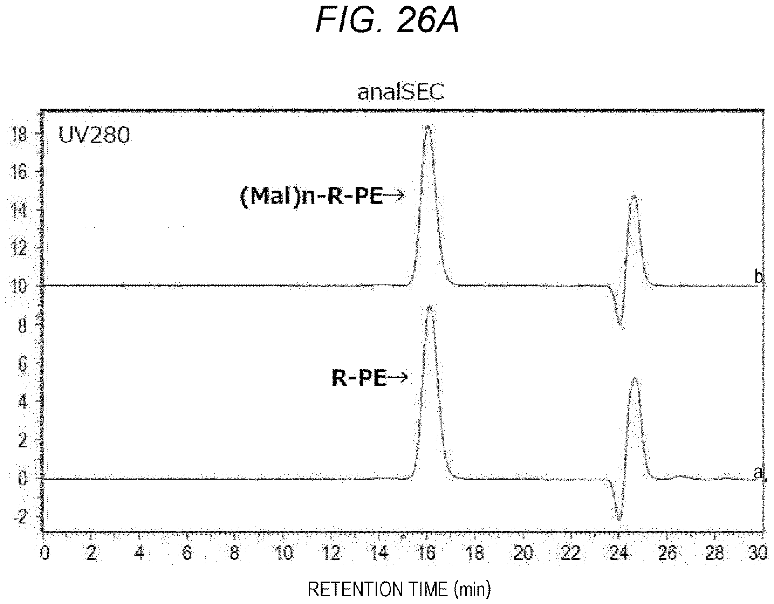

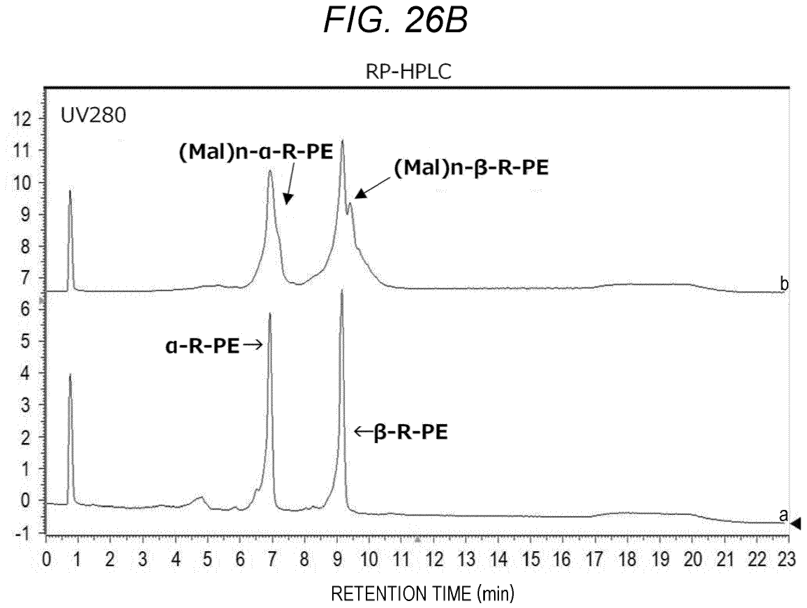

Analytical results of a reaction solution that contains R-PE and EMCS reagent, analyzed by SEC and reverse-phase HPLC were shown in Fig. 26A and 26B. As seen in Fig. 26A, there was no large difference in the retention time between the peaks assignable to maleimide-modified R-PE obtained by the reaction with the EMCS reagent, and to unmodified R-PE. This suggested maintenance of the R-PE structure. On the other hand, analytical results of reverse-phase HPLC demonstrated, as shown in Fig. 26B, peaks presumably assignable to α subunit and β subunit, which are main constituents of R-PE, were broadened towards the later region of the retention time, as a result of EMCS modification. It was thus concluded that a maleimide modified product ((Mal)ₙ-R-PE), having multiple highly hydrophobic EMCS reagents bound thereto, was prepared. Note that the peak assignment herein is merely provisional, although the early peak was tentatively assigned to the β subunit having larger molecular weight, and the later peak to the α subunit having a smaller molecular weight.

### (2) Coupling Reaction of SH-PEG-Fab, derived from csF001-25Fab-Qtag, and Maleimide-Modified R-PE

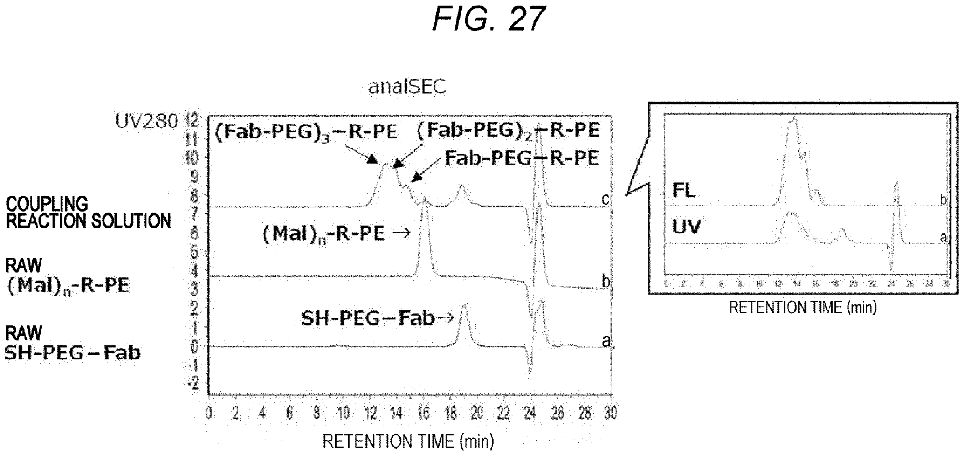

Analytical results of SH-PEG(2K)-Fab derived from sF001-25Fab-Qtag, (Mal)ₙ-R-PE, and a coupling reaction solution of them, analyzed by SEC were shown in Fig. 27. Despite only a small mixing ratio of Fab and R-PE such as 3: 1, the peak assignable to SH-PEG(2K)-Fab (at a retention time around 19.2 minutes) decreased, and only approximately 7% of the peak assignable to (Mal)ₙ-R-PE (at a retention time of around 16.0 minutes) remained. This means high reaction efficiency. From the retention time assuming the PEG effect (increase in apparent molecular weight), not only a peak assignable to Fab-PEG-R-PE (at a retention time of around 14.8 minutes), but also a peak assignable to (Fab-PEG)₂-R-PE (at a retention time of around 13.4 minutes), and a peak assignable to (Fab-PEG)₃-R-PE (at a retention time of around 12.8 minutes) were confirmed.

### (3) SEC Fractionation of Coupling Reaction Peaks, and SEC Analysis of Preparative Fraction

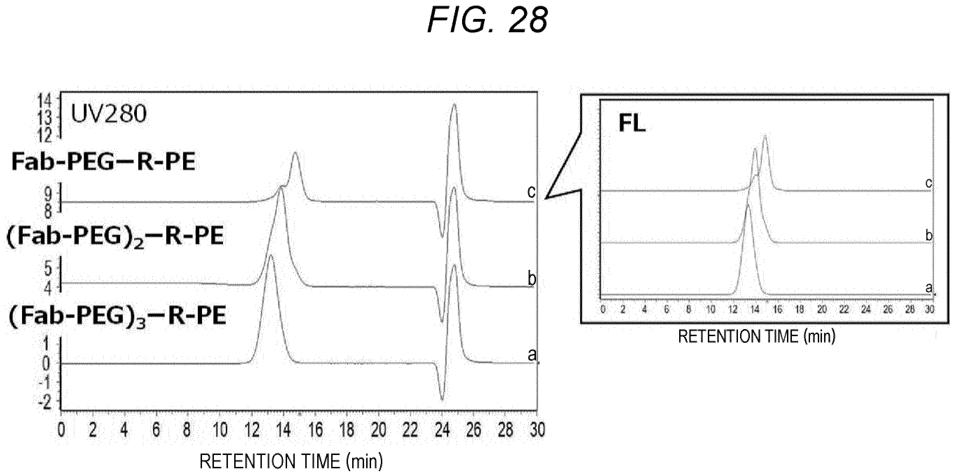

A coupling reaction solution that contains SH-PEG(2K)-Fab derived from sF001-25Fab-Qtag, and the maleimide-modified R-PE was fractioned by SEC, and the preparative fractions were analyzed by SEC. Results were shown in Fig. 28. The individual peaks in Fig. 28 were concluded to be (Fab-PEG)₃-R-PE, (Fab-PEG)₂-R-PE, and Fab-PEG-R-PE, in the sequential order from earlier to later retention time. The fluorescence intensity per UV peak area tended to decrease as the number of binding of Fab-PEG increased, reflecting the estimated conjugate structures. (Fab-PEG)₃-R-PE and (Fab-PEG)₂-R-PE demonstrated high purity, meanwhile Fab-PEG-R-PE demonstrated slightly lower purity. All fractions were, however, confirmed to contain the main products in the main peaks. The peaks assignable to the product were found to demonstrate fluorescence absorption ascribed to PE, in association with the reaction with R-PE.

### (4) Coupling Reaction of SH-PEG-Fab Derived from Anti-CD20 Fab-Qtag, with Maleimide-Modified R-PE

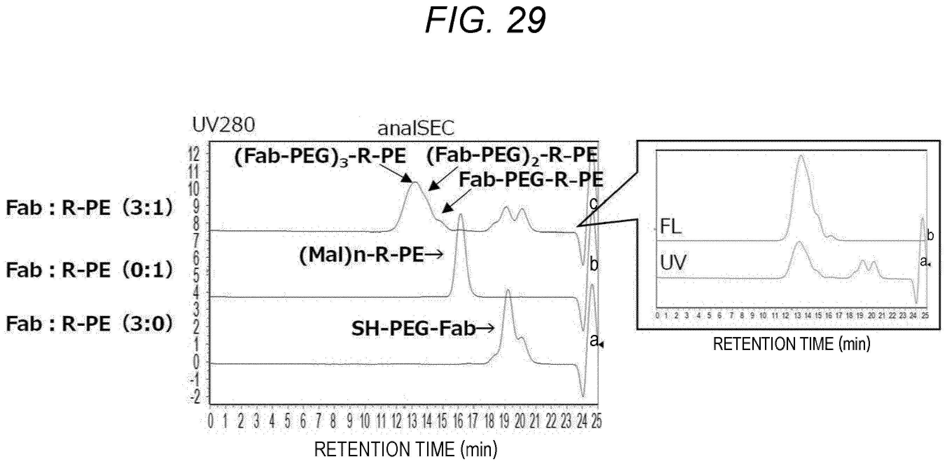

Analytical results of SH-PEG(2K)-Fab derived from anti-CD20 Fab-Qtag, (Mal)ₙ-R-PE, and a coupling reaction solution of them, analyzed by SEC were shown in Fig. 29. Despite only a small mixing ratio of Fab and R-PE such as 3: 1, the peak assignable to SH-PEG(2K)-Fab (at a retention time around 19.2 minutes) decreased, and also the peak assignable to (Mal)ₙ-R-PE (at a retention time of around 15.8 minutes) almost did not remain. This means high reaction efficiency. From the retention time assuming the PEG effect (increase in apparent molecular weight), not only a peak assignable to Fab-PEG-R-PE (at a retention time of around 14.8 minutes), but also a peak assignable to (Fab-PEG)₂-R-PE (at a retention time of around 13.4 minutes), and a peak assignable to (Fab-PEG)₃-R-PE (at a retention time of around 12.8 minutes) were confirmed. The peaks assignable to the product were found to demonstrate fluorescence absorption ascribed to PE, in association with the reaction with R-PE.

### (5) SEC Fractionation of Coupling Reaction Peaks, and SEC Analysis of Preparative Fraction

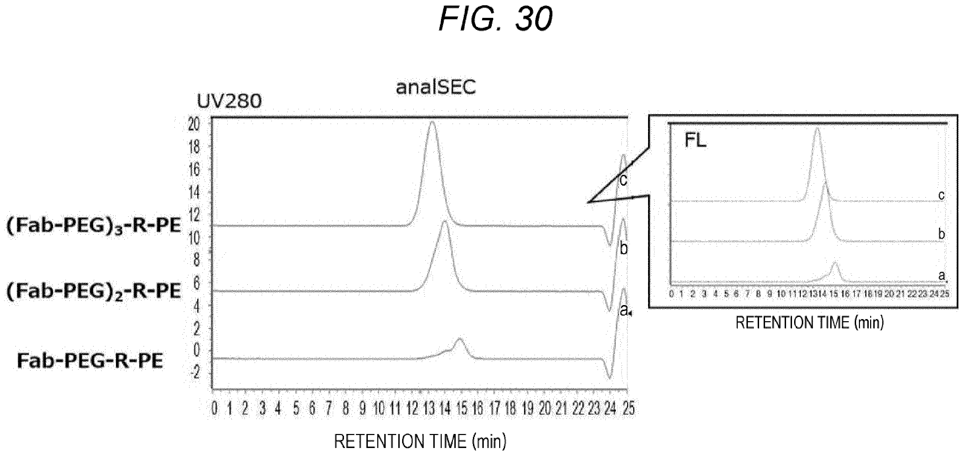

A reaction solution of coupling of SH-PEG(2K)-Fab derived from anti-CD20 Fab-Qtag with the maleimide-modified R-PE was fractioned by SEC, and the preparative fractions were analyzed by SEC. Results were shown in Fig. 30. The individual peaks in Fig. 30 were concluded to be (Fab-PEG)₃-R-PE, (Fab-PEG)₂-R-PE, and Fab-PEG-R-PE, in the sequential order from earlier to later retention time. The fluorescence intensity per UV peak area tended to decrease as the number of bindings of Fab-PEG increased, reflecting the estimated conjugate structures. (Fab-PEG)₃-R-PE and (Fab-PEG)₂-R-PE demonstrated high purity, meanwhile Fab-PEG-R-PE demonstrated slightly lower purity. All fractions were, however, confirmed to contain the main products in the main peaks. The peaks assignable to the product were found to demonstrate fluorescence absorption ascribed to PE, in association with the reaction with R-PE.

### 17. Evaluation of Antigen Binding Capacity of Alexa 488-Labeled Antibody (Test Example 9)

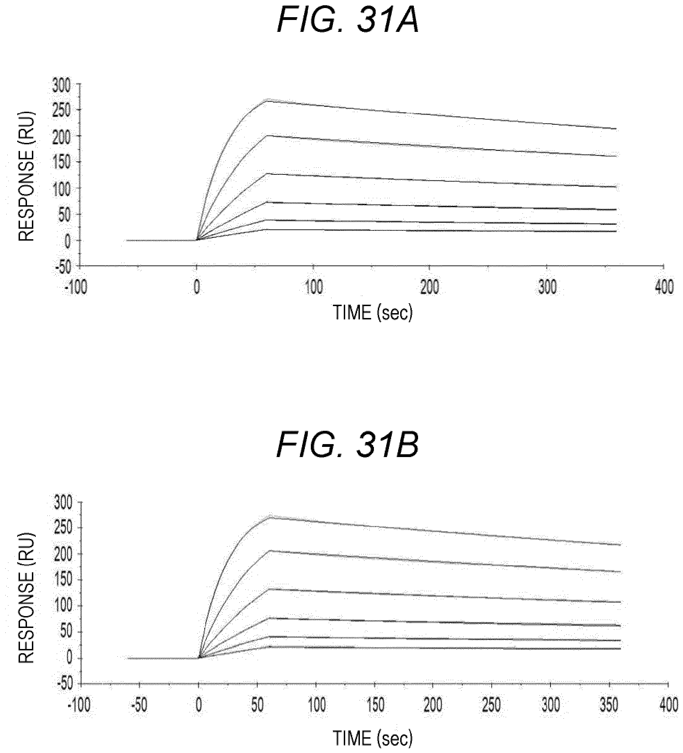

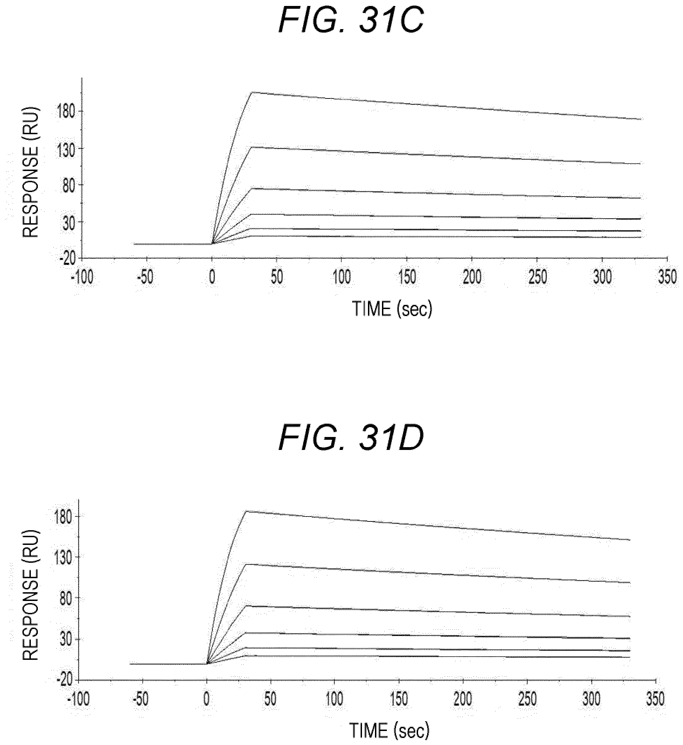

Interaction with antigen of csF001-25Fab-Qtag, SH-PEG(2K)-Fab thereof, and Alexa 488-PEG-Fab was measured with use of Biacore (registered trademark) T200 (Cytiva). Results were summarized and shown in Table 4 and Figs. 31A to 31D. Results for SH-PEG(2K)-Fab and Alexa 488-PEG-Fab were presented separately, because of the dates of measurement were different. As summarized in Table 4, both of SH-PEG(2K)-Fab and Alexa 488-PEG-Fab did not demonstrate difference of the KD value from that of the unmodified Fab (csF001-25Fab-Qtag). Also as seen in Figs. 31A to 31D, there was no large difference among patterns of the sensorgrams. Hence the labeling method had no effect on the antigen binding capacity.

**[Table 4]**

| Sample | KD (M) |
|---|---|
| Fab | 1.5 × 10⁻⁹ |
| SH-PEG(2K)-Fab | 1.5 × 10⁻⁹ |
| Fab | 1.3 × 10⁻⁹ |
| Alexa488-PEG-Fab | 1.3 × 10⁻⁹ |

### 18. Antigen-Binding Capacity of R-PE-Labeled Antibody (Test Example 10)

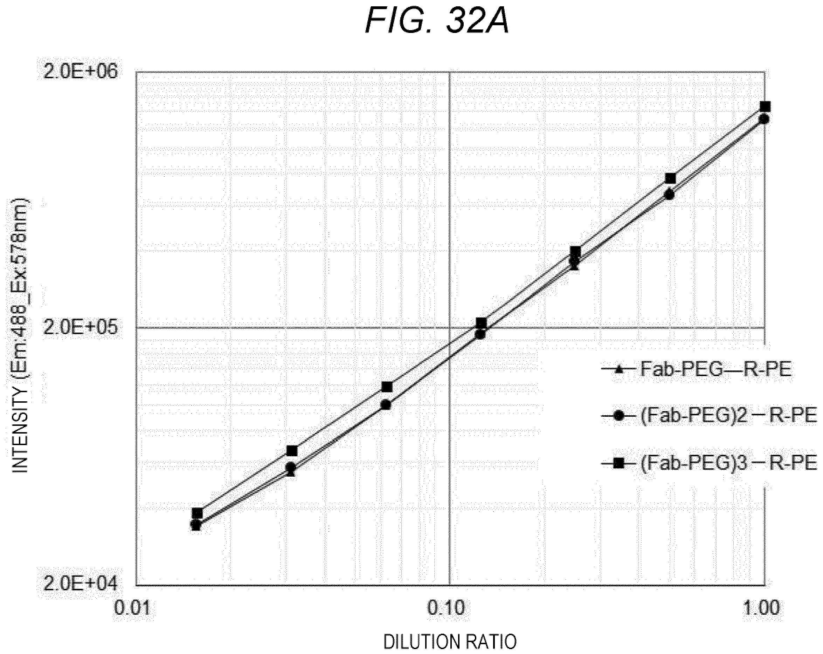

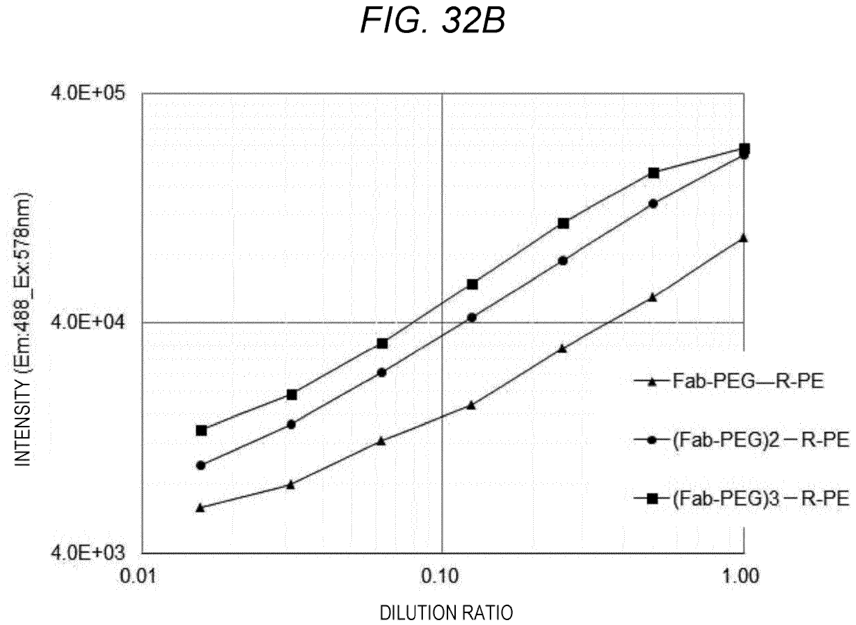

Antigen binding capacity of Fab-PEG-R-PE, (Fab-PEG)₂-R-PE, and (Fab-PEG)₃-R-PE derived from csF001-25Fab-Qtag was measured by ELISA. Results were shown in Figs. 32A and 32B. Fig. 32A illustrates the results in the case without addition of the antigen, meanwhile Fig. 32B illustrates results in the case with the antigen. Referring to Fig. 32A, fluorescence signal values at the individual dilution ratios were compared, while equalizing the fluorescence intensity of the detection reagent (that is, equalizing the mole concentration of the R-PE labeled antibody). No significant difference was observed among the detected fluorescence signal values, at any dilution rate. On the other hand, as seen in Fig. 32B, the case with addition of the antigen demonstrated difference of the detected fluorescence signal values among the R-PE-labeled antibodies. More specifically, when compared at the same dilution ratio, Fab-PEG-R-PE demonstrated a lowest fluorescence signal value, (Fab-PEG)₂-R-PE was the second highest, and (Fab-PEG)₃-R-PE was the highest. That is, the larger the number of bindings of Fab-PEG molecule, the higher the fluorescent signal values. This is presumably because the R-PE molecule can demonstrate avidity effect in antigen binding, upon binding thereon multiple Fab-PEG molecules, and enhanced the antigen capturing ability per R-PE molecule. In fact, (Fab-PEG)₂-R-PE and (Fab-PEG)₃-R-PE, after approximately 3-fold dilution and 5-fold dilution respectively from the concentration of Fab-PEG-R-PE, demonstrated fluorescence signal values comparable to that of Fab-PEG-R-PE. The results indicated that the R-PE-labeled antibody, having multiple Fab-PEG molecules bound to R-PE, can demonstrate an effect of enhancing fluorescence signal value of the antibody-R-PE conjugate on the molecule basis.

### 19. Performance Evaluation of Reagent That Contains R-PE-Labeled Antibody (Test Example 11)

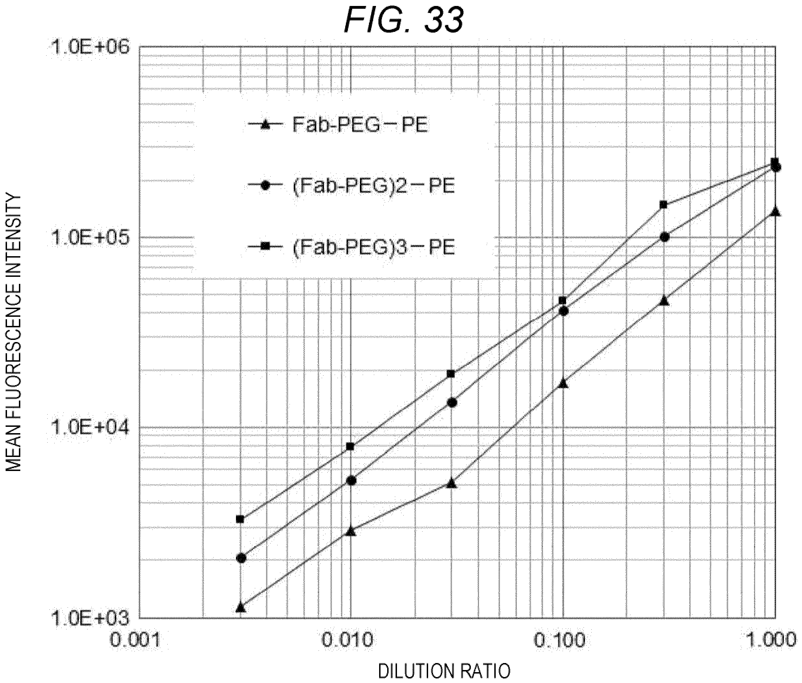

CD20-expressing cell (Ramos cell) was measured by FCM, with use of Fab-PEG-R-PE, (Fab-PEG)₂-R-PE, and (Fab-PEG)₃-R-PE derived from anti-CD20 Fab-Qtag, individually as the detection antibody. Results were shown in Fig. 33. Fluorescence signal values at the individual dilution ratios were compared, while equalizing the fluorescence intensity of the detection reagent (that is, equalizing the mole concentration of the R-PE labeled antibody). As seen in Fig. 33, differences of the detected fluorescence signal values were observed among the R-PE-labeled antibodies. More specifically, when compared at the same dilution ratio, Fab-PEG-R-PE demonstrated a lowest fluorescence signal value, (Fab-PEG)₂-R-PE was the second highest, and (Fab-PEG)₃-R-PE was the highest. That is, the larger the number of bindings of Fab-PEG molecule, the higher the fluorescent signal values. This is presumably because the R-PE molecule, having multiple Fab-PEG molecules bound thereon, can demonstrate avidity effect in binding to CD20 on cells, and enhanced the antigen capturing ability per R-PE molecule. In fact, (Fab-PEG)₂-R-PE and (Fab-PEG)₃-R-PE, after approximately 3-fold dilution and 4-fold dilution respectively from the concentration of Fab-PEG-R-PE, demonstrated fluorescence signal values comparable to that of Fab-PEG-R-PE. The results indicated that the R-PE-labeled antibody, having multiple Fab-PEG molecules bound to R-PE, can enhance the performance of the detection reagent used for FCM, owing to the avidity effect.

### 20. Results of Infusion MS of SH-PEG-NH₂ Linker (3.5K) and SH-PEG-NH₂ Linker (5K)

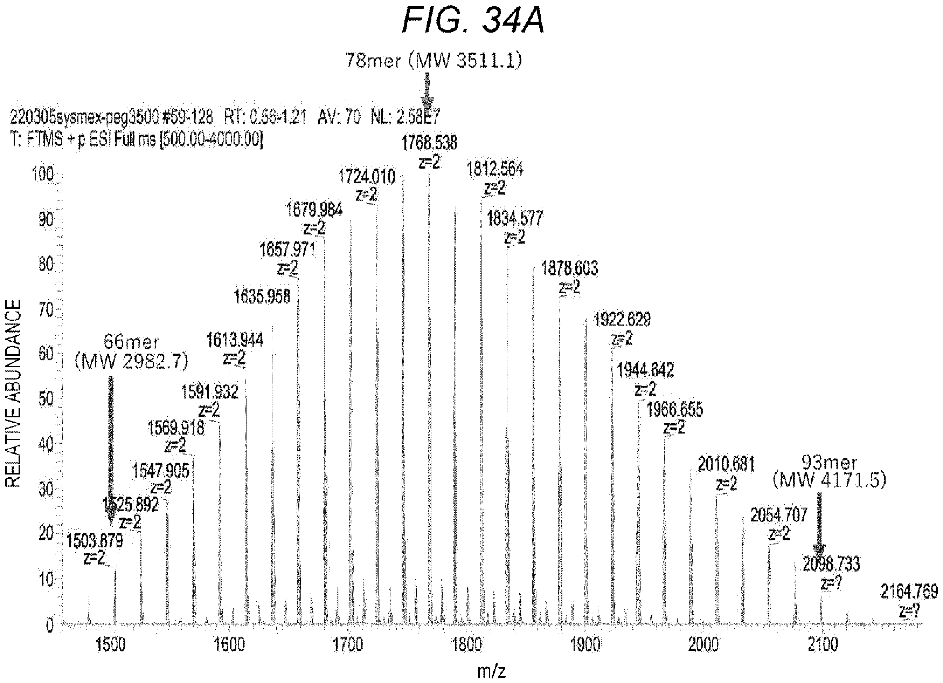

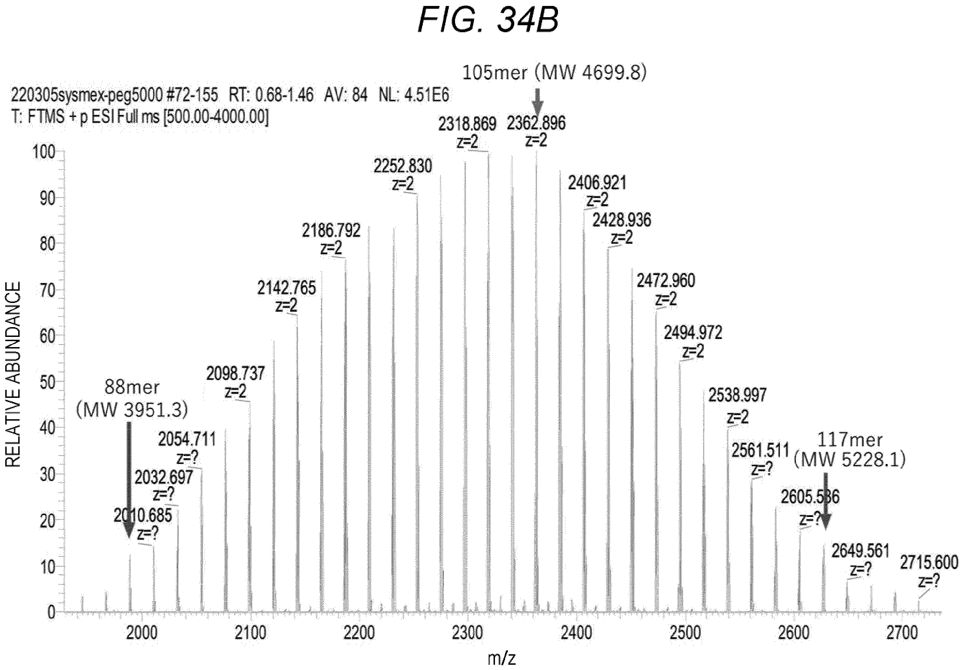

The aforementioned (d) SH-PEG-NH₂ linker and (e) SH-PEG-NH₂ linker (Sigma-Aldrich) were analyzed by Infusion MS, and results were shown in Figs. 34A and 34B, respectively. In the charts, the arrows indicate a peak corresponded to the minimum molecular weight, a peak corresponded to the mode molecular weight, and a peak corresponded to the maximum molecular weight. The mode molecular weights of the (d) and (e) linkers were 3511.1 and 4699.8, respectively. Also weight average molecular weight of the PEG chain was estimated from the analytical results. As seen in Figs. 34A and 34B, the weight average molecular weight and molecular weight distribution of each linker were found to have a profile close to the average molecular weight disclosed by the manufacturer. This will be detailed below.

· (d) Linker: weight average molecular weight 3544.2, molecular weight distribution 2982.7 to 4171.5
. (e) Linker: weight average molecular weight 4586.6, molecular weight distribution 3951.3 to 5228.1

Each SH-PEG-NH₂ linker, after addition of the BTGase reaction solution (no NaCl added, pH 8.2), was confirmed to cause almost no dimerization by a disulfide (S-S) bond, while only demonstrating an MS spectrum of the monomer correlated to the molecular weight distribution of the PEG chain. Hence, the results demonstrated that none of the (d) SH-PEG-NH₂ linker and (e) SH-PEG-NH₂ linker caused dimerization.

### 21. Fluorescence Labeling of SH-PEG-Fab with Alexa 488-Maleimide (Test Examples 12 and 13)

### (1) BTGase-Catalyzed Modification of csF001-5Fab-Qtag with SH-PEG-NH₂ Linker (3.5K/5K)

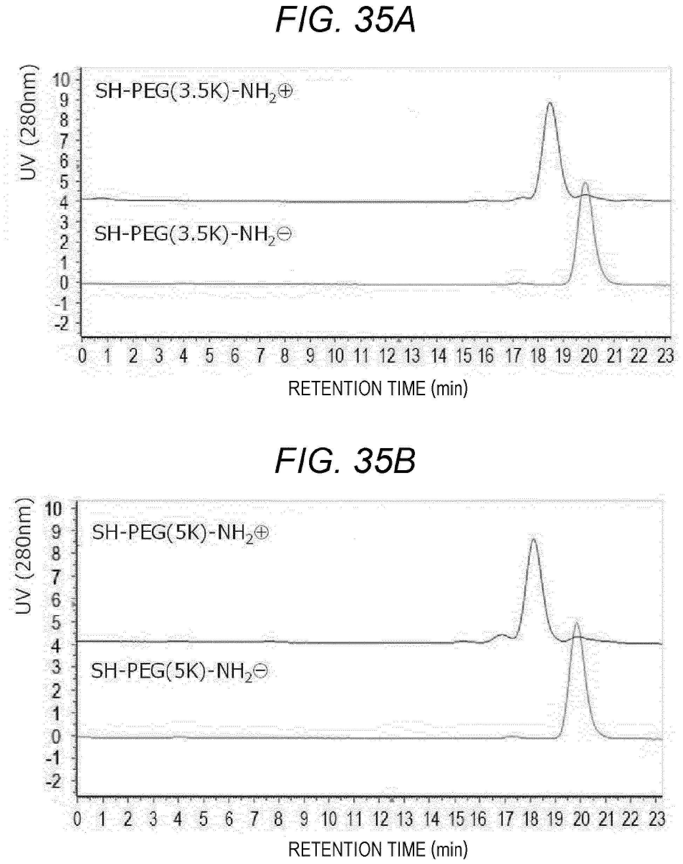

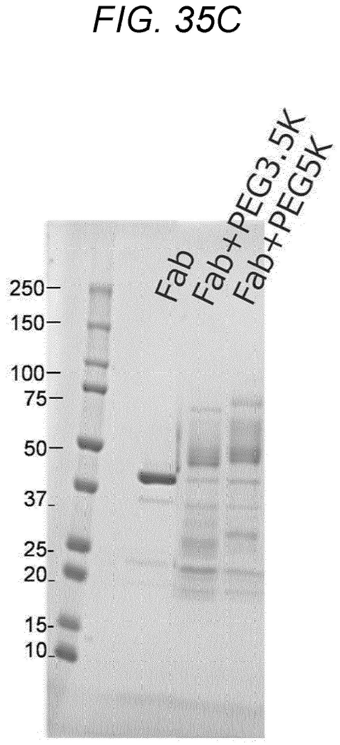

Analytical results of SEC and non-reducing SDS-PAGE of reaction solutions, desalted and concentrated after the BTGase-catalyzed reaction of csF001-25Fab-Qtag, with each of the aforementioned (d) SH-PEG-NH₂ linker (3.5K) (Sigma-Aldrich) and the aforementioned (e) SH-PEG-NH₂ linker (5K) (Sigma-Aldrich), were shown in Figs. 35A to 35C. Referring to Fig. 35A, a peak at around a retention time of 19.8 minutes assignable to the unmodified Fab almost disappeared, meanwhile new peaks individually shifted towards the higher molecular weight region up to around a retention time of 18.4 minutes (3.5K) and around 17.9 minutes (5K) were confirmed. These retention times shifted to the higher molecular side, from that of SH-PEG(2K)-Fab. Reaction efficiencies were estimated to be 98% (3.5K) and 92% (5K), from the peak area.

### (2) Fluorescence Labeling of SH-PEG-Fab with Alexa 488-Maleimide

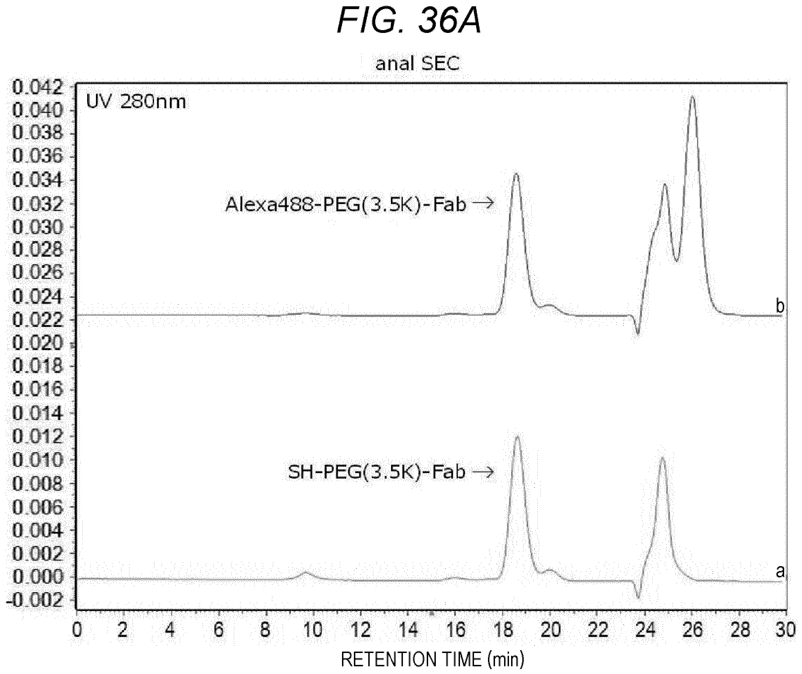

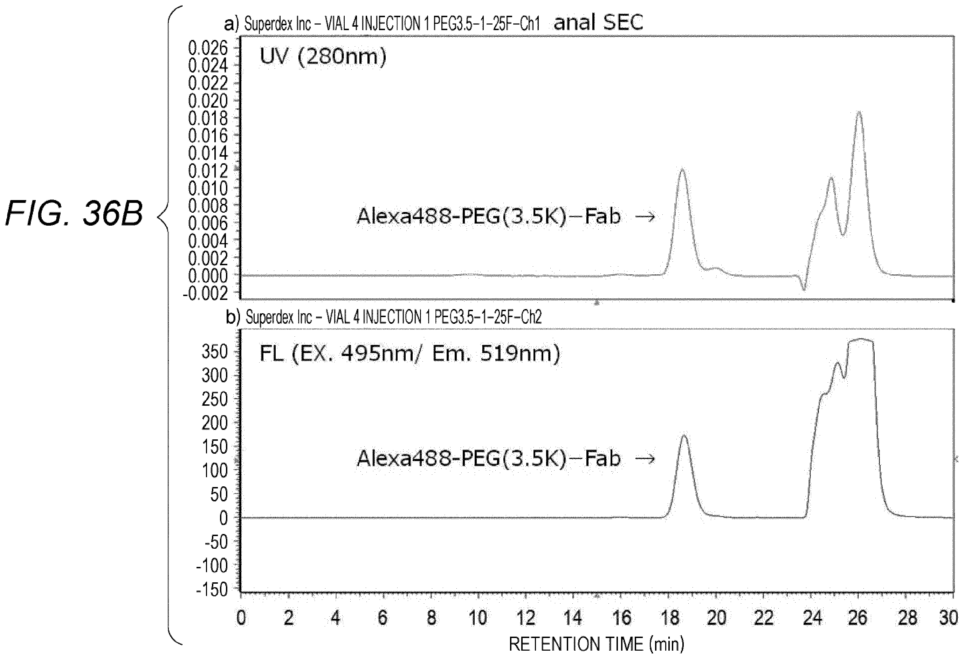

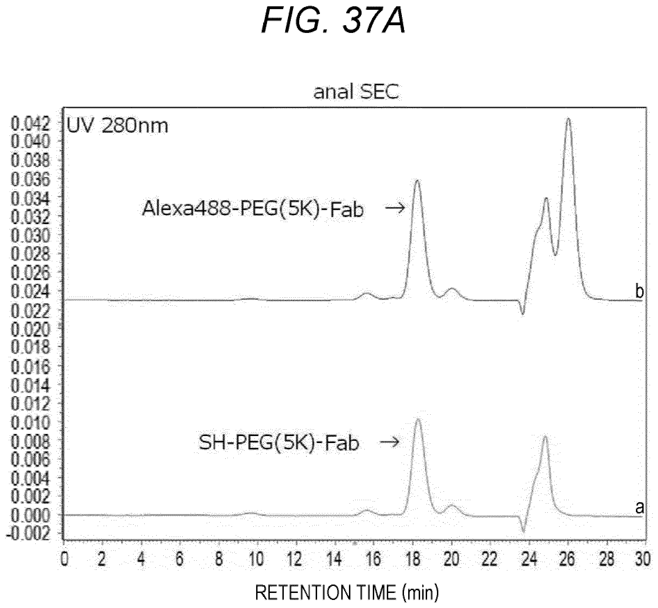

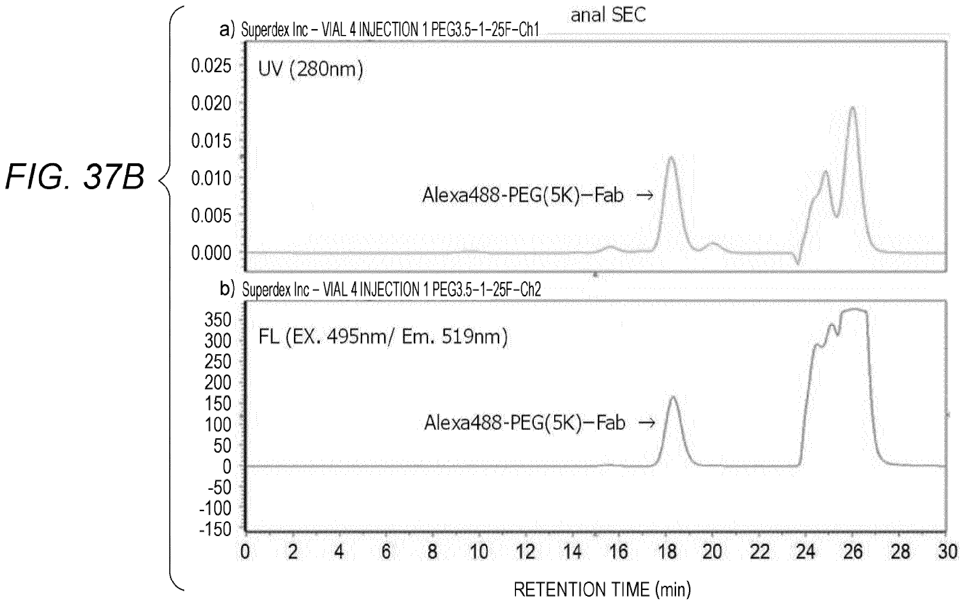

Results of SEC analysis of a reaction solution that contains SH-PEG(3.5K)-Fab derived from csF001-25Fab-Qtag, and Alexa 488-maleimide were shown in Figs. 36A and 36B. Results of SEC analysis of a reaction solution that contains SH-PEG(5K)-Fab derived from csF001-25Fab-Qtag and Alexa 488-maleimide were shown in Figs. 37A and 37B. In each of Figs. 36A and 37A, the upper chromatogram represents an analytical result of the reaction solution with addition of 20 equivalents of Alexa 488 maleimide, meanwhile the lower chromatogram represents an analytical result of the reaction solution without addition of Alexa 488.

As seen in Fig. 36A, SEC analysis of the reaction solution revealed a slight difference in retention time in association with binding of Alexa 488 (SH-PEG-Fab: around 18.6 minutes, Alexa 488-PEG-Fab: around 18.4 minutes), indicating absence of aggregation or the like, due to the labeling with the fluorophore. As seen in 36B, SEC analysis of the reaction solution revealed fluorescence absorption by the product at a retention time of around 18.4 minutes, which is attributable to Alexa 488. Hence, the results indicated production of Alexa 488-PEG-Fab, based on 1:1 binding of SH-PEG(3.5K)-Fab and Alexa 488-maleimide.

As seen in Fig. 37A, SEC analysis of the reaction solution revealed a slight difference in retention time in association with binding of Alexa 488 (SH-PEG-Fab: around 18.2 minutes, Alexa 488-PEG-Fab: around 17.9 minutes), indicating absence of aggregation or the like, due to the labeling with the fluorophore. As seen in Fig. 37B, SEC analysis of the reaction solution revealed fluorescence absorption by the product at a retention time of around 18.0 minutes, which is attributable to Alexa 488. Hence, the results indicated production of Alexa 488-PEG-Fab, based on 1:1 binding of SH-PEG(5K)-Fab and Alexa 488-maleimide.

### 22. Modification of Fab Antibody with SH-PEG-NH₂ Linker (400Da) (Test Example 14)

### (1) BTGase-Catalyzed Modification of csF001-5Fab-Qtag with SH-PEG-NH₂ Linker (400Da)

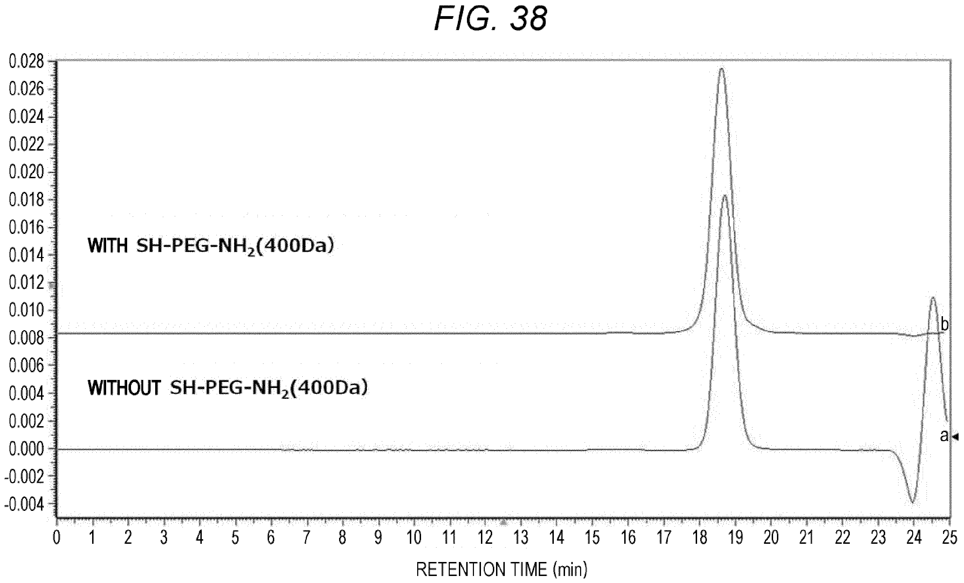

Analytical results of BTGase-catalyzed reaction solution that contains csF001-5Fab-Qtag and the aforementioned (f) SH-PEG-NH₂ linker (400Da)(Nanocs Inc.), analyzed (fractionated) by SEC, were shown in Fig. 38. As seen in Fig. 38, the peak top shifted slightly towards the earlier side on the scale of retention time, upon addition of SH-PEG-NH₂ linker (400Da). The Fab having one linker molecule bound thereto was also suspected to have the apparent size, similar to that of the unmodified Fab (csF001-5Fab-Qtag) on the chromatogram. The preparative fraction was then used for a coupling reaction with maleimide-modified ALP, as described next.

### (2) ALP Labeling of SH-PEG (400 Da)-Fab

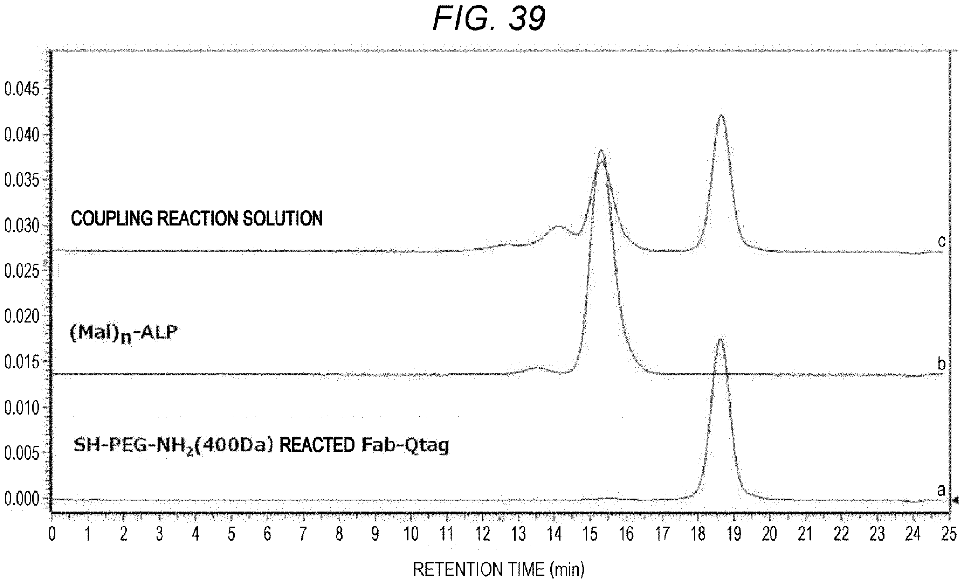

Analytical results of SH-PEG(400Da)-Fab derived from csF001-5Fab-Qtag, maleimide-modified ALP ((Mal)ₙ-ALP), and a coupling reaction solution of them, analyzed by SEC were shown in Fig. 39. As seen in Fig. 39, SEC analysis of the reaction solution revealed a new broad peak at a retention time of around 13.8 minutes, with the peak areas of the raw materials, attributable to the Fab reaction product and (Mal)ₙ-ALP, remained almost unreduced. From the results, the coupling reaction between SH-PEG (400Da)-Fab and (Mal)ₙ-ALP was confirmed to hardly occur, only with a slight yield of a coupled product if possible. Hence, the SH-PEG-NH₂ linker (400 Da) was concluded to be not suitable for the production method for a labeled polypeptide according to the invention.

### 23. Modification of Fab Antibody with SH-PEG-NH₂ Linker (1K) (Test Example 15)

### (1) BTGase-Catalyzed Modification of HBs628Fab-Qtag with SH-PEG-NH₂ Linker (1K)

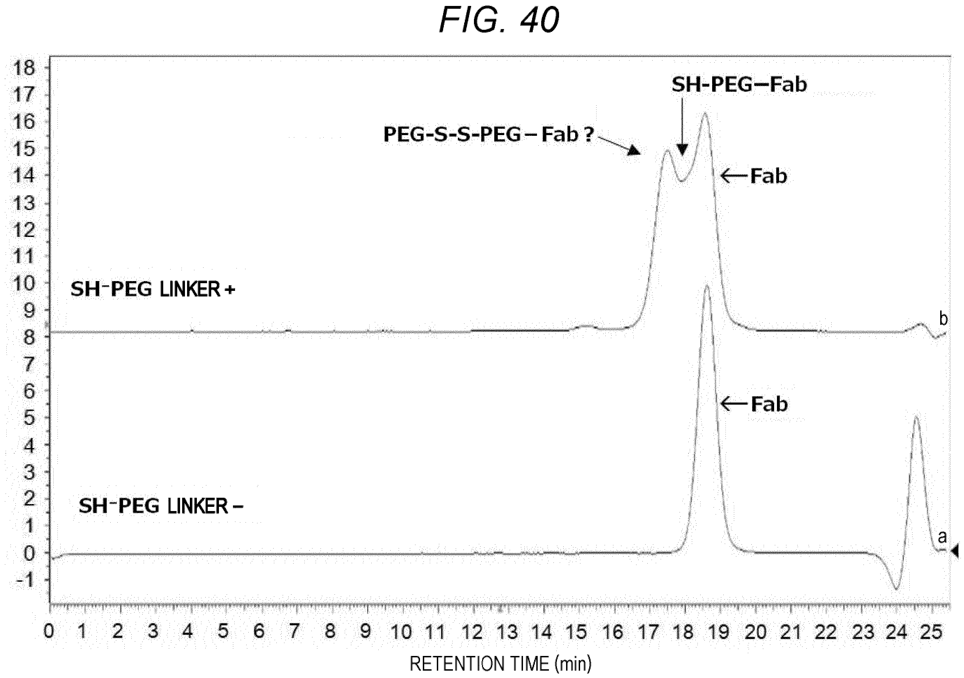

SEC analytical results of BTGase-catalyzed reaction solution that contains HBs628Fab-Qtag and the aforementioned (g) SH-PEG-NH₂ linker (1K) (Creative PEGWorks) were shown in Fig. 40. As seen in Fig. 40, the case without addition of the linker demonstrated no change in the Fab peak. This indicates that there was no intermolecular dimerization of Fabs occurred between the Gln residue of the Q-tag and the Lys residue of the Fab. On the other hand, in the case with addition of the linker, the peak assignable to the unmodified Fab decreased at around a retention time of 18.7 minutes, meanwhile a new peak appeared in the higher molecular weight region, although with a poor separability. Since the chain length of the SH-PEG-NH₂ linker (1K) is half the chain length of the SH-PEG-NH₂ linker (2K), so that the coupled product was not considered to largely increase the apparent size, upon binding with Fab-QTag. Hence, the peak at a retention time of around 17.7 minutes was presumably assignable to SH-PEG(1K)-Fab. An additional peak was observed at around a retention time of 16.9 minutes, which was attributable to a substance having a larger molecular weight. The point of the peak was found on the higher molecular weight side, in the SEC chart, of the peak presumably attributable to SH-PEG(1K)-Fab. The fraction thus appeared on the higher molecular weight side was presumably attributable to PEG-S-S-PEG-Fab, generated by dimerization of the SH-PEG linkers via a S-S bond formed between the SH groups. Dickgiesser S., et al. (see Bioconjugate Chemistry, 31, 1070-1076 (2020)) has made a trial of selectively introducing, by a BTGase-catalyzed reaction, an SH group-containing small molecule linker cysteamine (SH-CH₂-CH₂-NH₂) onto a Gln residue in the antibody which is a substrate for such TG, and has actually reported that the TG-catalyzed cysteamine-modified antibody had introduced therein dimerized cysteamine formed between the SH groups. The authors have therefore used, after the reaction, a reducing agent TCEP for complete reduction, which was followed by re-oxidation for reconstructing the S-S bond. Also judging from such finding, it was considered that the SH-PEG-NH₂ linker (1K), having been presumably dimerized, was undoubtfully dimerized via an S-S bond. The coupling reaction solution was desalted and then used for the subsequent coupling reaction with maleimide-modified ALP, even if the solution contains the unreacted Fab-Qtag and PEG-S-S-PEG-Fab, since these components have no free SH group, and therefore do not react with (Mal)ₙ-ALP.

### (2) ALP Labeling of SH-PEG(1K)-Fab

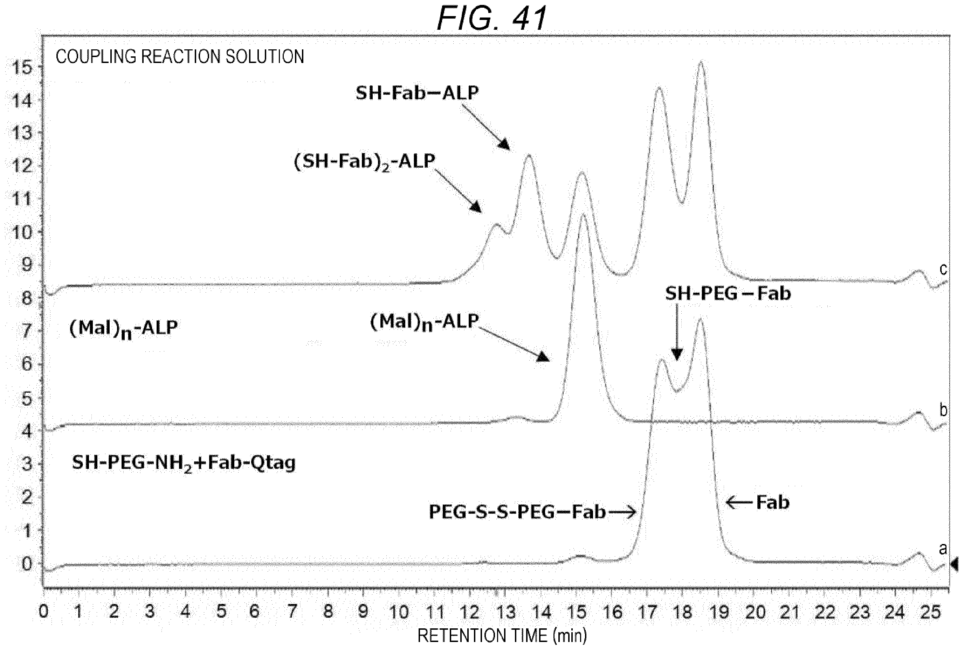

Analytical results of SH-PEG(1K)-Fab derived from HBs628Fab-Qtag, maleimide-modified ALP ((Mal)ₙ-ALP), and a coupling reaction solution of them, analyzed by SEC were shown in Fig. 41. The coupling reaction was conducted while adjusting the mixing ratio of Fab and ALP to 2 : 1. Results shown in Fig. 41 indicated that approximately 43% of (Mal)ₙ-ALP (retention time: approx. 15.2 minutes) has reacted as judged from the peak assignable to (Mal)ₙ-ALP, and from among SH-PEG(1K) reaction products, a peak component (retention time: approx. 17.8 minutes) presumably assignable to SH-PEG-Fab disappeared, meanwhile a new peak appeared, which was presumably attributable Fab-PEG-ALP having one Fab molecule bound to ALP. The product was, however, mainly found in a peak assignable to Fab-PEG-ALP (retention time: approx. 13.8 min), while yielding only a very small amount of (Fab-PEG)₂-ALP, having two Fab molecules bound to ALP (retention time: approx. 12.8 min). As described above, the SH-PEG-NH₂ linker (1K) was poor in coupling efficiency with (Mal)ₙ-ALP, since the linkers mutually dimerized when reacting with Fab-Qtag. The results indicated that the coupled product between the Fab-Qtag and (Mal)ₙ-ALP, while interposed by SH-PEG-NH₂ linker whose PEG chain has a molecular weight of approximately 1000, demonstrated only poor SEC separability between SH-PEG(1K)-Fab and unmodified Fab-Qtag. Since the coupling efficiency between proteins was low due to side reaction as a result of the linker dimerization, so that the use of the SH-PEG-NH₂ linker (1K) was judged to be unpractical.

These experimental results proved that the present invention excels in the efficiency of introducing the label into polypeptide, as compared with the case of using the SH-PEG-NH₂ linker (400Da) or the SH-PEG-NH₂ linker (1K).

## Claims

1. A labeled polypeptide comprising a side chain-containing glutamine residue, the side chain being represented by formula (I) below: 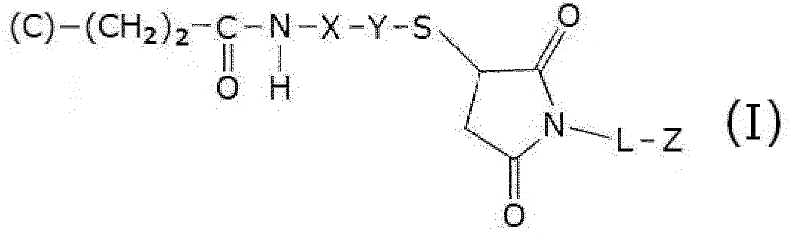 wherein, (C) represents an α-carbon of the glutamine residue, X represents a straight chain alkylene group, Y represents a polyethylene glycol chain, Z represents a label, L represents a spacer or an atomic bond, and the polyethylene glycol chain has a molecular weight of 1100 or larger.

2. The labeled polypeptide according to claim 1, wherein the labeled polypeptide is a fusion polypeptide of an antibody, with a peptide tag that comprises the side chain-containing glutamine residue.

3. A modified polypeptide comprising a side chain-containing glutamine residue, the side chain being represented by formula (II) below: 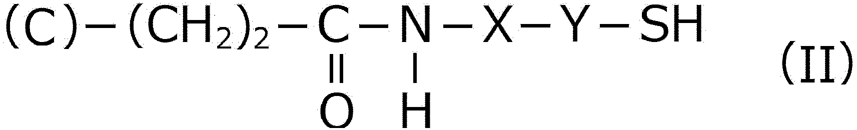 wherein, (C) represents an α-carbon of the glutamine residue, X represents a straight chain alkylene group, Y represents a polyethylene glycol chain, and the polyethylene glycol chain has a molecular weight of 1100 or larger, preferably has an average molecular weight of 1300 or larger, preferably an average molecular weight of 1700 or larger.

4. The modified polypeptide according to claim 3, wherein the modified polypeptide is a fusion polypeptide of an antibody, with a peptide tag that comprises the side chain-containing glutamine residue.

5. The polypeptide according to any one of claims 1 to 4, wherein the polyethylene glycol chain is represented by formula (III) below:
-(OCH₂CH₂)ₙ- or -(CH₂CH₂O)ₙ- (III)
(wherein, n represents an integer of 25 or larger, preferably n represents an integer of 39 or larger).

6. The polypeptide according to any one of claims 1 to 5, wherein the straight chain alkylene group has 2 or more and 10 or less carbon atoms.

7. The labeled polypeptide according to any one of claims 1 to 6, wherein the label is at least one substance selected from the group consisting of biotins, enzyme, fluorescent dye, fluorescent protein, and hapten.

8. The polypeptide according to claim 2 or 4, wherein the antibody is Fab, Fab', F(ab')₂, Fd, Fd', Fv, scFv, dAb, rIgG, light chain, heavy chain antibody, variable domain of heavy chain antibody, diabody, or triabody.

9. A reagent comprising the polypeptide according to any one of claims 1 to 8.

10. A production method for a modified polypeptide, the method comprising:
contacting, in the presence of a transglutaminase, a glutamine residue-containing polypeptide with a linker represented by formula (VI) below:
NH₂-X-Y-SH (VI)
(wherein X represents a straight chain alkylene group, Y represents a polyethylene glycol chain, the polyethylene glycol chain having a molecular weight of 1100 or larger), to bind the linker to a carboxamide side chain of the glutamine residue; and
obtaining a modified polypeptide produced by the binding of the carboxamide side chain with the linker;
and,
in the modified polypeptide,
a side chain of the glutamine residue is represented by formula (II) below: 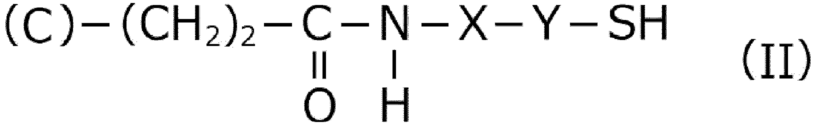 wherein, (C) represents an α-carbon atom of the glutamine residue, X represents a straight chain alkylene group, Y represents a polyethylene glycol chain, and the polyethylene glycol chain has a molecular weight of 1100 or larger.

11. A production method for a labeled polypeptide, the method comprising:
contacting, in the presence of a transglutaminase, a glutamine residue-containing polypeptide with a linker represented by formula (VI) below:
NH₂-X-Y-SH (VI)
wherein X represents a straight chain alkylene group, Y represents a polyethylene glycol chain, the polyethylene glycol chain having a molecular weight of 1100 or larger, to bind the linker to a carboxamide side chain of the glutamine residue;
obtaining a modified polypeptide produced by the binding of the carboxamide side chain with the linker;
contacting the modified polypeptide with a maleimide group-containing label, to bind the label to the linker bound to the modified polypeptide; and
obtaining a labeled polypeptide produced by the binding of the modified polypeptide and the label,
wherein,
in the labeled polypeptide,
a side chain of the glutamine residue is represented by formula (I) below: 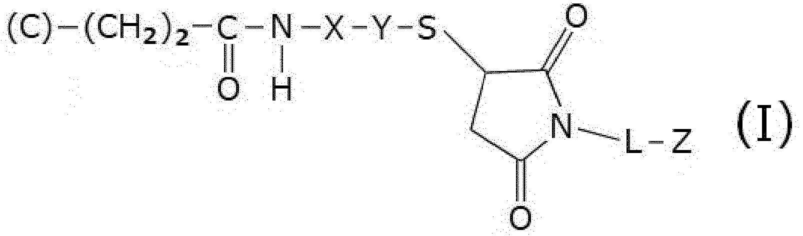
wherein, (C) represents an α-carbon atom of the glutamine residue, X represents a straight chain alkylene group, Y represents a polyethylene glycol chain, Z represents a label, L represents a spacer or an atomic bond, and the polyethylene glycol chain has a molecular weight of 1100 or larger, preferably an average molecular weight of 1300 or larger, more preferably an average molecular weight of 1700 or larger.

12. The production method according to claim 10 or 11, wherein the glutamine residue-containing polypeptide is a fusion polypeptide of an antibody, with a peptide tag that contains the glutamine residue.

13. The production method according to any one of claims 10 to 12, wherein the polyethylene glycol chain is represented by formula (III) below:
-(OCH₂CH₂)ₙ- or -(CH₂CH₂O)ₙ- (III)
wherein, n represents an integer of 25 or larger, preferably an integer of 39 or larger.

14. A measurement method for a target substance, the method comprising:
forming an immune complex of the labeled polypeptide according to claim 2, with a target substance; and
detecting a signal generated by the label contained in the immune complex.

15. The measurement method according to claim 14, wherein, in the forming step, the immune complex is formed on a solid phase.

## Patentansprüche

1. Markiertes Polypeptid, umfassend einen seitenkettenhaltigen Glutaminrest, wobei die Seitenkette durch die nachstehende Formel (I) dargestellt wird: 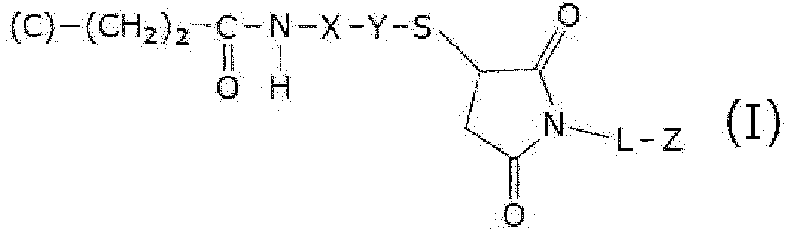 wobei
(C) für einen α-Kohlenstoff des Glutaminrests steht, X für eine geradkettige Alkylengruppe steht, Y für eine Polyethylenglykolkette steht, Z für eine Markierung steht, L für einen Spacer oder eine Atombindung steht und die Polyethylenglykolkette ein Molekulargewicht von 1100 oder größer aufweist.

2. Markiertes Polypeptid nach Anspruch 1, wobei es sich bei dem markierten Polypeptid um ein Fusionspolypeptid eines Antikörpers handelt, mit einem Peptid-Tag, das den seitenkettenhaltigen Glutaminrest umfasst.

3. Modifiziertes Polypeptid, umfassend einen seitenkettenhaltigen Glutaminrest, wobei die Seitenkette durch die nachstehende Formel (II) dargestellt wird: 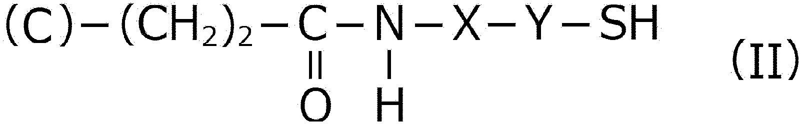 wobei
(C) für einen α-Kohlenstoff des Glutaminrests steht, X für eine geradkettige Alkylengruppe steht, Y für eine Polyethylenglykolkette steht und die Polyethylenglykolkette ein Molekulargewicht von 1100 oder größer, bevorzugt ein mittleres Molekulargewicht von 1300 oder größer, bevorzugt ein mittleres Molekulargewicht von 1700 oder größer, aufweist.

4. Modifiziertes Polypeptid nach Anspruch 3, wobei es sich bei dem modifizierten Polypeptid um ein Fusionspolypeptid eines Antikörpers handelt, mit einem Peptid-Tag, das den seitenkettenhaltigen Glutaminrest umfasst.

5. Polypeptid nach einem der Ansprüche 1 bis 4, wobei die Polyethylenglykolkette durch die nachstehende Formel (III) dargestellt wird:
-(OCH₂CH₂)ₙ- oder -(CH₂CH₂O)ₙ- (III)
(wobei n für eine ganze Zahl von 25 oder größer, bevorzugt für eine ganze Zahl von 39 oder größer steht).

6. Polypeptid nach einem der Ansprüche 1 bis 5, wobei die geradkettige Alkylengruppe 2 oder mehr und 10 oder weniger Kohlenstoffatome aufweist.

7. Markiertes Polypeptid nach einem der Ansprüche 1 bis 6, wobei es sich bei der Markierung um wenigstens einen aus der Gruppe bestehend aus Biotinen, Enzym, Fluoreszenzfarbstoff, Fluoreszenzprotein und Hapten ausgewählten Stoff handelt.

8. Polypeptid nach Anspruch 2 oder 4, wobei es sich bei dem Antikörper um Fab, Fab', F(ab')₂, Fd, Fd', Fv, scFv, dAb, rIgG, Leichte-Kette-, Schwere-Kette-Antikörper, variable Domäne von Schwere-Kette-Antikörper, Diabody oder Triabody handelt.

9. Reagens, umfassend das Polypeptid nach einem der Ansprüche 1 bis 8.

10. Herstellungsverfahren für ein modifiziertes Polypeptid, wobei das Verfahren Folgendes umfasst:
In-Kontakt-Bringen eines glutaminresthaltigen Polypeptids in Gegenwart einer Transglutaminase mit einem Linker, der durch die nachstehende Formel (VI) dargestellt wird:
NH₂-X-Y-SH (VI)
(wobei X für eine geradkettige Alkylengruppe steht, Y für eine Polyethylenglykolkette steht, wobei die Polyethylenglykolkette ein Molekulargewicht von 1100 oder größer aufweist), so dass der Linker an eine Carboxamid-Seitenkette des Glutaminrests gebunden wird; und
Gewinnen eines durch die Bindung der Carboxamid-Seitenkette mit dem Linker hergestellten modifizierten Polypeptids; und
im modifizierten Polypeptid
eine Seitenkette des Glutaminrests durch die nachstehende Formel (II) dargestellt wird: 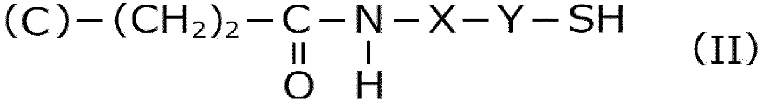
wobei (C) für ein α-Kohlenstoffatom des Glutaminrests steht, X für eine geradkettige Alkylengruppe steht, Y für eine Polyethylenglykolkette steht und die Polyethylenglykolkette ein Molekulargewicht von 1100 oder größer aufweist.

11. Herstellungsverfahren für ein markiertes Polypeptid, wobei das Verfahren Folgendes umfasst:
In-Kontakt-Bringen eines glutaminresthaltigen Polypeptids in Gegenwart einer Transglutaminase mit einem Linker, der durch die nachstehende Formel (VI) dargestellt wird:
NH₂-X-Y-SH (VI)
wobei X für eine geradkettige Alkylengruppe steht, Y für eine Polyethylenglykolkette steht, wobei die Polyethylenglykolkette ein Molekulargewicht von 1100 oder größer aufweist, so dass der Linker an eine Carboxamid-Seitenkette des Glutaminrests gebunden wird;
Gewinnen eines durch die Bindung der Carboxamid-Seitenkette mit dem Linker hergestellten modifizierten Polypeptids;
In-Kontakt-Bringen des modifizierten Polypeptids mit einer eine Maleimidgruppe enthaltenden Markierung, so dass die Markierung an den an das modifizierte Polypeptid gebundenen Linker gebunden wird; und
Gewinnen eines durch die Bindung des modifizierten Polypeptids und der Markierung hergestellten markierten Polypeptids,
wobei
im markierten Polypeptid
eine Seitenkette des Glutaminrests durch die nachstehende Formel (I) dargestellt wird: 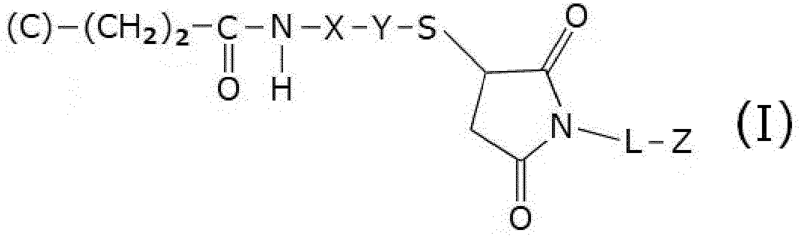
wobei (C) für einen α-Kohlenstoffatom des Glutaminrests steht, X für eine geradkettige Alkylengruppe steht, Y für eine Polyethylenglykolkette steht, Z für eine Markierung steht, L für einen Spacer oder eine Atombindung steht und die Polyethylenglykolkette ein Molekulargewicht von 1100 oder größer, bevorzugt ein mittleres Molekulargewicht von 1300 oder größer, bevorzugter ein mittleres Molekulargewicht von 1700 oder größer, aufweist.

12. Herstellungsverfahren nach Anspruch 10 oder 11, wobei es sich bei dem glutaminresthaltigen Polypeptid um ein Fusionspolypeptid eines Antikörpers handelt, mit einem Peptid-Tag, das den Glutaminrest enthält.

13. Herstellungsverfahren nach einem der Ansprüche 10 bis 12, wobei die Polyethylenglykolkette durch die nachstehende Formel (III) dargestellt wird:
-(OCH₂CH₂)ₙ- oder -(CH₂CH₂O)ₙ- (III)
wobei n für eine ganze Zahl von 25 oder größer, bevorzugt eine ganze Zahl von 39 oder größer, steht.

14. Messverfahren für einen markierten Zielstoff, wobei das Verfahren Folgendes umfasst:
Bilden eines Immunkomplexes des markierten Polypeptids nach Anspruch 2 mit einem Zielstoff; und
Nachweisen eines von der im Immunkomplex enthaltenen Markierung erzeugten Signals.

15. Messverfahren nach Anspruch 14, wobei der Immunkomplex im Bildungsschritt an einer Festphase gebildet wird.

## Revendications

1. Polypeptide marqué comprenant un résidu glutamine contenant une chaîne latérale, la chaîne latérale étant représentée par la formule (I) ci-dessous : 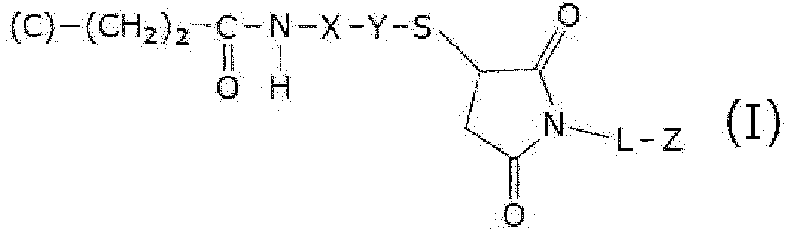 (C) représentant un carbone en α du résidu glutamine, X représentant un groupe alkylène à chaîne droite, Y représentant une chaîne de polyéthylène glycol, Z représentant un marqueur, L représentant un espaceur ou une liaison atomique, et la chaîne de polyéthylène glycol ayant un poids moléculaire de 1 100 ou plus.

2. Polypeptide marqué selon la revendication 1, le polypeptide marqué étant un polypeptide de fusion d'un anticorps, comportant une étiquette de peptide qui comprend le résidu glutamine contenant une chaîne latérale.

3. Polypeptide modifié comprenant un résidu glutamine contenant une chaîne latérale, la chaîne latérale étant représentée par la formule (II) ci-dessous : 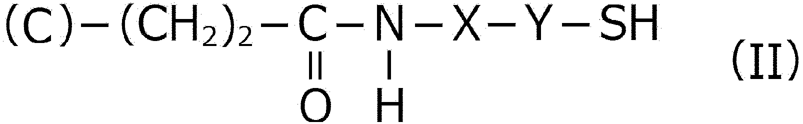 (C) représentant un carbone en α du résidu glutamine, X représentant un groupe alkylène à chaîne droite, Y représentant une chaîne de polyéthylène glycol et la chaîne de polyéthylène glycol ayant un poids moléculaire de 1 100 ou plus, préférablement ayant un poids moléculaire moyen de 1 300 ou plus, préférablement un poids moléculaire moyen de 1 700 ou plus.

4. Polypeptide modifié selon la revendication 3, le polypeptide modifié étant un polypeptide de fusion d'un anticorps, comportant une étiquette de peptide qui comprend le résidu glutamine contenant une chaîne latérale.

5. Polypeptide selon l'une quelconque des revendications 1 à 4, la chaîne de polyéthylène glycol étant représentée par la formule (III) ci-dessous :
-(OCH₂CH₂)ₙ- ou -(CH₂CH₂O)ₙ- (III)
(n représentant un entier de 25 ou plus, préférablement n représentant un entier de 39 ou plus).

6. Polypeptide selon l'une quelconque des revendications 1 à 5, le groupe alkylène à chaîne droite ayant 2 atomes de carbone ou plus et 10 atomes de carbone ou moins.

7. Polypeptide marqué selon l'une quelconque des revendications 1 à 6, le marqueur étant au moins une substance choisie dans le groupe constitué par des biotines, une enzyme, un colorant fluorescent, une protéine fluorescente et un haptène.

8. Polypeptide selon la revendication 2 ou 4, l'anticorps étant Fab, Fab', F(ab')₂, Fd, Fd', Fv, scFv, dAb, rIgG, une chaîne légère, un anticorps à chaîne lourde, un domaine variable d'anticorps à chaîne lourde, un dianticorps ou un trianticorps.

9. Réactif comprenant le polypeptide selon l'une quelconque des revendications 1 à 8.

10. Procédé de production pour un polypeptide modifié, le procédé comprenant :
la mise en contact, en la présence d'une transglutaminase, d'un polypeptide contenant un résidu glutamine avec un lieur représenté par la formule (VI) ci-dessous :
NH₂-X-Y-SH (VI)
(X représentant un groupe alkylène à chaîne droite, Y représentant une chaîne de polyéthylène glycol, la chaîne de polyéthylène glycol ayant un poids moléculaire de 1 100 ou plus), pour lier le lieur à une chaîne latérale carboxamide du résidu glutamine ; et
l'obtention d'un polypeptide modifié produit par la liaison de la chaîne latérale carboxamide avec le lieur ; et,
dans le polypeptide modifié,
une chaîne latérale du résidu glutamine est représentée par la formule (II) ci-dessous : 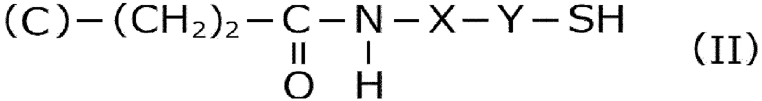
(C) représentant un atome de carbone en α du résidu glutamine, X représentant un groupe alkylène à chaîne droite, Y représentant une chaîne de polyéthylène glycol, et la chaîne de polyéthylène glycol ayant un poids moléculaire de 1 100 ou plus.

11. Procédé de production pour un polypeptide marqué, le procédé comprenant :
la mise en contact, en la présence d'une transglutaminase, d'un polypeptide contenant un résidu glutamine avec un lieur représenté par la formule (VI) ci-dessous :
NH₂-X-Y-SH (VI)
X représentant un groupe alkylène à chaîne droite, Y représentant une chaîne de polyéthylène glycol, la chaîne de polyéthylène glycol ayant un poids moléculaire de 1 100 ou plus, pour lier le lieur à une chaîne latérale carboxamide du résidu glutamine ;
l'obtention d'un polypeptide modifié produit par la liaison de la chaîne latérale carboxamide avec le lieur ;
la mise en contact du polypeptide modifié avec un marqueur contenant un groupe maléimide, pour lier le marqueur au lieur lié au polypeptide modifié ; et
l'obtention d'un polypeptide marqué produit par la liaison du polypeptide modifié et du marqueur,
dans le polypeptide marqué,
une chaîne latérale du résidu glutamine est représentée par la formule (I) ci-dessous : 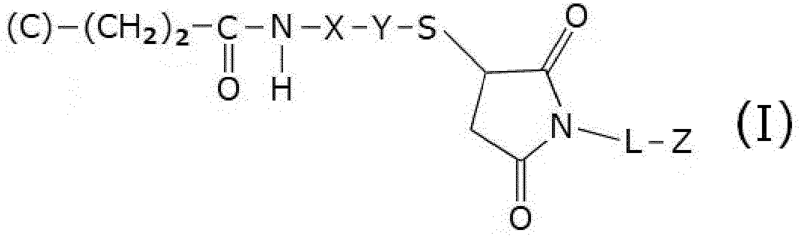
(C) représentant un atome de carbone en α du résidu glutamine, X représentant un groupe alkylène à chaîne droite, Y représentant une chaîne de polyéthylène glycol, Z représentant un marqueur, L représentant un espaceur ou une liaison atomique, et la chaîne de polyéthylène glycol ayant un poids moléculaire de 1 100 ou plus, préférablement un poids moléculaire moyen de 1 300 ou plus, plus préférablement un poids moléculaire moyen de 1 700 ou plus.

12. Procédé de production selon la revendication 10 ou 11, le polypeptide contenant un résidu glutamine étant un polypeptide de fusion d'un anticorps, comportant une étiquette de peptide qui contient le résidu glutamine.

13. Procédé de production selon l'une quelconque des revendications 10 à 12, la chaîne de polyéthylène glycol étant représentée par la formule (III) ci-dessous :
-(OCH₂CH₂)ₙ- ou -(CH₂CH₂O)ₙ- (III)
n représentant un entier de 25 ou plus, préférablement un entier de 39 ou plus.

14. Procédé de mesure pour une substance cible, le procédé comprenant :
la formation d'un complexe immunitaire du polypeptide marqué selon la revendication 2, avec une substance cible ; et
la détection d'un signal généré par le marqueur contenu dans le complexe immunitaire.

15. Procédé de mesure selon la revendication 14, dans l'étape de formation, le complexe immunitaire étant formé sur une phase solide.
